(19) Europäisches Patentamt
European Patent Office
Office européen des brevets

(11) Publication number: **0 235 198**
**B1**

(12) **EUROPEAN PATENT SPECIFICATION**

(45) Date of publication of patent specification: **05.12.90**

(51) Int. Cl.[5]: **C 08 G 63/68,** C 09 B 69/10

(21) Application number: **86905107.8**

(22) Date of filing: **06.08.86**

(86) International application number: **PCT/US86/01651**

(87) International publication number: **WO 87/01121 26.02.87 Gazette 87/05**

# (54) COLORED UNSATURATED POLYESTER MATERIAL CONTAINING COPOLYMERIZED METHINE DYES, AND PRODUCTS THEREFROM.

(30) Priority: **16.08.85 US 766218**

(43) Date of publication of application: **09.09.87 Bulletin 87/37**

(45) Publication of the grant of the patent: **05.12.90 Bulletin 90/49**

(84) Designated Contracting States: **DE FR GB IT SE**

(56) References cited:
**DE-A-1 182 061**
**FR-A-2 375 273**
**US-A-3 706 700**

**Chemical Abstract, volume 71, no. 12, 22 September 1969, (Columbus, Ohio, US), V.V. Korshak et al.: "Colored polyesters based on bis (hydroxyalkoxy) azo-benzenes", see page 24, abstract no. 50783u, & Izv. Akad. Nauk SSSR, Ser. Khim. 1969, (5), 1078-85**

(73) Proprietor: **EASTMAN KODAK COMPANY (a New Jersey corporation)**
**343 State Street**
**Rochester New York 14650 (US)**

(72) Inventor: **PRUETT, Wayne, Payton**
**P.O. Box 1972**
**Kingsport, TN 37662 (US)**
Inventor: **WANG, Richard, Hsu-Shien**
**P.O. Box 1972**
**Kingsport, TN 37662 (US)**
Inventor: **HILBERT, Samuel, David**
**P.O. Box 1972**
**Kingsport, TN 37662 (US)**
Inventor: **WEAVER, Max, Allen**
**P.O. Box 1972**
**Kingsport, TN 37662 (US)**

(74) Representative: **Parent, Yves et al**
**Kodak-Pathé Département Brevets et Licences**
**Centre de Recherches et de Technologie Zone Industrielle**
**F-71102 Chalon-sur-Saône Cédex (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 235 198 B1

## Description

This invention relates to colored, unsaturated polyesters which contain certain methine dye moieties chemically linked through copolymerization into the polyester backbone whereby the dye moieties become essentially nonextractable from the polyester. These colored polyesters are thus highly suitable for incorporation into curable polyester materials used in the production of bathroom fixtures (sinks, showers, tubs), boats, automotive parts, and the like wherein resistance to dye extractability is of great consequence. The addition of the methine dye monomers during the polyester preparation also gives greater colorant uniformity than possible, for example, where such colorants are simply blended with the polyester material prior to casting or molding thereof.

It is known, of course, that some methine dyes may be used to color polyester fibers using conventional dyeing procedures. It is also known that certain dyes can be melt blended (not copolymerized) with preformed polyesters or dispersed with reactive solvent (curing monomer) into unsaturated polyester material prior to molding and curing, to produce colored product. In other limited cases, certain dyes such as selected anthraquinones are reactable into the polyester chain by copolymerization.

Advantages of the present methine moieties over others which might be copolymerized into the polyester include (1) greater thermal stability, for example, than azo dyes which generally are not stable to polymer preparation temperatures, (2) methine dyes can be selected or tailored to absorb light at the proper wavelengths to protect particular contents of a package, (3) certain anthraquinone dyes, for example, which absorb light below 420 nm are generally not stable to UV, (4) the present methine dyes have high extinction coefficients, i.e., less dye is needed to give equivalent color, and (5) generally speaking, methine dyes are much less costly than anthraquinone dyes.

In accordance with the present invention, the methine moieties have at least one methine unit defined herein as "the group >C=C< conjoined with a conjugated aromatic system." This unit imparts to the moiety and to the polymer the property of ultraviolet or visible light absorption generally within the range of about 320 nm to about 650 nm. The moieties preferably have molecular weights of from about 200 to about 600 although lower and higher molecular weights are also operable. The moieties are derived from reactants (monomers) having one or more groups which condense during esterification or polycondensation to enter the moiety into the polymer chain. These groups include hydroxyl, carboxyl, carboxylic ester, acid halide, amino and the like. As aforesaid, these methine moieties are thermally stable at polymer processing conditions, for example, including polycondensation temperatures of up to about 300°C. Of course, where only one condensable group is present, the methine monomer would act as a chain terminator in known manner. These moieties are useful in total concentrations ranging from about 1.0 to about 5,000, preferably 2.0 to about 1,500 parts by weight of moiety per million parts by weight of final polymer (ppm).

The present invention is defined in its broad embodiment as unsaturated polyester material having copolymerized therein a total of from 1.0 to about 5,000 ppm, of at least one methine moiety having one or more methine units, said moiety absorbing in the range of from 320 nm to about 650 nm, and being nonextractable from said polyester material and stable under processing conditions therefor.

The extractabilities of the present methine moieties can be determined as follows:

### Extraction Procedure

All extractions are done in glass containers with distilled solvents (water, heptane, etc.) under the time and temperature conditions described below. The sample form is 0.5 inch × 2.5 inch (12.7 × 63.5 mm) molded segments of about 10 to 20 ml thickness, the thickness, however, not being critical. All samples are washed with cold solvent to remove surface contaminants and are then contacted with 2 ml of solvent/inch$^2$ (1 ml of solvent/3.2 cm$^2$) of surface area. After the specified period of ageing in the solvent, the solvent is transferred to glass flasks, concentrated, spiked and analyzed. Solvent blanks (controls) are run under the same conditions without polymer.

### Extraction Conditions

1. *Water.* The samples are added to the solvent at room temperature and heated at 250°F (121°C) (15 psi, 1.03 bar) for two hours. For half the samples, the solvent is transferred to glass flasks, concentrated, and analyzed; the remaining samples are placed in a 120°F (49°C) oven for 30 days and the solvent then transferred to glass flasks, concentrated, and analyzed.

2. *50% Ethanol/water (v/v).* The samples are added to solvent at room temperature, placed in an oven at 120°F (49°C), concentrated, and analyzed after 24 hours and 30 days.

3. *Heptane.* The samples are added to solvent at 150°F (65°C) and heated at 150°F (65°C) for 2 hours. Part of the samples are transferred to glass flasks, concentrated, and analyzed. The remainder of the samples are placed in a 120°F (49°C) oven for 30 days and the solvent then transferred to glass flasks, concentrated, and analyzed.

Analysis

Instrument — Hewlett-Packard 8450A spectrophotometer

Cell Path Length — 1 cm

Scanning Range — 400 nm to 650 nm

Calibration for Red Methine dye in Anisole — 520 nm absorbance maximum

Calibration for Red Methine dye in DMF — 530 nm absorbance maximum

The Hewlett-Packard 8450 A spectrophotometer is equipped with microprocessor and programming capability with RAM memory and floppy disk storage. The instrument is calibrated at 1000 ppb, 1500 ppb, and 2000 ppb dye in anisole and 970 ppb, 1460 ppb, and 1940 ppb in the DMF. The calibration data are stored on a floppy disk for recall when needed.

The extractability of the present methine moieties from the present cured polyester material is essentially nonexistent.

Useful methine reactants or monomers for the present invention have the géneral formulas:

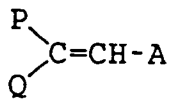

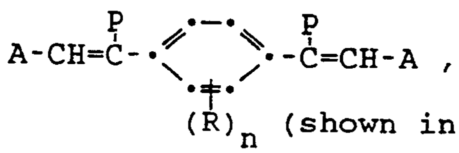 ,

(shown in Table 12)

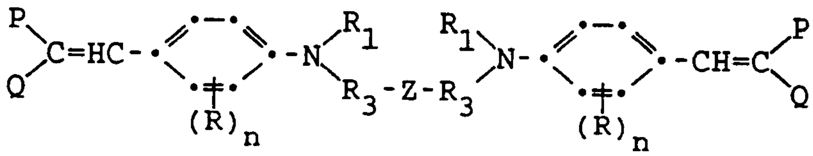

(shown in Table 2)

and 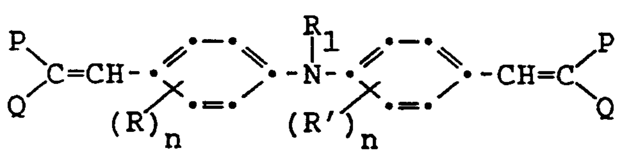

(Shown in Table 11)

wherein A is selected from the following radicals designated by their exemplary table numbers:

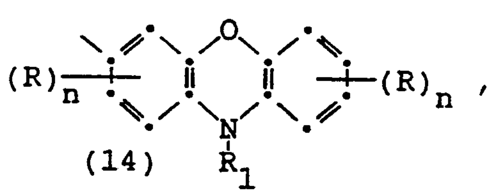 ,

(14)

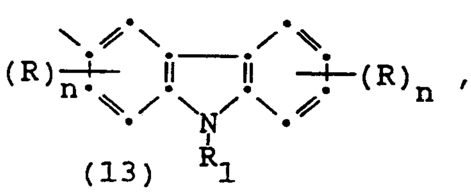 ,

(13)

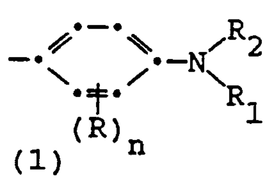 ,

(1)

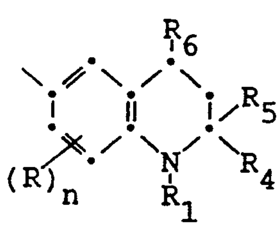 ,

(3)

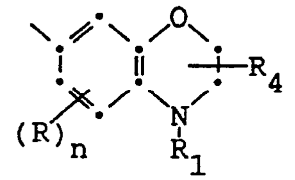

(4)

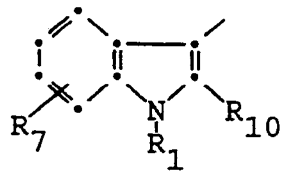

(5)

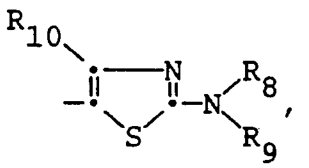

(6)

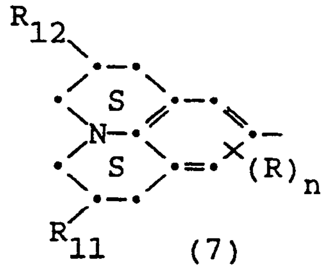

(7)

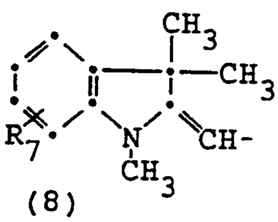

(8)

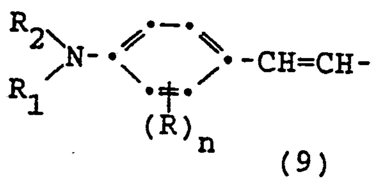

(9)

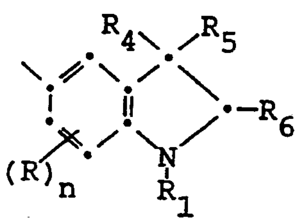

(10)

, and

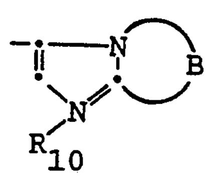

(15)

wherein:

R and R′ are selected from hydrogen, fluorine, chlorine, bromine, alkyl, alkoxy, phenyl, phenoxy, alkythio, and arylthio; n is 0, 1, 2;

$R_1$ and $R_2$ are selected from hydrogen; cycloalkyl; cycloalkyl substituted with one or two of alkyl, —OH, alkoxy, halogen, or hydroxy substituted alkyl; phenyl; phenyl substituted with alkyl, alkoxy, halogen, alkanoylamino, carboxy, cyano, or alkoxycarbonyl; straight or branched lower alkenyl; straight or branched alkyl of 1—8 carbons and such alkyl substituted with the following: hydroxy; halogen; cyano; succinimido; hydroxysuccinimido; acyloxysuccinimido; glutarimido; phenylcarbamoyloxy; phthalimido; 4-carboxyphthalimido; phthalimidino; 2-pyrrolidono; cyclohexyl; phenyl; phenyl substituted with alkyl, alkoxy, halogen, hydroxy alkanoylamino; carboxy, cyano, or alkoxycarbonyl; alkylsulfamoyl; vinylsulfonyl; acrylamido; sulfamyl; benzoylsulfonicimido; alkylsulfonamido; phenylsulfonamido; alkoxycarbonylamino; alkylcarbamoyloxy; alkoxycarbonyl; alkoxycarbonyloxy; alkenylcarbonylamino; groups of the formula

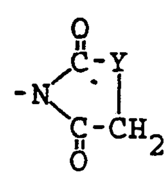

wherein Y is —NH—,

—N—alkyl,

—O—, —S—, or —$CH_2O$—; —S—$R_{14}$; $SO_2CH_2CH_2SR_{14}$; wherein $R_{14}$ is alkyl, phenyl, phenyl substituted with halogen, alkyl, alkoxy, alkanoylamino, cyano, or alkoxycarbonyl; pyridyl; pyrimidinyl; benzoxazolyl; benzimidazoylyl; benzothiazolyl; radicals of the formulae

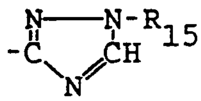 ;

—$OXR_{16}$; —$NHXR_{16}$; —X—$R_{16}$; —$CONR_{15}R_{15}$; and —$S_2NR_{15}R_{15}$; wherein $R_{15}$ is selected from H, aryl, alkyl, and alkyl substituted with halogen, —OH, phenoxy, aryl, —CN, cycloalkyl, alkylsulfonyl, alkylthio, alkanoyloxy, or alkoxy; X is —CO—, —COO—, or —$SO_2$; $R_{16}$ is selected from alkyl and alkyl substituted with halogen, hydroxy, phenoxy, aryl, cyano, cycloalkyl, alkylsulfonyl, alkylthio, alkanoyloxy, and alkoxy; and when X is —CO—, $R_{16}$ also can be hydrogen, amino, alkenyl, alkylamino, dialkylamino, arylamino, aryl, or furyl; alkoxy, alkoxy substituted with hydroxy, cyano, alkanoyloxy, or alkoxy; phenoxy; phenoxy substituted with one or more of alkyl, carboxy, alkoxy, carbalkoxy, or halogen; $R_1$ and $R_2$ can be a single combined group such as pentamethylene, tetramethylene, ethyleneoxyethylene, ethylene sulfonylethylene, or

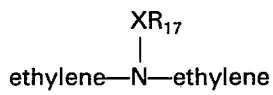

which, with the nitrogen to which it is attached, forms a ring; $R_{17}$ is alkyl, aryl, or cycloalkyl;

$R_3$ is alkylene, arylene, aralkylene, alkyleneoxy, or alkyleneoxyalkylene;

Z is a direct single bond, OCO, O, S, $SO_2$, $R_{17}SO_2N$=,

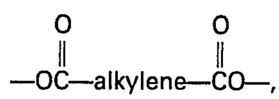, 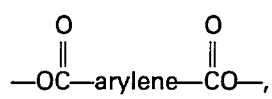, —S—S—, 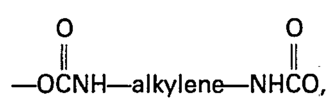,

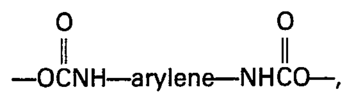, 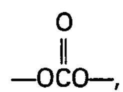,

arylene, or alkylene;

$R_4$, $R_5$, and $R_6$ are each selected from hydrogen and alkyl;

$R_7$ is carboxy, carbalkoxy, or $(R)_n$;

$R_{10}$ is hydrogen, alkyl, and aryl;

$R_8$ and $R_9$ are selected from hydrogen and substituted or unsubstituted alkyl, aryl, or cycloalkyl;

$R_{11}$ and $R_{12}$ are hydrogen, alkyl, hydroxyl, or acyloxy;

B represents the atoms necessary to complete a five or six membered ring and is selected from

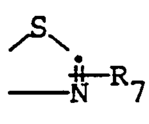 , 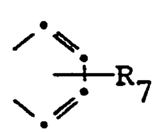 , 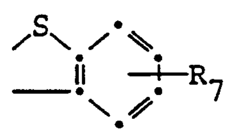 ,

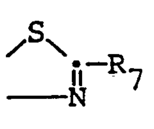 , 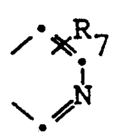 , 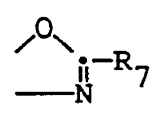 ,

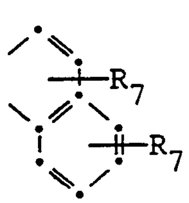 , 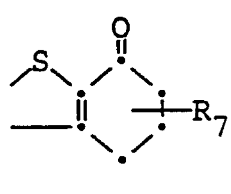 , 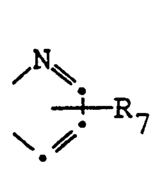

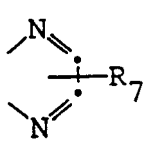 and 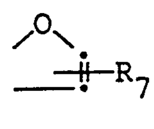 ;

P and Q are selected from cyano, carbalkoxy, carbaryloxy, carbaralkyloxy, carbamyl, carboxy, N-alkylcarbamyl, N-alkyl-N-arylcarbamyl, N,N-dialkylcarbamyl, N-arylcarbamyl, N-cyclohexylcarbamyl, aryl, 2-benzoxazolyl, 2-benzothiazolyl, 2-benzimidazolyl, 1,3,4-thiadiazol-2-yl, 1,3,4-oxadiazol-2-yl, $SO_2$ alkyl, $SO_2$ aryl, and acyl, or P and Q may be combined as

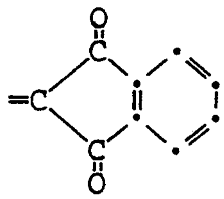

or

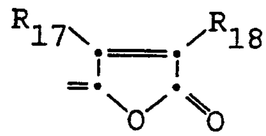

wherein $R_{17}$ is defined above and $R_{18}$ is CN, COOH, $CO_2$ alkyl, carbamyl, or N-alkylcarbamyl;

wherein at least one of A, P, and Q for each dye molecule must be or bear a condensable group selected from carboxy, carbalkoxy, carbaryloxy, N-alkylcarbamyloxy, acyloxy, chlorocarbonyl, carbamyloxy, N-(alkyl)$_2$carbamyloxy, amino, alkylamino, hydroxyl, N-phenylcarbamyloxy, cyclohexanoyloxy, and carbocyclohexyloxy; and

wherein in the above definitions, each alkyl, aryl, or cycloalkyl moiety or portion of a group or radical may be substituted where appropriate with hydroxyl, acyloxy, alkyl, cyano, alkoxycarbonyl, halogen, alkoxy, or aryl, aryloxy, or cycloalkyl. Also in the above definitions, at least one of A, P, and Q for each dye molecule must be or bear a group capable of reacting under polymerization conditions, to incorporate the methine dye into the polymer, including the following, carboxy, carbalkoxy, carbaryloxy, N-alkylcarbamyloxy, acyloxy, chlorocarbonyl, carbamyloxy, N-alkylcarbamyloxy, amino, alkylamino, hydroxyl, N-phenylcarbamyloxy, cyclohexanoyloxy, and carbocyclohexyloxy, wherein the alkyl and/or aryl groups may contain common substituents such as hydroxyl, cyano, acyloxy, carbalkoxy, phenyl, and halogen which do not interfere with the condensation reaction.

In all of the above definitions the alkyl or alkylene moieties or portions of the various groups contain from 1—8 carbons, straight or branched chain.

The unsaturated polyesters useful in this invention include the esterification and polycondensation products of one or more unsaturated dicarboxylic acids or their anhydrides and one or more dihydric or polyhydric alcohols. As the acid component, the α,β-ethylenically unsaturated polycarboxylic acids include those having 2—12 carbon atoms, e.g., maleic, fumaric, substituted fumaric, citraconic, mesaconic, tetraconic, glutaconic, muconic, and the like, as well as mixtures thereof. Noncurable polycarboxylic acids, i.e., those which do not contain reactive α,β-ethylenic unsaturation, may also be used if employed in combination with one or more of the aforementioned α,β-ethylenically unsaturated polycarboxylic acids. Where such noncurable polycarboxylic acids are employed, the amount thereof should not constitute more than about 80% of the total equivalents of carboxyl groups in the esterification mixture. Preferably, such noncurable polycarboxylic acids will be employed in amounts varying between about 35% and 75% of the above indicated equivalence basis. Among the noncurable polycarboxylic acids that may be used are oxalic, malonic, succinic, glutaric, sebacic, adipic, phthalic, isopthalic, terephthalic, substituted phthalic, such as tetrachlorophthalic, suberic, azelaic, tricarballylic, citric, tartaric, cyclopropanedicarboxylic, cyclohexanedicarboxylic, and the like, as well as mixtures thereof.

As the alcohol component, the saturated aliphatic polyhydric alcohols include those preferably containing only two hydroxyl groups. Among such diols are ethylene glycol, propylene glycol, butylene glycol, diethylene glycol, dipropylene glycol, triethylene glycol, tetraethylene glycol, butanediol-1,2, butanediol-1,3, butanediol-1,4, pentanediol-1,2, pentanediol-1,4, pentanediol-1,5, hexanediol-1,6, neopentyl glycol, 2,2,4-trimethyl-1,3-pentanediol and the like, as well as mixtures thereof. However, saturated aliphatic polyhydric alcohols containing more than two hydroxyl groups may also be employed and include glycerol, trimethylol ethane, trimethylol propane, pentaerythritol, sorbitol, and the like, as well as mixtures thereof. It is usually desirable that such polyols be employed in minor proportions relative to the diol or diols.

The components may be reacted in the manner customarily used in preparing ethylenically unsatruated polyester resins, i.e., at elevated temperatures and atmospheric pressure, although pressures slightly above or below atmospheric may be employed if desired. The reaction temperature is not critical but preferably is just below the boiling point of the most volatile component of the reaction mixture which is generally the alcohol component. However, temperatures in excess of the boiling point of the most volatile constituent may be employed if the reaction vessel has been equipped with a steam-heated reflux condenser which permits water of esterification to escape from the reaction vessel while condensing volatilized reaction components and returning them to the reaction system.

In preparing the curable compositions, the unsaturated polyesters are blended with a reactive curing agent, operably in a weight ratio of agent/polyester of 10/90 to 90/10, but preferably from 30/60 to 70/40. These agents contain one or more $CH_2=C<$ groups and desirably have boiling points at atmospheric pressure of 60°C or greater. Such agents include styrene, side-chain substituted styrenes such as the α-methyl styrene, α-ethyl styrene, and the like, ring substituted styrenes, such as alkyl styrenes, e.g., ortho-

meta and para-alkyl styrenes, including o-methyl styrene, p-ethyl styrene, meta-propyl styrene, 2,4-dimethyl styrene, 2,5-diethyl styrene, and the like, halostyrenes, e.g., o-bromostyrene, p-chlorostyrene, 2,4-dichlorostyrene, and the like. Also included are alkyl esters of acrylic and methacrylic acid, e.g., methyl, ethyl, or butyl acrylate, methyl methacrylate, and the like. Also useful are vinyl acetate, vinyl butyrate, vinyl laurate, acrylonitrile, methacrylonitrile, vinyl chloride, acrylamide, methacrylamide and their dderivatives, allyl compounds such as diallyl phthalate, allyl acetate, allyl methacrylate, diallyl carbonate, allyl lactate, allyl α-hydroxyisobutyrate, allyl trichlorosilane, allyl acrylate, diallyl malonate, diallyl oxalate, diallyl gluconate, dially methyl gluconate, dially adipate, diallyl sebacate, diallyl tartronate, diallyl tartarte, diallyl mesaconate, diallyl citraconate, the diallyl ester of muconic acid, diallyl itaconate, diallyl chlorophthalate, diallyl dichlorosilane, the diallyl ester of endomethylenetetrahydrophthalic anhydride, triallyl tricarballylate, triallyltrimesate, triallyl aconitate, triallyl cyanurate, triallyl citrate, triallyl phosphate, trimethyallyl phosphate, tetraallyl silane, tetraallyl silicate, hexallyldisiloxane, and the like. These curing agents may be used singly or in combination with one another.

In order to facilitate the curing, it is preferred that a polymerization catalyst be incorporated in the blend at the time of its curing. The type and amounts of these catalytic materials are well known in the art, and any material which normally induces polymerization of polyester resinous compositions can be utilized. The optimum reaction conditions are modified to some extent by the choice of the particular catalyst used in the process. A very active catalyst should be used in lower concentrations, and preferably at lower temperatures, than a less reactive material. The preferred catalysts comprise a wide variety of organic superoxides, i.e., organic peroxides ("acidic peroxides") and hydroperoxides ("alcoholic peroxides"). Mixtures of peroxides and hydroperoxides, including commercially available mixtures such as methyl ethyl ketone peroxide, cyclohexanone peroxide, and the like, are especially effective as catalysts. Among the useful organic peroxide catalysts are acetyl peroxide, benzoyl peroxide, substituted benzoyl peroxides, halogenated benzyl peroxides such as p-bromobenzoyl peroxide, and 2,4-dichlorobenzoyl peroxide, benzoyl acetyl peroxide, phthalyl peroxide, succinyl peroxide, fatty oil acid peroxides, such as coconut oil peroxide, lauryl peroxide, stearyl peroxide, oleyl peroxide, anisoyl peroxide, toluyl peroxide, and the like. Organic peracids, such as peracetic acid and perbenzoic acid, may also be employed. The useful organic hdyroperoxide catalysts include tertiary butyl hydroperoxide, cumene hydroperoxide, diisopropyl benzene hydroperoxide, 1-hydroxycyclohexyl hydroperoxide, the terpene oxides, such as ascaridole, 1-p-methane hydroperoxide, and the like. Various other types of polymerization catalysts may also be employed, for example, compounds such as aluminum chloride, stannic chloride, boron trifluoride, or the azo-type catalysts such as α,α'-azobisisobutyronitrile.

Since the unsaturated polyester may contain a high degree of polymerizable or reactive unsaturation, it is often desirable to blend a polymerization inhibitor therewith to retard internal polymerization of the polyester during any storage period encountered prior to curing. Once the curable composition is contacted with a sufficient amount of a polymerization catalyst, however, the effect of the inhibitor will be overcome. Among the useful inhibitors are phenol, the monoalkyl phenols, such as ortho-, meta-, and para-cresol as well as mixtures of such isomers, polyalkyl phenols having a plurality of the same or different substituents, e.g., ethyl, propyl, butyl, and higher alkyl radicals attached to their nuclei, catechol, tertiary butyl catechol, hydroquinone, tertiary butyl hydroquinone, resorcinol, eugenol, guaiacol, pyrogallol, benzaldehyde, tannic acid, ascorbic acid, isoascorbic acid, phenylene diamine, sym-di-β-naphthyl-p-phenylene diamine, aniline, and the like. The amount of polymerization inhibitor employed depends on the nature of the unsaturated polyester as well as the period of storage stability required. Generally, from about 0.001% to 0.3% by weight, based on the total weight of the curable polyester blend will be sufficient.

Inhibitors of this type may be added during preparation of the unsaturated polyester or optionally added later to the curable blend. In addition, other known additives may be employed such as promoters used in conjunction with the catalyst, mold lubricants, fillers and reinforcing materials, other colorants, flow promoters, ultraviolet absorbing agents, and the like.

The conditions necessary for curing the above blends do not depart from the practice ordinarily observed in curing these types of compositions in general. They may be cured in contact with air or in enclosed molds at temperatures ranging from about 10°C to about 160°C, or even higher as long as they are kept below the point at which the particular curable blend employed begins to decompose. Where it is convenient, it is especially desirable to cure the catalyzed blends by heating to between 90°C and about 150°C for a period of about 3 to 90 minutes.

In general, the methine reactants (monomers) are prepared, for example, by reacting the hydrogenated parent of an aromatic moiety A above, which is electron rich, with a Vilsmeier complex to produce an aldehyde [Bull. Societe Chim. de France, No. 10:1898—99 (October 1962); Angewandte Chemie *72*, No. 22, 836—845, November 21, 1960]. For reasons of cost and convenience, phosphorus oxychloride ($POCl_3$) and N,N-dimethylformamide (DMF) are the preferred reagents. Thus, aromatic amines such as anilines, m-toluidines, 2,5-dimethylanilines, 2,5-dimethoxyanilines, or the like are converted in high yields into the corresponding aldehydes via the Vilsmeier reaction, which aldehydes are subsequently reacted with an active methylene compound of the formula P—$CH_2$—Q in the presence of a base, such as piperidine to produce the methine monomers, as shown in the following reaction sequence

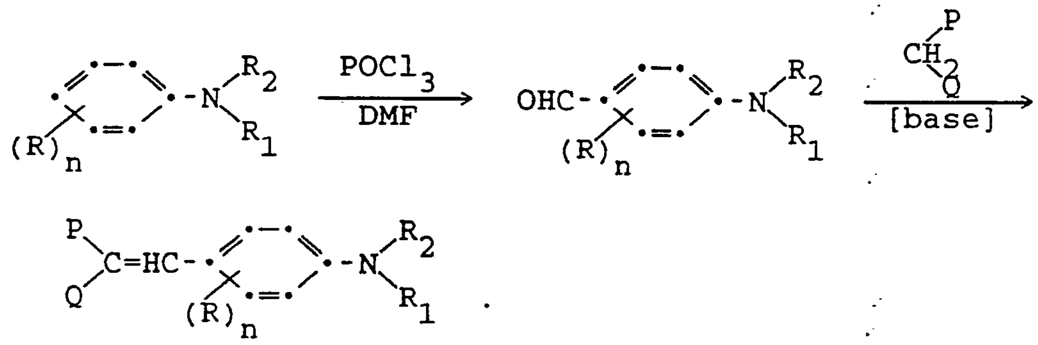


Tetrahydroquinolines, benzomorpholines, indoles, thiazoles, and Fischer's base also undergo the Vilsmeier reactions to produce aldehydes. Preparation of the methine monomers and intermediate aldehydes via the above route is disclosed in considerable detail in many patents including: U.S. 2,649,471; U.S. 2,850,520; U.S. 3,247,211; U.S. 3,260,737; U.S. 3,326,960; U.S. 3,349,098; U.S. 3,386,491; U.S. 3,390,168; U.S. 3,453,270; U.S. 3,453,280; U.S. 3,468,619; U.S. 3,504,010; U.S. 3,555,016; U.S. 3,597,434; U.S. 3,652,636; U.S. 3,661,899; U.S. 3,728,374; U.S. 3,787,476; U.S. 3,829,410; U.S. 3,829,461; U.S. 3,846,069; U.S. 3,869,495; U.S. 3,869,498; U.S. 4,879,434; U.S. 3,920,719; and U.S. 4,077,962. It is also known that it is not necessary to isolate the intermediate aldehyde before preparing the methine monomers as the Vilsmeier reaction mixture can be reacted directly to produce the desired product (U.S. Patents 3,917,604 and 4,088,673). All of these patent teachings are incorporated herein by reference.

Since the Vilsmeier complexes also convert hydroxyl groups to halogen, other methods must be employed to prepare methine monomers bearing hydroxyl groups. For example, the monomers can be prepared according to the scheme

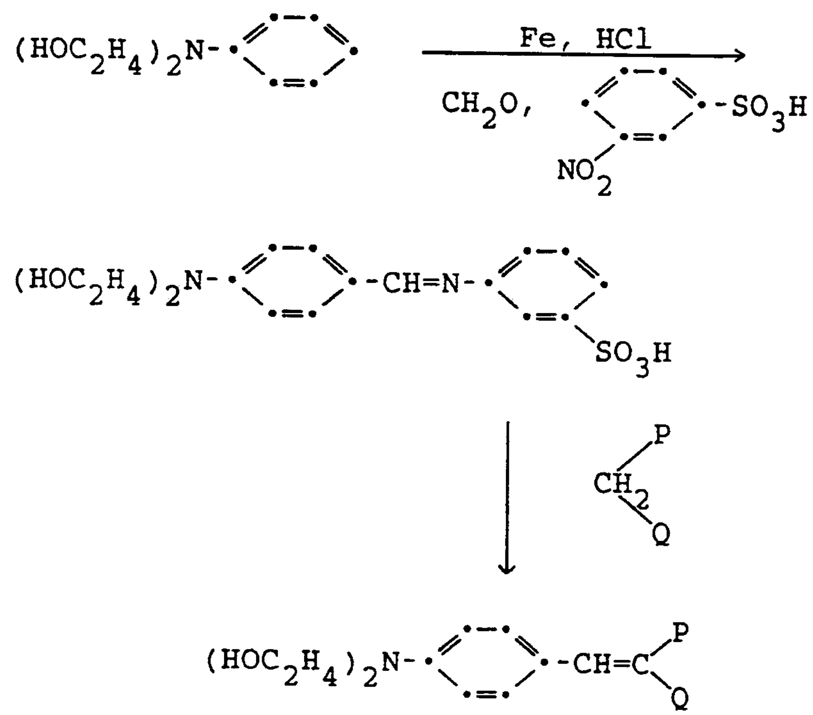


comprising reacting N,N-di-β-hydroxyethylaniline with formaldehyde and m-nitrobenzenesulfonic acid in the presence of concentrated HCl and iron filings to produce the intermediate 4'-(di-β-hydroxyethylamino)-benzalaniline-m-sulfonic acid, which can be reacted with active methylenes to produce the monomers (U.S. 2,583,551).

Another method for producing methine monomers containing dicyanovinyl groups is described in U.S. 4,006,178 wherein aromatic amines are reacted with 1-halogeno-2,2-dicyanoethylene to produce corresponding methine compounds as follows:

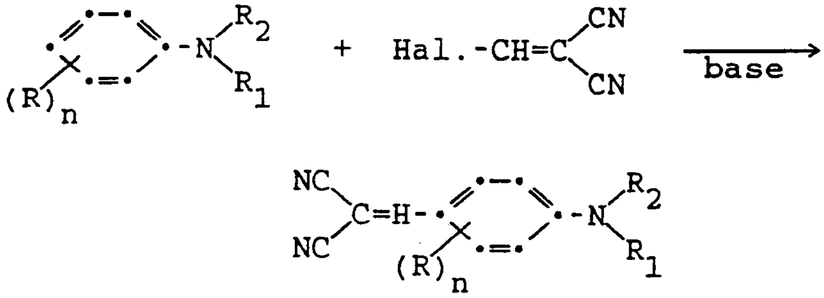

Intermediate aldehyde compounds containing groups such as acyloxy or alkoxycarbonyl can be hydrolyzed to prepare methine monomers containing hydroxyl or carboxy groups, respectively, which are capable of being reacted into the condensation polymer.

The following examples will illustrate the common reaction of an aromatic aldehyde with an active methylene to produce a typical methine monomer.

Example 1

4-(N,N-Dimethylamino)cinnamaldehyde (1,75 g, 0.01 m), methyl cyanoacetate (0.99 g, 0.01 m), methanol (20 mL), and piperidine (3 drops) are mixed and heated together at reflux for 30 minutes. After being allowed to cool, the reaction mixture is filtered. The orange dye (2.5 g) is washed with methanol and air-dried and has the following structure.

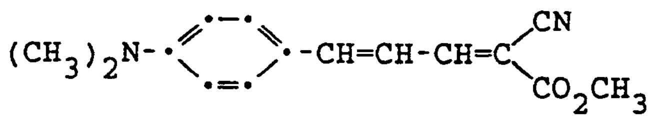

This yellow dye absorbs light at $\lambda_{max}$ 464 nm with a molar extinction coefficient of 38,000.

Example 2

Ethyl [[4-(dimethylamino)phenyl]methylene]propenedioate shown below is prepared by the reaction of 4-(dimethylamino)benzaldehyde with diethyl malonate in the presence of a base catalyst in toluene. This pale yellow dye absorbs UV light at $\lambda_{max}$ 373 nm with a molar extinction coefficient of 33,000.

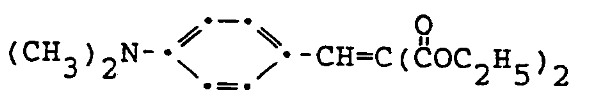 .

The following tables further exemplify the useful methine reactants.

TABLE 1

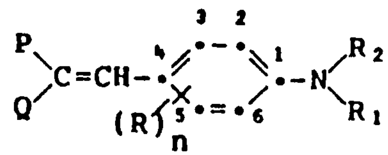

| Example No. | $(R)_n$ | $R_1$ | $R_2$ | P, Q |
|---|---|---|---|---|
| 3 | H | $CH_2CH_2OH$ | $CH_2CH_2OH$ | CN, CN |
| 4 | H | $CH_2CH_2OH$ | $CH_2CH_2OH$ | CN, $SO_2CH_3$ |
| 5 | $3-CH_3$ | $CH_2CH_2OH$ | $CH_2CH_2OH$ | CN, $CONHC_6H_5$ |
| 6 | $3-CH_3$ | $CH_2CH_2OH$ | $CH_2CH_2OH$ | CN, $SO_2CH_3$ |
| 7 | H | $CH_2CH_2OH$ | $CH_2CH_2OH$ | CN, $COC_6H_5$ |
| 8 | H | $CH_2CH_2OH$ | $CH_2CH_2OH$ | CN, 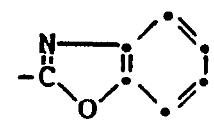 |
| 9 | $3-CH_3$ | $CH_2CH(OH)CH_3$ | $C_2H_5$ | CN, $CO_2CH_3$ |
| 10 | 2,5-di-$OCH_3$ | $CH_2CH(OH)CH_2OH$ | $C_2H_5$ | CN, $CONH_2$ |
| 11 | $2-OCH_3$, $5-CH_3$ | $C_2H_5$ | $C_2H_5$ | CN, $CO_2C_2H_5$ |
| 12 | $2-OCH_3$, 5-Cl | $C_2H_5$ | $C_2H_5$ | CN, $CO_2C_6H_5$ |
| 13 | $2-SCH_3$ | $C_2H_5$ | $C_2H_5$ | CN, $CO_2C_6H_{11}$ |
| 14 | $2-OC_6H_5$ | $C_2H_5$ | $C_2H_5$ | CN, $CONHC_2H_4OH$ |

TABLE 1 (continued)

| Example No. | $(R)_n$ | $R_1$ | $R_2$ | P, Q |
|---|---|---|---|---|
| 15 | H | $C_2H_4O\overset{O}{\overset{\|}{C}}CH_3$ | $C_2H_5$ | CN, $CO_2CH_3$ |
| 16 | H | $C_2H_4O\overset{O}{\overset{\|}{C}}CH_3$ | $C_2H_4O\overset{O}{\overset{\|}{C}}CH_3$ | CN, CN |
| 17 | 3-$CH_3$ | $C_2H_4O\overset{O}{\overset{\|}{C}}CH_3$ | $C_2H_4O\overset{O}{\overset{\|}{C}}CH_3$ | CN, $CO_2CH_3$ |
| 18 | 3-$CH_3$ | $C_2H_4O\overset{O}{\overset{\|}{C}}CH_3$ | $C_2H_4O\overset{O}{\overset{\|}{C}}CH_3$ | $CO_2C_2H_5$, $CO_2C_2H_5$ |
| 19 | H | $C_2H_4O\overset{O}{\overset{\|}{C}}CH_3$ | $C_2H_4O\overset{O}{\overset{\|}{C}}CH_3$ | 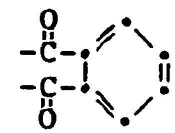 |
| 20 | H | $C_2H_4O\overset{O}{\overset{\|}{C}}CH_3$ | $C_2H_4O\overset{O}{\overset{\|}{C}}CH_3$ | 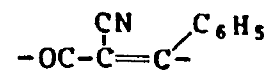 |
| 21 | H | $C_2H_4O\overset{O}{\overset{\|}{C}}CH_3$ | $C_2H_5$ | 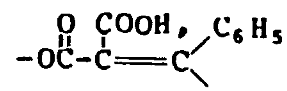 |
| 22 | H | $CH_3$ | $CH_3$ | 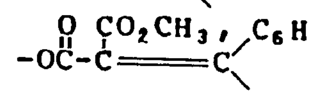 |
| 23 | H | $C_6H_5$ | $CH_2CH_2O\overset{O}{\overset{\|}{C}}CH_3$ | CN, $CO_2CH_3$ |
| 24 | 3-$CH_3$ | $C_6H_5$ | $CH_2CH_2O\overset{O}{\overset{\|}{C}}CH_3$ | CN, COOH |
| 25 | 3-$OC_2H_4O\overset{O}{\overset{\|}{C}}CH_3$ | p-$CH_3C_6H_4$ | $CH_2CH_2O\overset{O}{\overset{\|}{C}}CH_3$ | CN, $CO_2CH_2CH_2CH_2CH_3$ |
| 26 | 3-$CH_3$ | m-$ClC_6H_4$ | $CH_2CH_2OH$ | CN, $CO_2CH_3$ |
| 27 | H | $CH_2C_6H_5$ | $CH_2C_6H_5$ | CN, $CO_2CH_3$ |
| 28 | 3-$CH_3$ | $CH_2C_6H_5$ | 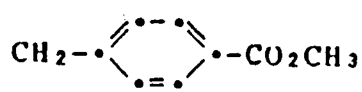 | CN, CN |

TABLE 1 (continued)

| Example No. | $(R)_n$ | $R_1$ | $R_2$ | P, Q |
|---|---|---|---|---|
| 29 | $3-CH_3$ | 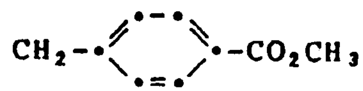 | 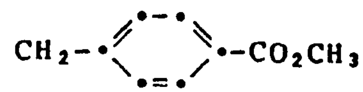 | CN, $COC(CH_3)_3$ |
| 30 | $3-CH_3$ | 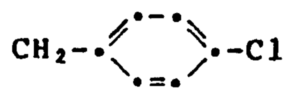 | $CH_2CH_2O\overset{O}{\overset{\parallel}{C}}CH_3$ | CN, $CO_2CH_3$ |
| 31 | $3-CH_3$ | 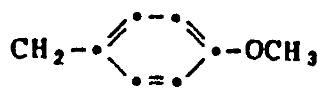 | $CH_2CH_2O\overset{O}{\overset{\parallel}{C}}C_6H_5$ | CN, $CO_2CH_2CH_2OCH_3$ |
| 32 | $3-CH_3$ | $CH_2CH_2O\overset{O}{\overset{\parallel}{C}}C_6H_5$ | $C_2H_5$ | CN, $CO_2CH_3$ |
| 33 | $3-CH_3$ | $CH_2CH_2O\overset{O}{\overset{\parallel}{C}}NHC_6H_5$ | $C_2H_5$ | CN, $CO_2CH_3$ |
| 34 | H | $C_6H_5$ | $C_6H_5$ | CN, $CO_2CH_3$ |
| 35 | $3-CH_3$ | $CH_2CH_2CN$ | $C_2H_5$ | CN, $CO_2CH_3$ |
| 36 | H | $CH_2CH_2CO_2CH_3$ | $CH_2CH_2CO_2CH_3$ | 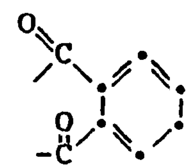 |
| 37 | H | $CH_2CH_2CO_2H$ | $CH_2CH_2CO_2H$ | CN, CN |
| 38 | H | $CH_2CH_2C_6H_5$ | $CH_2CH_2OH$ | CN, $CONHC_4H_9-n$ |
| 39 | H | $CH_2CH_2Cl$ | $CH_2CH_2CN$ | CN, $CO_2CH_3$ |
| 40 | H | 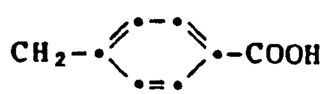 | 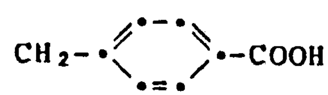 | CN, CN |
| 41 | H | 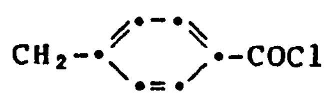 | 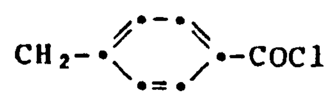 | CN, CN |
| 42 | H | $-CH_2CH_2SO_2CH_2CH_2-$ | | CN, $CO_2CH_3$ |

TABLE 1 (continued)

| Example No. | $(R)_n$ | $R_1$ | $R_2$ | P, Q |
|---|---|---|---|---|
| 43 | H | $-CH_2CH_2N(SO_2CH_3)CH_2CH_2-$ | | CN, $CO_2CH_3$ |
| 44 | $3-CH_3$ | $-CH_2CH_2-OCH_2CH_2-$ | | CN, $CO_2CH_3$ |
| 45 | H | $-CH_2CH_2CH_2CH_2CH_2-$ | | CN, $CO_2CH_3$ |
| 46 | $3-CH_3$ | $C_2H_5$ | 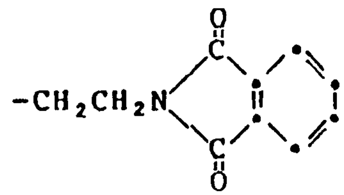 | CN, $CO_2CH_2$ |
| 47 | $3-CH_3$ | $C_2H_5$ | 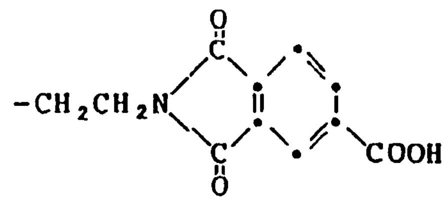 | CN, $CO_2CH_3$ |
| 48 | $3-CH_3$ | $C_2H_5$ | $-CH_2CH_2N(C_6H_5)SO_2CH_3$ | CN, $CO_2CH_3$ |
| 49 | $3-CH_3$ | $C_2H_5$ | $-CH_2CH_2OC_6H_5$ | CN, $CO_2CH_3$ |
| 50 | $3-CH_3$ | $C_2H_5$ | 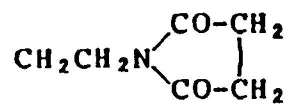 | CN, $CO_2CH_3$ |
| 51 | H | $CH_2CH_2OC_2H_5$ | $CH_2CH_2OC_2H_5$ | CN, $CO_2CH_3$ |
| 52 | $3-CH_3$ | $CH_2CH_3$ | $CH_2CH_2OCH_2CH_2OC_2H_5$ | CN, $CO_2CH_3$ |
| 53 | $3-CH_3$ | $CH_2CH_3$ | 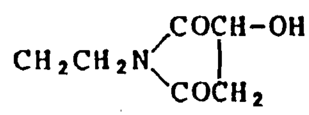 | CN, $CO_2CH_3$ |
| 54 | $3-CH_3$ | $CH_2CH_3$ | 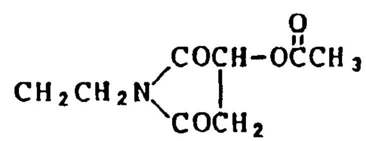 | CN, $CO_2CH_3$ |

TABLE 1 (continued)

| Example No. | (R)n | R1 | R2 | P, Q |
|---|---|---|---|---|
| 55 | 3-$CH_3$ | $CH_3$ | 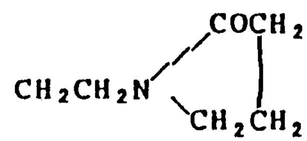 | CN, $CO_2CH_3$ |
| 56 | 3-$CH_3$ | $CH_2C_6H_5$ | 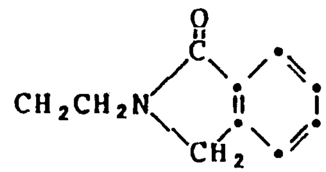 | CN, $CO_2CH_3$ |
| 57 | H | $CH_2C_6H_{11}$ | $C_2H_5$ | CN, $CO_2CH_3$ |
| 58 | 3-$CH_3$ | $C_2H_5$ | $CH_2CH_2CH_2SO_2CH_3$ | CN, $CO_2CH_3$ |
| 59 | 3-$CH_3$ | $C_2H_5$ | $CH_2CH_2O\overset{O}{\overset{\|}{C}}OC_2H_3$ | CN, $CO_2CH_3$ |
| 60 | 3-$CH_3$ | $-CH_2CH{=}CH_2$ | $-CH_2CH{=}CH_2$ | CN, $CO_2CH_3$ |
| 61 | 3-$CH_3$ | $-C_2H_5$ | 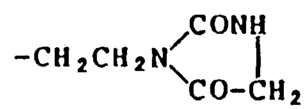 | CN, COOH |
| 62 | 3-$CH_3$ | $-C_2H_5$ | 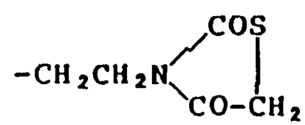 | CN, $CO_2CH(CH_3)_2$ |
| 63 | 3-$CH_3$ | $-C_2H_5$ | 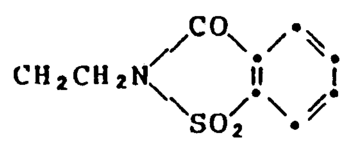 | $CO_2CH_3$, $CO_2CH_3$ |
| 64 | 3-$CH_3$ | $-C_2H_5$ | 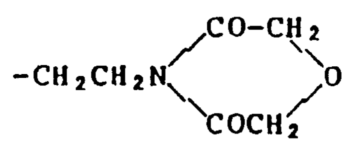 | $CO_2CH_3$, CN |

TABLE 1 (continued)

| Example No. | $(R)_n$ | $R_1$ | $R_2$ | P, Q |
|---|---|---|---|---|
| 65 | $3-CH_3$ | $-C_2H_5$ | $-CH_2CH_2SO_2CH=CH_2$ | $CO_2CH_3$, CN |
| 66 | $3-CH_3$ | $-C_2H_5$ | $-CH_2CH_2SO_2CH_2CH_2SC_6H_5$ | $CO_2CH_3$, CN |
| 67 | $3-CH_3$ | $-C_2H_5$ | $-CH_2CH_2S-$ 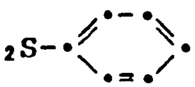 $-Cl$ | $CO_2CH_3$, CN |
| 68 | $3-CH_3$ | $-C_2H_5$ | $-CH_2CH_2SC_4H_{9}$ n | $CO_2CH_3$, CN |
| 69 | $3-CH_3$ | $-C_2H_5$ | $-CH_2CH_2N(C_2H_4CN)SO_2CH_3$ | $CO_2CH_3$, CN |
| 70 | $3-CH_3$ | $-C_2H_5$ | $-CH_2CH_2CONH_2$ | $CO_2CH_3$, CN |
| 71 | $3-CH_3$ | $-C_2H_5$ | $-CH_2CH_2CON(C_2H_5)_2$ | $CO_2CH_3$, CN |
| 72 | $3-CH_3$ | $-C_2H_5$ | $-CH_2CH(OCOCH_3)CH_2OCOCH_3$ | CN, CN |
| 73 | $3-CH_3$ | $-C_2H_5$ | $-CH_2CH_2O\overset{O}{\overset{\|}{C}}C_6H_5$ | CN, CN |
| 74 | $3-CH_3$ | $-C_2H_5$ | $-CH_2CH_2O\overset{O}{\overset{\|}{C}}C_6H_{11}$ | CN, CN |
| 75 | $3-CH_3$ | $-C_2H_5$ | $-CH_2CH_2SO_2N(C_2H_5)_2$ | CN, $CO_2CH_3$ |
| 76 | $3-CH_3$ | $-C_2H_5$ | $-CH_2CH_2SO_2N(CH_3)C_6H_5$ | CN, $CO_2CH_3$ |
| 77 | $3-CH_3$ | $-C_2H_5$ | $-CH_2CHS_2-$ 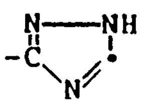 | CN, $CO_2CH_3$ |
| 78 | $3-CH_3$ | $-C_2H_5$ | $-CH_2CH_2-S-$ 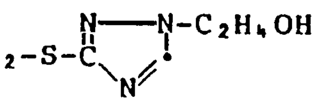 | CN, $CO_2CH_2C_6H_5$ |
| 79 | $3-CH_3$ | $-C_2H_5$ | $-CH_2CH_2S-$ 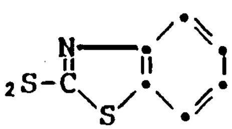 | CN, $CO_2CH_3$ |

TABLE 1 (continued)

| Example No. | $(R)_n$ | $R_1$ | $R_2$ | P, Q |
|---|---|---|---|---|
| 80 | $3-CH_3$ | $-C_2H_5$ | $-CH_2CH_2SO_2-$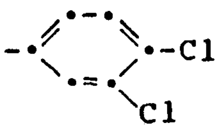 | CN, $CO_2CH_3$ |
| 81 | $3-CH_3$ | $-C_2H_5$ | $-CH_2CH_2S-$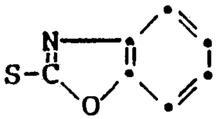 | CN, $CO_2CH_3$ |
| 82 | $3-CH_3$ | $-C_2H_5$ | $-CH_2CH_2S-$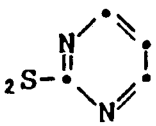 | CN, $CO_2CH_3$ |
| 83 | $3-CH_3$ | $-C_2H_5$ | $-CH(CH_3)CH_2CO_2CH_3$ | CN, $CO_2CH_3$ |
| 84 | $3-CH_3$ | $-C_2H_5$ | $-CH(CH_3)CH_2CO_2C_2H_5$ | CN, 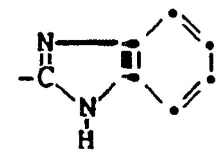 |
| 85 | $3-CH_3$ | $-C_2H_5$ | $-CH_2CH_2OCH_2CH_2OH$ | CN, 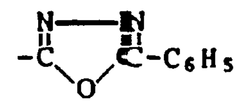 |
| 86 | $3-CH_3$ | $-C_2H_5$ | $-CH_2CH_2O\overset{O}{\overset{\parallel}{C}}CH_2C_6H_5$ | CN, CONH-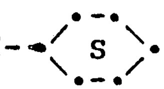 |
| 87 | $3-CH_3$ | $-C_2H_5$ | $-CH_2CH_2O\overset{O}{\overset{\parallel}{C}}CH_2OC_6H_5$ | CN, $CO_2CH_3$ |
| 88 | $3-CH_3$ | $-C_2H_5$ | $-CH_2CH(C_6H_5)O\overset{O}{\overset{\parallel}{C}}CH_3$ | $C_6H_5$, CN |
| 89 | $3-CH_3$ | $-C_2H_5$ | $-CH_2CH(OCOCH_3)CH_2Cl$ | $CO_2CH_3$, CN |
| 90 | $3-CH_3$ | $-C_2H_5$ | $-CH_2CH_2O\overset{O}{\overset{\parallel}{C}}H_2Cl$ | $CO_2CH_3$, CN |
| 91 | $3-CH_3$ | $-C_2H_5$ | $-CH_2CH_2O\overset{O}{\overset{\parallel}{C}}CH_2OCH_3$ | $CO_2CH_3$, CN |

TABLE 2

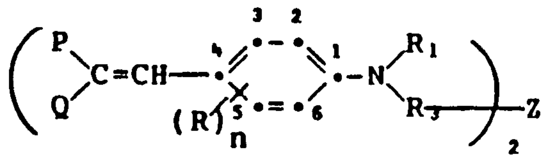

| Example No. | (R)n | $R_1$ | $R_3$ | Z | P, Q |
|---|---|---|---|---|---|
| 92 | H | $C_2H_5$ | $-CH_2CH_2-$ | $-SO_2-$ | CN, $CO_2CH_3$ |
| 93 | $3-CH_3$ | $C_2H_5$ | $-CH_2CH_2-$ | $-SO_2-$ | CN, $CO_2CH_3$ |
| 94 | $3-CH_3$ | $C_2H_5$ | $-CH_2CH_2-$ | -O- | CN, $CO_2CH_3$ |
| 95 | $3-CH_3$ | $C_2H_5$ | $-CH_2CH_2O-$ | $-CH_2CH_2-$ | CN, $CO_2CH_3$ |
| 96 | $3-CH_3$ | $C_2H_5$ | $-CH_2CH_2-$ | $-O\overset{O}{\overset{\|}{C}}O-$ | CN, $CO_2CH_3$ |
| 97 | $3-CH_3$ | $C_2H_5$ | $-CH_2-$ | 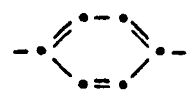 | CN, $CO_2CH_3$ |
| 98 | $3-CH_3$ | $C_2H_5$ | $-CH_2CH_2-$ | -S-S- | CN, $CO_2CH_3$ |
| 99 | $3-CH_3$ | $C_2H_5$ | $-CH_2CH_2-$ | $-O\overset{O}{\overset{\|}{C}}CH_2CH_2\overset{O}{\overset{\|}{C}}O-$ | CN, $CO_2CH_3$ |
| 100 | $3-CH_3$ | $C_2H_5$ | $-CH_2CH_2-$ | $-O\overset{O}{\overset{\|}{C}}(CH_2)_4\overset{O}{\overset{\|}{C}}O-$ | CN, $CO_2CH_3$ |
| 101 | $3-CH_3$ | $C_2H_5$ | $-CH_2CH_2-$ | $-NH\overset{O}{\overset{\|}{C}}(CH_2)_6\overset{O}{\overset{\|}{C}}NH-$ | CN, $CO_2CH_3$ |

TABLE 2 (continued)

| Example No. | (R)n | $R_1$ | $R_3$ | Z | P, Q |
|---|---|---|---|---|---|
| 102 | 3-$CH_3$ | $C_2H_5$ | $-CH_2CH_2-$ | 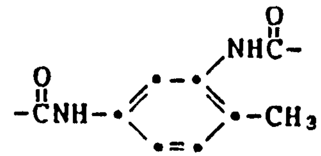 | CN, $CO_2CH_3$ |
| 103 | 3-$CH_3$ | $C_2H_5$ | $-CH_2CH_2-$ | 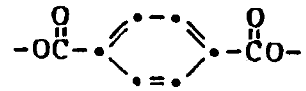 | CN, $CO_2CH_3$ |
| 104 | H | $C_2H_5$ | $-CH_2CH_2-$ | 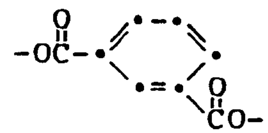 | CN, $CO_2CH_3$ |
| 105 | H | $CH_2CH_2CN$ | $-CH_2CH_2-$ | $-O\overset{O}{\overset{\|}{C}}O-$ | CN, $CO_2CH_3$ |
| 106 | H | $CH_2CH_2CN$ | $-CH_2CH_2-$ | $-O\overset{O}{\overset{\|}{C}}-$ | CN, $CO_2CH_3$ |
| 107 | H | $CH_2CH_2CN$ | $-CH_2CH_2-$ | $-O\overset{O}{\overset{\|}{C}}(CH_2)_4\overset{O}{\overset{\|}{C}}O-$ | CN, CN |
| 108 | H | $CH_2CH_2CN$ | $-CH_2CH_2OCH_2CH_2-$ | 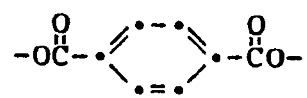 | CN, CN |
| 109 | H | $CH_2CH_2CN$ | $-CH_2CH_2-$ | 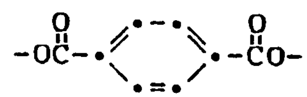 | CN, $SO_2CH_3$ |
| 110 | H | $CH_2CH_2CN$ | $-CH_2CH_2-$ | 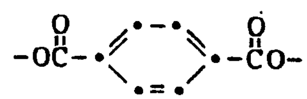 | CN, $SO_2C_6H_5$ |
| 111 | H | $CH_2CH_2O\overset{O}{\overset{\|}{C}}CH_3$ | $-CH_2CH_2-$ | 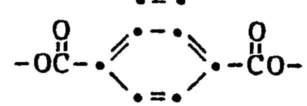 | CN, $CONHC_6H_5$ |

TABLE 2 (continued)

| Example No. | (R)n | $R_1$ | $R_3$ | Z | P, Q |
|---|---|---|---|---|---|
| 112 | 2,5-diOCH$_3$ | $C_2H_5$ | $-CH_2CH_2-$ | 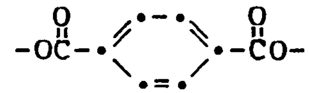 | 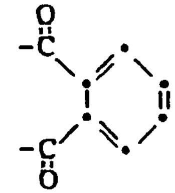 |
| 113 | 3-Cl | $C_2H_5$ | $-CH_2CH_2-$ | -S- | CN, $CO_2C_6H_5$ |
| 114 | 2-OCH$_3$, 5-Cl | $C_2H_5$ | $-CH_2CH_2-$ | $-O\overset{O}{\overset{\|}{C}}CH_2CH_2CH_2\overset{O}{\overset{\|}{C}}O-$ | CN, $CO_2C_2H_4CN$ |
| 115 | 3-CH$_3$ | $C_6H_5$ | $-CH_2CH_2-$ | $-O\overset{O}{\overset{\|}{C}}CH_2CH_2CH_2\overset{O}{\overset{\|}{C}}O-$ | CN, $CO_2CH_3$ |
| 116 | 3-CH$_3$ | $C_6H_{11}$ | $-CH_2CH(CH_3)-$ | $-O\overset{O}{\overset{\|}{C}}CH_2CH_2CH_2\overset{O}{\overset{\|}{C}}O-$ | CN, $CO_2CH_3$ |
| 117 | 3-CH$_3$ | $CH_2CH_2C_6H_5$ | $-CH_2-$ | 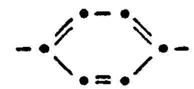 | CN, $CO_2CH_3$ |
| 118 | 3-CH$_3$ | $CH_2CH_2OC_2H_5$ | $-CH_2CH_2CH_2-$ | 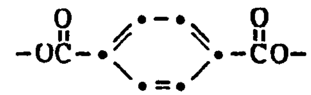 | CN, $CO_2CH_3$ |
| 119 | 3-CH$_3$ | $CH_2CH_2OC_6H_5$ | $-CH_2CH_2CH_2CH_2-$ | 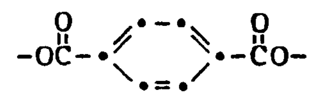 | CN, $CO_2CH_3$ |
| 120 | 3-CH$_3$ | 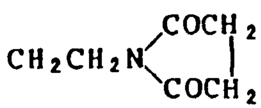 | $-CH_2CH_2-$ | 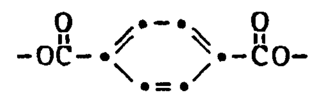 | CN, 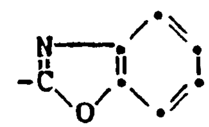 |
| 121 | 3-CH$_3$ | $CH_2CH_2CH_2CH_3$ | $-CH_2CH_2-$ | 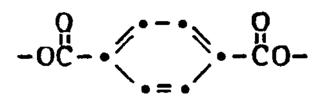 | CN, 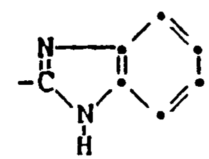 |
| 122 | 3-CH$_3$ | $CH_2CH_2OH$ | $-CH_2CH_2-$ | $-SO_2-$ | CN, CN |

TABLE 2 (continued)

| Example No. | (R)n | $R_1$ | $R_3$ | Z | P, Q |
|---|---|---|---|---|---|
| 123 | $3-CH_3$ | $CH_2CH_2CO_2CH_3$ | $-CH_2CH_2-$ | $-SO_2-$ | CN, CN |
| 124 | $3-CH_3$ | $CH_2CH_2Cl$ | $-CH_2CH_2-$ | $-O\overset{\overset{O}{\|}}{C}(CH_2)_4\overset{\overset{O}{\|}}{C}O-$ | CN, $CO_2CH_3$ |
| 125 | $3-CH_3$ | $C_2H_5$ | $-CH_2CH_2-$ | single bond | $-O\overset{\overset{O}{\|}}{C}-\overset{\overset{CN}{\|}}{C}=\overset{\overset{C_6H_5}{\|}}{C}-$ |
| 126 | $3-CH_3$ | $C_2H_5$ | $-CH_2CH_2-$ | $-O\overset{\overset{O}{\|}}{C}(CH_2)_4\overset{\overset{O}{\|}}{C}O-$ | $-O\overset{\overset{O}{\|}}{C}-\overset{\overset{CN}{\|}}{C}=\overset{\overset{C_6H_5}{\|}}{C}-$ |
| 127 | $3-CH_3$ | $C_2H_5$ | $-CH_2CH_2-$ | $-O\overset{\overset{O}{\|}}{C}(CH_2)_4\overset{\overset{O}{\|}}{C}O-$ | $CO_2C_2H_5$, $CO_2C_2H_5$ |
| 128 | $3-CH_3$ | $C_2H_5$ | $-CH_2CH_2-$ | $-O\overset{\overset{O}{\|}}{C}(CH_2)_4\overset{\overset{O}{\|}}{C}O-$ | $CO_2C_2H_5$, $SO_2CH_3$ |
| 129 | $3-CH_3$ | $C_2H_5$ | $-CH_2CH_2-$ | $-O\overset{\overset{O}{\|}}{C}(CH_2)_4\overset{\overset{O}{\|}}{C}O-$ | CN, $C_6H_5$ |
| 130 | $3-CH_3$ | $CH(CH_3)CH_2CO_2CH_3$ | $-CH_2CH_2-$ | $-O\overset{\overset{O}{\|}}{C}(CH_2)_4\overset{\overset{O}{\|}}{C}O-$ | CN, $CONH_2$ |
| 131 | $3-CH_3$ | $C_2H_5$ | $-CH_2CH_2-$ | $-O\overset{\overset{O}{\|}}{C}(CH_2)_4\overset{\overset{O}{\|}}{C}O-$ | $COC_6H_5$, $COC_6H_5$ |
| 132 | $3-CH_3$ | $C_2H_5$ | $-CH_2CH_2-$ | $-O\overset{\overset{O}{\|}}{C}(CH_2)_4\overset{\overset{O}{\|}}{C}O-$ | $CO_2C_6H_5$, $CO_2C_6H_5$ |
| 133 | $3-CH_3$ | $C_2H_5$ | $-CH_2CH_2-$ | $-O\overset{\overset{O}{\|}}{C}(CH_2)_4\overset{\overset{O}{\|}}{C}O-$ | CN, $CONHC_2H_4OH$ |
| 134 | $3-CH_3$ | $C_2H_5$ | $-CH_2CH_2-$ | $-O\overset{\overset{O}{\|}}{C}(CH_2)_4\overset{\overset{O}{\|}}{C}O-$ | CN, $CONHC_4H_9$-n |
| 135 | $3-CH_3$ | $C_2H_5$ | $-CH_2CH_2-$ | $-O\overset{\overset{O}{\|}}{C}(CH_2)_4\overset{\overset{O}{\|}}{C}O-$ | CN, $CONHC_6H_{11}$ |
| 136 | $3-CH_3$ | $C_2H_5$ | $-CH_2CH_2-$ | $-O\overset{\overset{O}{\|}}{C}(CH_2)_4\overset{\overset{O}{\|}}{C}O-$ | $CONH_2$, $CONH_2$ |

TABLE 3

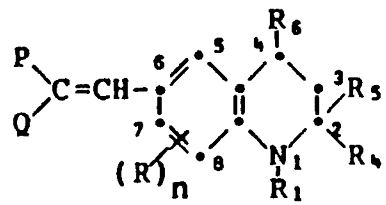

| Example No. | $(R)_n$ | $R_1$ | $R_4$, $R_5$ | $R_6$ | P, Q |
|---|---|---|---|---|---|
| 137 | H | $C_2H_5$ | $CH_3$, $CH_3$ | $CH_3$ | CN, $CO_2CH_3$ |
| 138 | H | $CH_2CH_2OH$ | $CH_3$, $CH_3$ | $CH_3$ | CN, $SO_2CH_3$ |
| 139 | H | $CH_2CH_2OH$ | $CH_3$, $CH_3$ | $CH_3$ | $CO_2CH_3$, $CO_2CH_3$ |
| 140 | H | $CH_2CH_2OH$ | $CH_3$, $CH_3$ | $CH_3$ | CN, $SO_2C_6H_5$ |
| 141 | 7-$CH_3$ | $CH_2CH_2OH$ | $CH_3$, $CH_3$ | $CH_3$ | CN, $CO_2C_2H_5$ |
| 142 | 7-$CH_3$ | $C_2H_4O\overset{O}{\overset{\|}{C}}CH_3$ | $CH_3$, $CH_3$ | $CH_3$ | CN, CN |
| 143 | 7-$CH_3$ | $C_2H_4O\overset{O}{\overset{\|}{C}}NH-$ 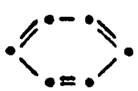 | $CH_3$, $CH_3$ | $CH_3$ | CN, CN |
| 144 | 7-$CH_3$ | $C_2H_4CN$ | $CH_3$, $CH_3$ | $CH_3$ | CN, $CO_2CH_3$ |
| 145 | 7-$CH_3$ | $C_2H_4OH$ | $CH_3$, $CH_3$ | $CH_3$ | CN, $CONHC_6H_5$ |
| 146 | 7-$CH_3$ | $C_2H_4OH$ | $CH_3$, $CH_3$ | $CH_3$ | CN, 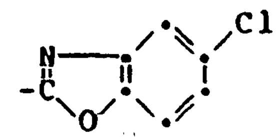 |
| 147 | 7-$CH_3$ | $C_2H_4OH$ | $CH_3$, $CH_3$ | $CH_3$ | $-O\overset{O}{\overset{\|}{C}}-\overset{CN}{\overset{\|}{C}}=\overset{C_6H_5}{\overset{\|}{C}}-$ |

TABLE 3 (continued)

| Example No. | $(R)_n$ | $R_1$ | $R_4$, $R_5$ | $R_6$ | P, Q |
|---|---|---|---|---|---|
| 148 | 7-$CH_3$ | $C_2H_4OH$ | $CH_3$, $CH_3$ | $CH_3$ | $-O\overset{O}{\overset{\|}{C}}-\overset{COOH}{\overset{\mid}{C}}=\overset{C_6H_5}{\overset{\mid}{C}}-$ |
| 149 | 7-$CH_3$ | $C_2H_4OH$ | $CH_3$, $CH_3$ | $CH_3$ | CN, $CONHC_2H_4OH$ |
| 150 | 7-$CH_3$ | $C_2H_4OH$ | $CH_3$, $CH_3$ | $CH_3$ | $COC(CH_3)_2$, CN |
| 151 | H | $C_2H_4OH$ | $CH_3$, $CH_3$ | $CH_3$ | CN, $CO_2CH_3$ |
| 152 | H | $C_2H_4O\overset{O}{\overset{\|}{C}}C_2H_5$ | $CH_3$, $CH_3$ | $CH_3$ | CN, $CO_2C_2H_5$ |
| 153 | H | $C_2H_4O\overset{O}{\overset{\|}{C}}C_6H_5$ | $CH_3$, $CH_3$ | $CH_3$ | CN, $CONH_2$ |
| 154 | H | $C_2H_4O\overset{O}{\overset{\|}{C}}OC_2H_5$ | $CH_3$, $CH_3$ | $CH_3$ | CN, $CO_2C_6H_5$ |
| 155 | H | $CH_2C_6H_5$ | $CH_3$, $CH_3$ | $CH_3$ | CN, $CO_2CH_3$ |
| 156 | H | 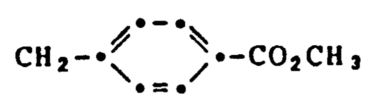 | $CH_3$, $CH_3$ | $CH_3$ | CN, $CO_2CH_3$ |
| 157 | H | $CH_2C_6H_{11}$ | $CH_3$, $CH_3$ | $CH_3$ | CN, $CO_2C_2H_4CN$ |
| 158 | 7-$CH_3$ | $C_4H_9$-n | H, $CH_3$ | H | CN, $CO_2CH_3$ |
| 159 | 7-$CH_3$ | $CH_2CH_2OCH_2CH_2OH$ | H, $CH_3$ | H | CN, CN |
| 160 | 7-$CH_3$ | $CH_2CH_2O\overset{O}{\overset{\|}{C}}CH_3$ | H, $CH_3$ | H | CN, $SO_2CH_3$ |
| 161 | 7-$CH_3$ | $CH_2CH_2N(C_6H_5)SO_2CH_3$ | H, $CH_3$ | H | CN, $CO_2CH_3$ |
| 162 | 7-$CH_3$ | 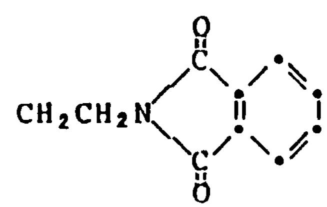 | H, $CH_3$ | H | CN, $CO_2CH_3$ |

TABLE 3 (continued)

| Example No. | $(R)_n$ | $R_1$ | $R_4$, $R_5$ | $R_6$ | P, Q |
|---|---|---|---|---|---|
| 163 | 7-$CH_3$ | 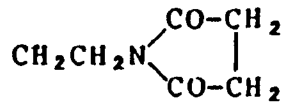 | H, $CH_3$ | H | CN, $CO_2CH_3$ |
| 164 | 7-$CH_3$ | 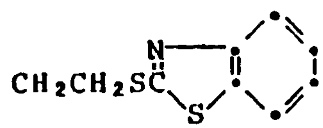 | H, $CH_3$ | H | CN, $CO_2CH_3$ |
| 165 | 5-$CH_3$, 8-$CH_3O$ | $CH_2CH_2O\overset{O}{\overset{\|}{C}}CH_3$ | H, $CH_3$ | H | CN, $CO_2CH_3$ |
| 166 | 5,8-di-$OCH_3$ | $CH_2CH_2O\overset{O}{\overset{\|}{C}}CH_3$ | H, $CH_3$ | H | CN, $CO_2CH_3$ |
| 167 | 7-$OC_2H_5$ | $CH_2CH_2O\overset{O}{\overset{\|}{C}}CH_3$ | H, $CH_3$ | H | CN, $CONHC_6H_{11}$ |
| 168 | 7-$CH_3$ | 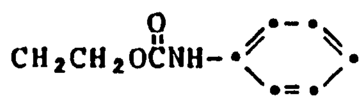 | H, $CH_3$ | H | 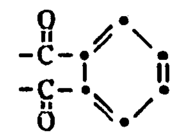 |
| 169 | 7-$CH_3$ | $CH_2CH_2CO_2CH_3$ | H, $CH_3$ | H | CN, CN |
| 170 | 7-$CH_3$ | $CH_2CH_2COOH$ | H, $CH_3$ | H | CN, COOH |
| 171 | 7-$CH_3$ | $CH_2CH_2CON(C_2H_5)_2$ | H, $CH_3$ | H | $CONH_2$, $CO_2CH_3$ |
| 172 | H | $CH_2CH(OH)CH_3$ | H, H | H | CN, CN |
| 173 | H | $CH_2CH(C_6H_5)OH$ | H, H | H | CN, CN |
| 174 | 7-$CH_3$ | $C_6H_5$ | H, $CH_3$ | $CH_3$ | CN, $CO_2CH_3$ |
| 175 | 7-$CH_3$ | 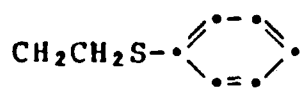 | H, $CH_3$ | H | CN, $CO_2C_2H_4OH$ |
| 176 | 7-$CH_3$ | $CH_2CH_2OC_6H_5$ | H, $CH_3$ | H | CN, $CO_2C_2H_4Cl$ |
| 177 | 7-$CH_3$ | $CH_2CH_2CH_2SO_2CH_3$ | H, $CH_3$ | H | CN, $CO_2C_4H_9$-n |

TABLE 3 (continued)

| Example No. | $(R)_n$ | $R_1$ | $R_4$, $R_5$ | $R_6$ | P, Q |
|---|---|---|---|---|---|
| 178 | 7-Br | 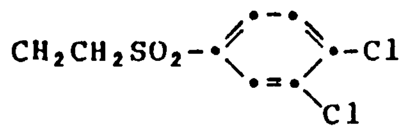 | $CH_3$, $CH_3$ | $CH_3$ | $CO_2C_2H_5$, $CO_2C_2H_5$ |
| 179 | 7-Cl | 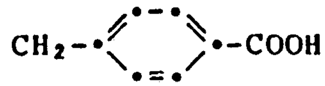 | $CH_3$, $CH_3$ | $CH_3$ | $COC_6H_5$, $SO_2CH_3$ |
| 180 | 7-$OCH_3$ | $CH_2CH(O\overset{O}{\overset{\|}{C}}CH_3)CH_2O\overset{O}{\overset{\|}{C}}CH_3$ | $CH_3$, $CH_3$ | $CH_3$ | CN, CN |
| 181 | 7-$CH_3$ | $CH_2CH(OH)CH_2OH$ | $CH_3$, H | H | CN, CN |
| 182 | 7-$CH_3$ | $CH_2CH(OH)CH_2OH$ | $CH_3$, H | H | 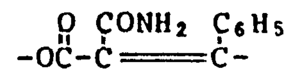 |
| 183 | 7-$CH_3$ | $CH_2CH{=}CH_2$ | $CH_3$, H | H | CN, $CO_2CH_3$ |
| 184 | 7-$CH_3$ | 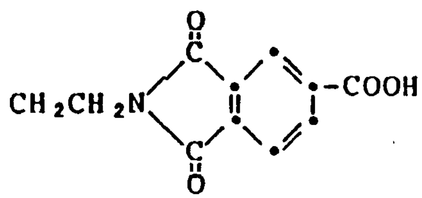 | $CH_3$, H | H | CN, $CO_2CH_3$ |
| 185 | 7-$CH_3$ | 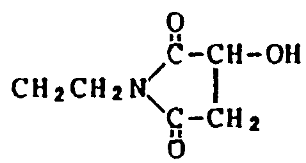 | $CH_3$, H | H | CN, $CO_2CH_3$ |

TABLE 4

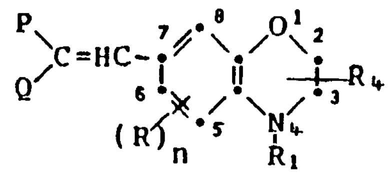

| Example No. | (R)n | $R_1$ | $R_4$ | P, Q |
|---|---|---|---|---|
| 186 | H | $C_2H_4OH$ | H | CN, CN |
| 187 | H | $C_2H_4OH$ | H | CN, $SO_2CH_3$ |
| 188 | H | $C_2H_4OH$ | H | CN, $SO_2C_6H_5$ |
| 189 | H | $C_2H_4OH$ | H | CN, $CONHC_6H_5$ |
| 190 | H | $C_2H_4OH$ | H | CN, $CONHC_2H_4OH$ |
| 191 | H | $CH_2CH_2O\overset{O}{\overset{\|}{C}}CH_3$ | H | CN, CN |
| 192 | H | $CH_2CH_2O\overset{O}{\overset{\|}{C}}CH_3$ | H | CN, $SO_2CH_3$ |
| 193 | 6-$CH_3$ | $CH_2CH_2O\overset{O}{\overset{\|}{C}}CH_3$ | H | CN, $CO_2CH_3$ |
| 194 | 6-$CH_3$ | $CH_2CH_2O\overset{O}{\overset{\|}{C}}-C_6H_5$ | 3-$CH_3$ | CN, $CO_2CH_3$ |
| 195 | 6-$CH_3$ | $CH_2CH_2O\overset{O}{\overset{\|}{C}}CH_3$ | 3-$CH_3$ | $-O\overset{O}{\overset{\|}{C}}-\overset{CN}{\overset{\mid}{C}}=C\diagup^{C_6H_5}$ |

TABLE 4 (continued)

| Example No. | (R)n | R1 | R4 | P, Q |
|---|---|---|---|---|
| 196 | $6-CH_3$ | $CH_2CH(OH)CH_3$ | $3-CH_3$ | 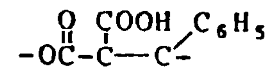 |
| 197 | $6-CH_3$ | $CH_2CH_2OCH_2CH_2O\overset{O}{\overset{\|}{C}}CH_3$ | $3-CH_3$ | 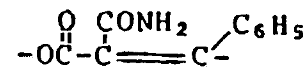 |
| 198 | $6-CH_3$ | $CH_2C_6H_5$ | $3-CH_3$ | $CN$, $CO_2CH_3$ |
| 199 | $6-CH_3$ | 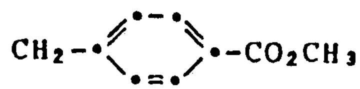 | $3-CH_3$ | $CN$, $CN$ |
| 200 | $6-CH_3$ | $C_6H_5$ | $3-CH_3$ | $CN$, $CO_2CH_3$ |
| 201 | $6-CH_3$ | 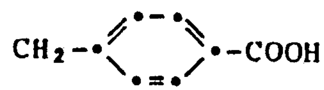 | $3-CH_3$ | $CN$, $CO_2CH_3$ |
| 202 | $6-CH_3$ | 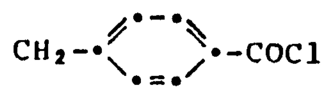 | $3-CH$ | $CN$, $CN$ |
| 203 | $6-CH_3$ | $CH_2CH_2O\overset{O}{\overset{\|}{C}}CH_3$ | $3-CH_3$ | 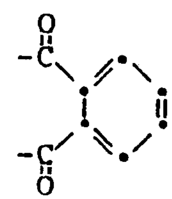 |
| 204 | $6-CH_3$ | $CH_2CH_2CN$ | $3-CH_3$ | $CN$, $CO_2CH_3$ |
| 205 | $6-CH_3$ | $CH_2CH_2N(CH_3)SO_2CH_3$ | $3-CH_3$ | $CN$, $CO_2CH_3$ |
| 206 | $6-CH_3$ | $CH_2CH_2O\overset{O}{\overset{\|}{C}}NHC_6H_5$ | $3-CH_3$ | $CN$, $CO_2CH_3$ |
| 207 | $6-CH_3$ | $CH_2CH_2Cl$ | $3-CH_3$ | $CN$, $CO_2CH_3$ |
| 208 | $6-CH_3$ | $CH_2CH_2OC_6H_5$ | $3-CH_3$ | $CN$, $CO_2CH_3$ |
| 209 | $6-CH_3$ | $CH_2CH_2SC_6H_5$ | $3-CH_3$ | $CN$, $CO_2CH_3$ |

TABLE 4 (continued)

| Example No. | (R)n | R1 | R4 | P, Q |
|---|---|---|---|---|
| 210 | 6-$CH_3$ | 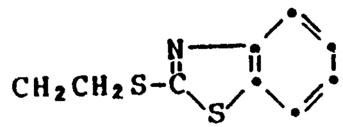 | 3-$CH_3$ | CN, COOH |
| 211 | 6-$CH_3$ | 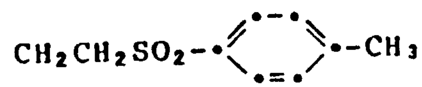 | 3-$CH_3$ | CN, $CO_2CH_3$ |
| 212 | 6-$CH_3$ | 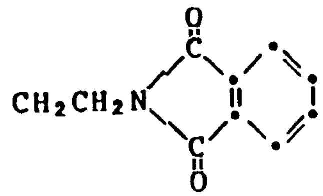 | 3-$CH_3$ | CN, $CO_2CH_3$ |
| 213 | 6-$CH_3$ | 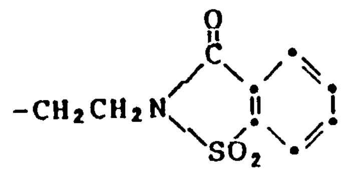 | 3-$CH_3$ | CN, $CO_2CH_3$ |
| 214 | 6-$CH_3$ | $-CH_2CH=CH_2$ | 3-$CH_3$ | CN, $CO_2CH_3$ |
| 215 | 6-$CH_3$ | $-CH_2CH_2OC_2H_4OH$ | 3-$CH_3$ | CN, CN |
| 216 | 6-$CH_3$ | $-CH_2CH_2SO_2CH=CH_2$ | 3-$CH_3$ | CN, $CO_2CH_3$ |
| 217 | 6-$CH_3$ | $-CH_2CH(OH)CH_2OH$ | 3-$CH_3$ | CN, $COC(CH_3)_2$ |
| 218 | 6-$CH_3$ | $-CH_2C_6H_{11}$ | 3-$CH_3$ | CN, $CO_2CH_3$ |
| 219 | 6-$CH_3$ | $-CH_2CH_2SO_2N(CH_3)_2$ | 3-$CH_3$ | CN, $CO_2CH_3$ |
| 220 | 6-$CH_3$ | $-CH_2CH_2CON(CH_3)C_6H_5$ | 3-$CH_3$ | CN, $CO_2CH_3$ |
| 221 | 6-$CH_3$ | $-CH_2CH_2O\overset{\overset{O}{\|}}{C}CH_2Cl$ | 3-$CH_3$ | CN, $CO_2CH_3$ |
| 222 | 6-$CH_3$ | $-CH_2CH_2O\overset{\overset{O}{\|}}{C}CH_3$ | 3-$CH_3$ | CN, 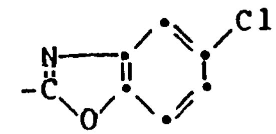 |

TABLE 4 (continued)

| Example No. | (R)n | R1 | R4 | P, Q |
|---|---|---|---|---|
| 223 | $6-CH_3$ | $-CH_2CH_2O\overset{O}{\overset{\|}{C}}CH_3$ | $3-CH_3$ | CN, 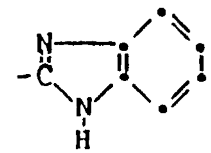 |
| 224 | $6-CH_3$ | $-CH_2CH_2O\overset{O}{\overset{\|}{C}}CH_3$ | $3-CH_3$ | CN, 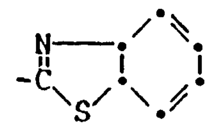 |
| 225 | $6-CH_3$ | $CH_2CH_2$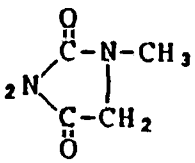 | $3-CH_3$ | CN, $CO_2CH_3$ |
| 226 | $6-CH_3$ | $CH_2CH_2$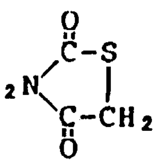 | $3-CH_3$ | CN, $CO_2CH_3$ |
| 227 | $6-CH_3$ | $CH_2CH_2-S-$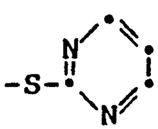 | $3-CH_3$ | CN, $CO_2CH_3$ |

TABLE 5

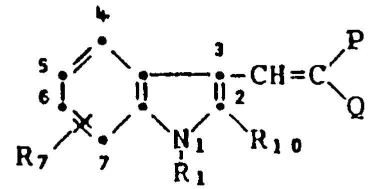

| Example No. | R7 | R1 | R10 | P, Q |
|---|---|---|---|---|
| 228 | H | $CH_3$ | $CH_3$ | CN, $CO_2CH_3$ |
| 229 | H | $CH_3$ | $C_6H_5$ | CN, $CO_2CH_3$ |
| 230 | H | H | $C_6H_5$ | CN, $CO_2C_2H_5$ |
| 231 | H | $CH_2CH_2O\overset{O}{\overset{\|}{C}}CH_3$ | $C_6H_5$ | CN, $SO_2CH_3$ |
| 232 | H | $CH_2CH_2O\overset{O}{\overset{\|}{C}}CH_3$ | $C_6H_5$ | CN, $SO_2C_6H_5$ |
| 233 | H | $CH_2CH_2O\overset{O}{\overset{\|}{C}}CH_3$ | $C_6H_5$ | CN, CN |
| 234 | H | $CH_2CH_2O\overset{O}{\overset{\|}{C}}CH_3$ | $C_6H_5$ | 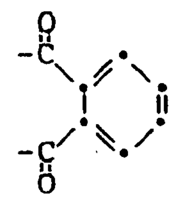 |
| 235 | H | $CH_2CH_2O\overset{O}{\overset{\|}{C}}CH_3$ | $C_6H_5$ | CN, $CONHC_6H_5$ |
| 236 | H | $CH_2CH_2OH$ | $C_6H_5$ | $-O\overset{O}{\overset{\|}{C}}-\overset{CN}{\overset{\|}{C}}=\overset{C_6H_5}{\overset{\|}{C}}-$ |

TABLE 5 (continued)

| Example No. | R7 | R1 | R10 | P, Q |
|---|---|---|---|---|
| 237 | H | $CH_2CH_2OH$ | $C_6H_5$ | CN, $CONH_2$ |
| 238 | H | $CH_2CH_2OH$ | $C_6H_5$ | CN, 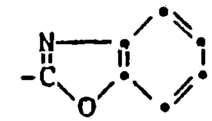 |
| 239 | H | $CH_2CH_2OH$ | $C_6H_5$ | CN, $CO_2C_6H_5$ |
| 240 | H | $CH_2CH_2OH$ | $C_6H_5$ | CN, $CONH_2$ |
| 241 | H | $CH_3$ | $C_6H_5$ | 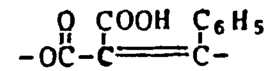 |
| 242 | H | $CH_3$ | 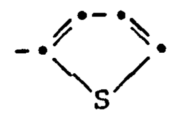 | CN, $CO_2CH_3$ |
| 243 | H | $CH_3$ | 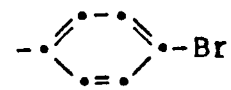 | CN, $CO_2CH_3$ |
| 244 | H | $CH_3$ | 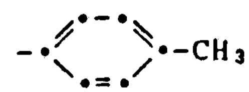 | CN, $CO_2C_2H_4OH$ |
| 245 | 5-$CH_3$ | $CH_3$ | $CH_3$ | CN, $CO_2CH_3$ |
| 246 | 5-Cl | $CH_3$ | $CH_3$ | CN, $CO_2CH_3$ |
| 247 | 5-$OCH_3$ | $CH_3$ | $CH_3$ | CN, $CO_2CH_3$ |
| 248 | H | $CH_2C_6H_5$ | $C_6H_5$ | CN, $CO_2CH_3$ |
| 249 | H | 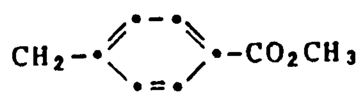 | $C_6H_5$ | CN, CN |
| 250 | H | 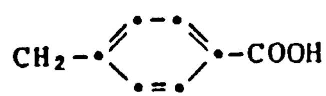 | $C_6H_5$ | CN, CN |
| 251 | H | $CH_2CH_2CN$ | $C_6H_5$ | CN, $CO_2CH_3$ |
| 252 | H | $CH_2CH_2CONH_2$ | $C_6H_5$ | CN, $CO_2CH_2CH_2CN$ |

TABLE 5 (continued)

| Example No. | $R_7$ | $R_1$ | $R_{10}$ | P, Q |
|---|---|---|---|---|
| 253 | H | $CH_2CH_2CH_2NH\overset{O}{\overset{\parallel}{C}}C_6H_5$ | $C_6H_5$ | CN, $CO_2CH_3$ |
| 254 | H | $CH_2CH_2CH_2NHSO_2C_6H_5$ | $C_6H_5$ | CN, $CO_2CH_3$ |
| 255 | H | $CH_2CH_2CH_2N(CH_3)SO_2CH_3$ | $C_6H_5$ | CN, $CO_2CH_3$ |
| 256 | H | $CH_2CH_2CH_2N(C_2H_4OH)SO_2CH_3$ | $C_6H_5$ | CN, $CO_2CH_3$ |
| 257 | H | $CH_3$ | H | CN, $CO_2CH_3$ |
| 258 | H | $CH_3$ | H | $CO_2CH_3$, 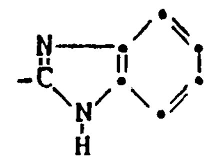 |
| 250 | H | $CH_3$ | H | CN, COOH |
| 260 | H | $CH_3$ | $C_6H_5$ | CN, $CO_2CH_2C_6H_5$ |
| 261 | H | $CH_3$ | $C_6H_4$-p-COOH | CN, $CO_2CH_2C_6H_{11}$ |
| 262 | H | $C_4H_9$-n | $C_6H_4$-p-$CO_2CH_3$ | CN, $CO_2CH_3$ |
| 263 | H | $CH_2CH_2CH_2$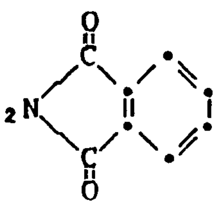 | $C_6H_5$ | CN, $CO_2CH_3$ |
| 264 | H | $CH_2CH_2CH_2$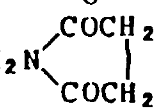 | $C_6H_5$ | CN, $CO_2CH_3$ |
| 265 | H | $CH_2CH_2CH_2$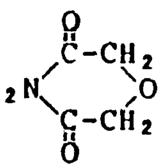 | $C_6H_5$ | CN, $CO_2CH_3$ |

TABLE 5 (continued)

| Example No. | R7 | R1 | R10 | P, Q |
|---|---|---|---|---|
| 266 | H | 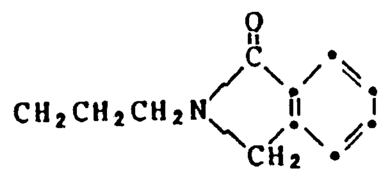 | $C_6H_5$ | CN, $CO_2CH_3$ |
| 267 | H | 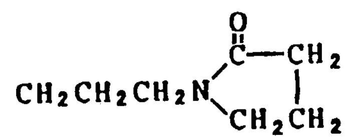 | $C_6H_5$ | CN, $CO_2CH_3$ |
| 268 | H | $C_6H_5$ | $C_6H_5$ | CN, $CO_2CH_3$ |

TABLE 6

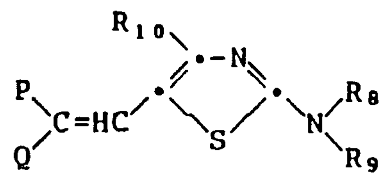

| Example No. | $R_8$ | $R_9$ | $R_{10}$ | P, Q |
|---|---|---|---|---|
| 269 | $CH_3$ | $CH_3$ | $CH_3$ | CN, $CO_2CH_3$ |
| 270 | $CH_3$ | $CH_3$ | $CH_3$ | CN, $CO_2C_2H_5$ |
| 271 | $CH_3$ | $CH_3$ | $C_6H_5$ | CN, $CO_2CH_3$ |
| 272 | $CH_3$ | $CH_3$ | $C_6H_5$ | CN, COOH |
| 273 | $CH_3$ | $CH_3$ | $C_6H_5$ | $CO_2C_2H_5$, $CO_2C_2H_5$ |
| 274 | $CH_3$ | $CH_3$ | $C_6H_5$ | CN, $CONHC_2H_4OH$ |
| 275 | $CH_3$ | $CH_2CH_2OH$ | $C_6H_5$ | CN, CN |
| 276 | $CH_3$ | $CH_2CH_2OH$ | $C_6H_5$ | CN, $SO_2CH_3$ |
| 277 | $CH_3$ | $CH_2CH_2OH$ | $C_6H_5$ | CN, $SO_2C_6H_5$ |
| 278 | $CH_3$ | $CH_2CH_2OH$ | $C_6H_5$ | CN, $CONHC_6H_5$ |
| 279 | $CH_3$ | $CH_2CH_2OH$ | $C_6H_5$ | 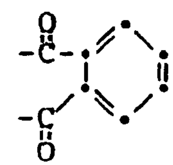 |
| 280 | $CH_3$ | $CH_2CH_2OH$ | $C_6H_5$ | 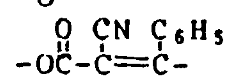 |

TABLE 6 (continued)

| Example No. | $R_8$ | $R_9$ | $R_{10}$ | P, Q |
|---|---|---|---|---|
| 281 | $CH_3$ | $CH_2CH_2O\overset{O}{\overset{\|}{C}}CH_3$ | $C_6H_5$ | CN, CN |
| 282 | $CH_3$ | $CH_2CH_2O\overset{O}{\overset{\|}{C}}OC_2H_5$ | $C_6H_5$ | CN, CN |
| 283 | $C_2H_5$ | $C_2H_5$ | $C_6H_5$ | CN, $CO_2CH_3$ |
| 284 | $C_2H_5$ | $C_6H_5$ | $C_6H_5$ | CN, $CO_2CH_2CH_2CN$ |
| 285 | $C_2H_5$ | 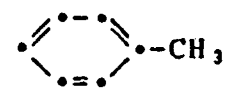 | $C_6H_5$ | CN, $CO_2CH_3$ |
| 286 | $C_2H_5$ | 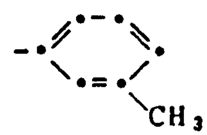 | $C_6H_5$ | CN, $CO_2CH_3$ |
| 287 | $C_2H_5$ | 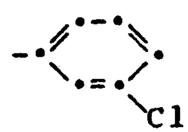 | $C_6H_5$ | CN, $CO_2CH_3$ |
| 288 | $CH_2CH_2OH$ | 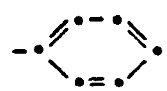 | $C_6H_5$ | CN, CN |
| 289 | $CH_3$ | $CH_3$ | H | CN, $CO_2CH_3$ |
| 290 | $CH_2CH_2OH$ | 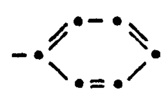 | $C_6H_5$ | -CN, 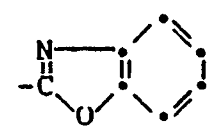 |
| 291 | $CH_2CH_2OH$ | 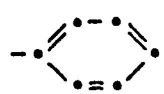 | $C_6H_5$ | -CN, 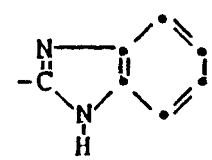 |

TABLE 6 (continued)

| Example No. | $R_8$ | $R_9$ | $R_{10}$ | P, Q |
|---|---|---|---|---|
| 292 | $CH_2CH_2OH$ | 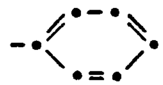 | $C_6H_5$ | CN, 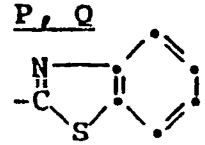 |
| 293 | $CH_2CH_2OH$ | 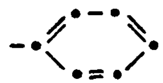 | $C_6H_5$ | CN, $SO_2$- 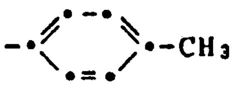 |
| 294 | $CH_2CH_2OH$ | 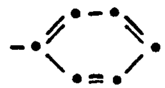 | $C_6H_5$ | CN, $SO_2$- 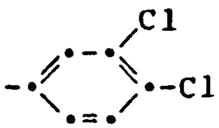 |
| 295 | $CH_2CH_2OH$ | 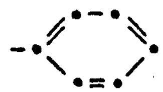 | $C_6H_5$ | CN, $SO_2$- 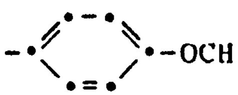 |
| 296 | $CH_3$ | $CH_2C_6H_5$ | $C_6H_5$ | CN, $CO_2CH_3$ |
| 297 | $CH_2C_6H_5$ | $CH_2C_6H_5$ | $C_6H_5$ | CN, $CO_2CH_3$ |
| 298 | $CH_3$ | $C_6H_{11}$ | $C_6H_5$ | CN, $CO_2CH_3$ |
| 299 | $C_4H_9$-n | $C_4H_9$-n | $C_6H_5$ | CN, $CO_2CH_3$ |
| 300 | $CH_3$ | $CH_3$ | 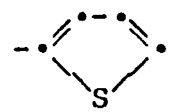 | CN, $CO_2CH_3$ |
| 301 | $CH_3$ | $C_6H_5$ | 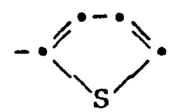 | CN, $CO_2CH_3$ |
| 302 | $CH_3$ | $C_6H_5$ | 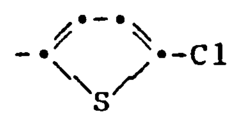 | CN, $CO_2CH_3$ |
| 303 | $CH_3$ | $C_6H_5$ | 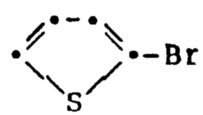 | CN, $CO_2CH_3$ |

TABLE 6 (continued)

| Example No. | R8 | R9 | R10 | P, Q |
|---|---|---|---|---|
| 304 | $CH_3$ | $C_6H_5$ | 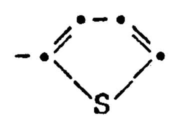 | 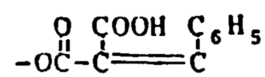 |
| 305 | $C_4H_9$-n | $C_6H_5$ | 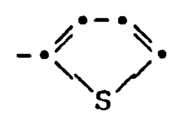 | 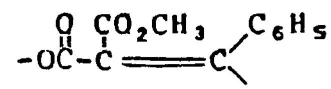 |

TABLE 7

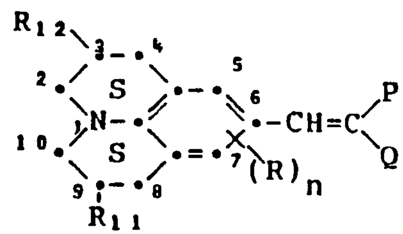

| Example No. | (R)n | $R_{11}$ | $R_{12}$ | P, Q |
|---|---|---|---|---|
| 306 | H | H | H | CN, $CO_2CH_3$ |
| 307 | H | H | H | CN, $CO_2CH_2CH_2CN$ |
| 308 | H | H | H | CN, $CO_2CH_2CH_2OH$ |
| 309 | H | H | H | CN, $CO_2CH_2CH_2C_6H_5$ |
| 310 | H | H | H | CN, $CO_2CH_2C_6H_{11}$ |
| 311 | H | H | H | $CO_2C_2H_5$, $CO_2C_2H_5$ |
| 312 | H | H | H | COOH, CN |
| 313 | H | H | H | 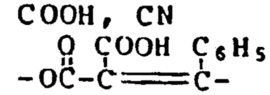 |
| 314 | 5-$CH_3$ | H | H | CN, $CO_2CH_3$ |
| 315 | 5-$OCH_3$ | H | H | CN, $CO_2CH_3$ |
| 316 | 5-Cl | H | H | CN, $CO_2CH_3$ |
| 317 | 5,7-di-$CH_3$ | H | H | CN, $CO_2CH_3$ |
| 318 | 5-$CH_3$ | OH | H | CN, CN |

TABLE 7 (continued)

| Example No. | (R)n | $R_{11}$ | $R_{12}$ | P, Q |
|---|---|---|---|---|
| 319 | 5-$CH_3$ | OH | H | 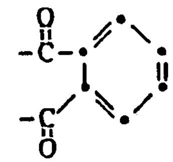 |
| 320 | 5-$CH_3$ | OH | H | CN, 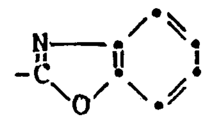 |
| 321 | 5-$CH_3$ | OH | H | CN, 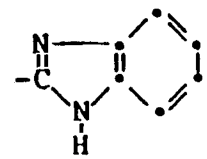 |
| 322 | 5-$CH_3$ | OH | H | CN, 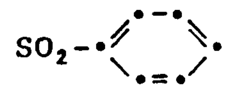 |
| 323 | 5-$CH_3$ | OH | H | CN, 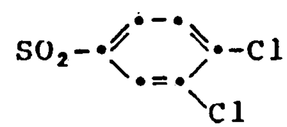 |
| 324 | 5-$CH_3$ | OH | H | CN, 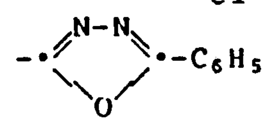 |
| 325 | 5-$CH_3$ | OH | H | 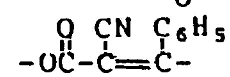 |
| 326 | 5-$CH_3$ | $O\overset{O}{\overset{\|}{C}}OC_2H_5$ | H | CN, CN |
| 327 | 5-$CH_3$ | $O\overset{O}{\overset{\|}{C}}C_6H_5$ | H | CN, $CO_2CH_3$ |
| 328 | 5-$CH_3$ | $O\overset{O}{\overset{\|}{C}}CH_2Cl$ | H | CN, $CO_2CH_3$ |

TABLE 7 (continued)

| Example No. | (R)n | $R_{11}$ | $R_{12}$ | P, Q |
|---|---|---|---|---|
| 329 | H | OH | OH | CN, CN |
| 330 | H | $O\overset{\overset{O}{\|}}{C}CH_3$ | $O\overset{\overset{O}{\|}}{C}CH_3$ | CN, CN |
| 331 | H | $CH_3$ | H | CN, $CO_2CH_3$ |
| 332 | H | $CH_3$ | $CH_3$ | CN, $CO_2CH_3$ |
| 333 | H | $O\overset{\overset{O}{\|}}{C}NHC_6H_5$ | H | CN, $CO_2CH_3$ |
| 334 | H | $O\overset{\overset{O}{\|}}{C}CH_2OCH_3$ | H | CN, 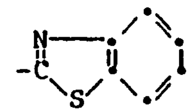 |
| 335 | H | $O\overset{\overset{O}{\|}}{C}CH_2OC_6H_5$ | H | CN, 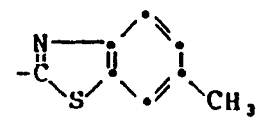 |
| 336 | H | $O\overset{\overset{O}{\|}}{C}CH_2C_6H_5$ | H | CN, 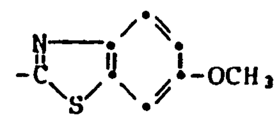 |
| 337 | H | $O\overset{\overset{O}{\|}}{C}NHC_2H_5$ | H | CN, 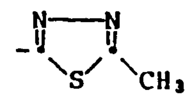 |
| 338 | H | 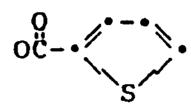 | H | CN, CN |
| 339 | H | 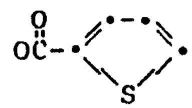 | H | CN, $CO_2CH_3$ |
| 340 | H | $O\overset{\overset{O}{\|}}{C}CH_3$ | H | CN, 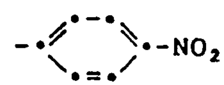 |
| 341 | H | $O\overset{\overset{O}{\|}}{C}CH_3$ | H | CN, 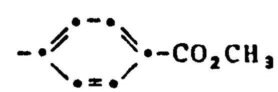 |

TABLE 8

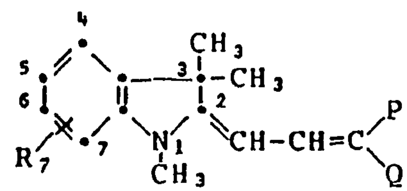

| Example No. | R7 | P, Q |
|---|---|---|
| 342 | H | CN, $CO_2CH_3$ |
| 343 | H | $CO_2CH_2CH_3$, $CO_2CH_2CH_3$ |
| 344 | H | $CO_2CH_3$, $SO_2CH_3$ |
| 345 | H | $CO_2CH_3$, $SO_2C_6H_5$ |
| 346 | H | CN, $CONHC_2H_4OH$ |
| 347 | H | CN, $CO_2CH_2CH_2OH$ |
| 348 | H | CN, $CO_2CH_2CH_2OC_2H_5$ |
| 349 | H | CN, $CO_2CH_2CH_2Cl$ |
| 350 | H | CN, $CO_2CH_2CH_2C_6H_5$ |
| 351 | H | CN, $CO_2CH_2CH_2OC_6H_5$ |
| 352 | H | CN, $CO_2C_6H_5$ |
| 353 | H | CN, $CO_2C_6H_{11}$ |
| 354 | H | CN, $CO_2CH_2C_6H_{11}$ |
| 355 | H | CN, $CO_2CH_2C_6H_5$ |
| 356 | 5-$CH_3$ | CN, $CO_2CH_2CH(CH_3)_2$ |

TABLE 8 (continued)

| Example No. | $R_7$ | P, Q |
|---|---|---|
| 357 | 5-$OCH_3$ | CN, $CO_2CH_2CH_2CN$ |
| 358 | 5-Cl | CN, $CO_2CH_2$- 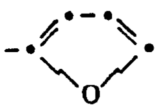 |
| 359 | 5-COOH | CN, CN |
| 360 | 5-COOH | CN, $CO_2CH_3$ |
| 361 | 5-COOH | CN, COOH |
| 362 | 5-COOH | CN, $CONHC_6H_5$ |
| 363 | 5-COOH | CN, $CONHC_2H_5$ |
| 364 | 5-COOH | CN, $SO_2CH_3$ |
| 365 | 5-COOH | CN, $SO_2C_6H_5$ |
| 366 | 5-$CO_2CH_3$ | CN, $COC(CH_3)_3$ |
| 367 | 5-$CO_2CH_3$ | 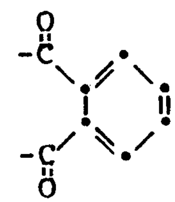 |
| 368 | 5-$CO_2CH_3$ | 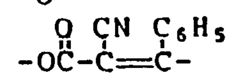 |
| 369 | 5-$CO_2CH_3$ | 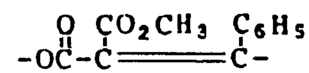 |
| 370 | 5-$CO_2CH_3$ | CN, 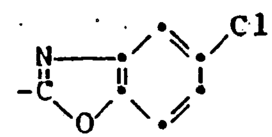 |
| 371 | 5-$CO_2CH_3$ | CN, 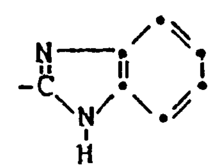 |

TABLE 8 (continued)

| Example No. | $R_7$ | P, Q |
|---|---|---|
| 372 | 5-$CO_2CH_3$ | CN, $SO_2$- 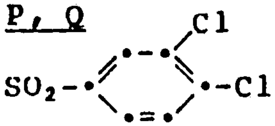 |
| 373 | 5-$CO_2CH_2CH_3$ | CN, $CO_2C_2H_5$ |
| 374 | 5-$CO_2CH_2C_6H_5$ | CN, $CO_2CH_3$ |
| 375 | 5-$CO_2CH_2CH_2OH$ | CN, $CO_2CH_3$ |
| 376 | 5-$CO_2CH_2CH_2C_6H_5$ | CN, CN |
| 377 | 5-$CO_2CH_2CH_2CN$ | CN, $CO_2CH_3$ |
| 378 | 5-$CO_2CH(CH_3)_2$ | CN, $CO_2CH_3$ |
| 379 | 5-COOH | CN, COOH |
| 380 | 5-COOH | 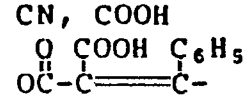 |
| 381 | 5-COOH | 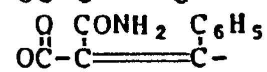 |
| 382 | 5-COOH | $CO_2CH_3$, 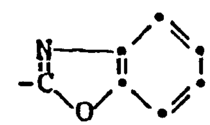 |
| 383 | 5-COOH | CN, 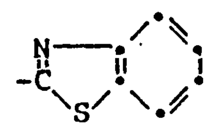 |
| 384 | 5-COOH | CN, CONH- 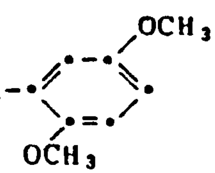 |

TABLE 9

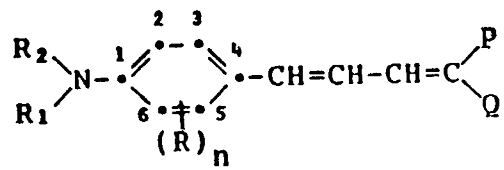

| Example No. | (R)n | R1 | R2 | P, Q |
|---|---|---|---|---|
| 385 | H | $CH_3$ | $CH_3$ | CN, $CO_2C_2H_5$ |
| 386 | H | $CH_3$ | $CH_3$ | CN, $CO_2C_2H_4OH$ |
| 387 | 3-$CH_3$ | $CH_3$ | $CH_3$ | CN, $CO_2CH_3$ |
| 388 | 3-$OC_2H_5$ | $CH_3$ | $CH_3$ | CN, $CO_2CH_3$ |
| 389 | 3-Cl | $CH_3$ | $CH_3$ | CN, $CO_2CH_2CH_2CN$ |
| 390 | 2-$OCH_3$, 5-$CH_3$ | $C_2H_5$ | $C_2H_5$ | CN, $CO_2CH_2CH_2OCH_3$ |
| 391 | 2,5-di-$OCH_3$ | $C_2H_5$ | $C_2H_5$ | CN, $CO_2C_6H_5$ |
| 392 | 2,5-di-$CH_3$ | $C_2H_5$ | $C_2H_5$ | CN, $CO_2CH_2CH_2C_6H_5$ |
| 393 | 3-$CH_3$ | $C_4H_9$-n | $C_4H_9$-n | CN, $CO_2CH_2CH_2OC_6H_5$ |
| 394 | 3-$C_2H_5$ | $CH_2CH(CH_3)_2$ | $C_2H_5$ | CN, $CONHC_2H_4OH$ |
| 395 | H | $C_6H_5$ | $C_2H_5$ | CN, $CO_2CH_3$ |
| 396 | 3-$OCH_3$ | $C_6H_5$ | $C_2H_5$ | CN, $CO_2CH_3$ |
| 397 | H | $C_6H_5$ | $C_6H_5$ | CN, $CO_2CH_3$ |
| 398 | H | $C_6H_5$ | $C_6H_5$ | $CO_2C_2H_5$, $CO_2C_2H_5$ |
| 399 | H | $C_6H_{11}$ | $C_6H_5$ | CN, $CO_2CH_3$ |

TABLE 9 (continued)

| Example No. | (R)n | R1 | R2 | P, Q |
|---|---|---|---|---|
| 400 | H | 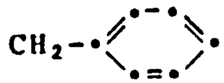 | 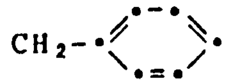 | CN, $CO_2CH_3$ |
| 401 | H | 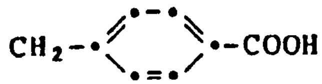 | 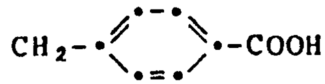 | CN, CN |
| 402 | H | 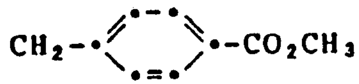 | 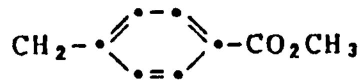 | CN, CN |
| 403 | H | 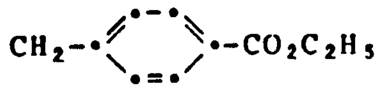 | 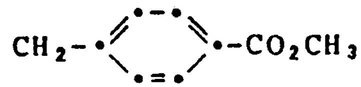 | CN, $SO_2CH_3$ |
| 404 | H | 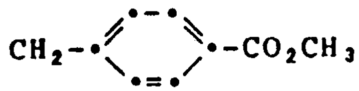 | $C_2H_5$ | CN, 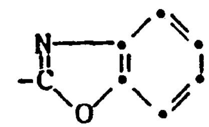 |
| 405 | 3-$CH_3$ | 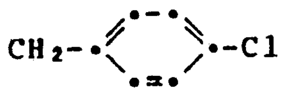 | 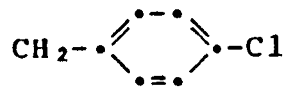 | 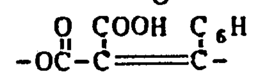 |
| 406 | 3-$CH_3$ | $CH_2CH_2Cl$ | $CH_2CH_2Cl$ | CN, $CO_2CH_3$ |
| 407 | 3-$CH_3$ | $CH_2CH_2OC_2H_4OC_2H_5$ | $C_2H_5$ | CN, $CO_2CH_3$ |
| 408 | 3-$CH_3$ | $CH_2CH_2OH$ | $CH_2CH_2OH$ | CN, CN |
| 409 | 3-$CH_3$ | $CH_2CH_2OH$ | $CH_2CH_2OH$ | 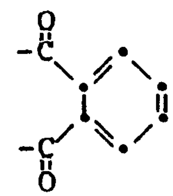 |
| 410 | 3-$CH_3$ | $CH_2CH_2O\overset{O}{\overset{\|}{C}}CH_3$ | $CH_2CH_2O\overset{O}{\overset{\|}{C}}CH_3$ | 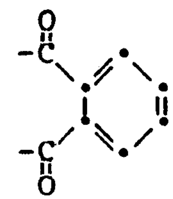 |

TABLE 9 (continued)

| Example No. | (R)n | R1 | R2 | P, Q |
|---|---|---|---|---|
| 411 | $3-CH_3$ | $CH_2CH_2O\overset{O}{\overset{\parallel}{C}}CH_3$ | $CH_2CH_2O\overset{O}{\overset{\parallel}{C}}CH_3$ | CN, 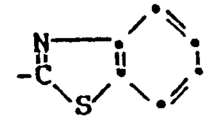 |
| 412 | $3-CH_3$ | $CH_2CH(OH)CH_2OH$ | $C_2H_5$ | CN, CN |
| 413 | $3-CH_3$ | $CH_2CH(OH)CH_2OH$ | $C_2H_5$ | CN, $SO_2CH_3$ |
| 414 | $3-CH_3$ | $CH_2CH(O\overset{O}{\overset{\parallel}{C}}CH_3)CH_2O\overset{O}{\overset{\parallel}{C}}CH_3$ | $C_2H_5$ | CN, $SO_2C_6H_5$ |
| 415 | $3-CH_3$ | $CH_2CH(OH)CH_3$ | $C_2H_5$ | CN, $SO_2C_6H_{11}$ |
| 416 | $3-CH_3$ | $CH_2CH_2O\overset{O}{\overset{\parallel}{C}}OC_2H_5$ | $CH_2CH_2O\overset{O}{\overset{\parallel}{C}}OC_2H_5$ | CN, CN |
| 417 | $3-CH_3$ | $CH_2CH_2O\overset{O}{\overset{\parallel}{C}}C_6H_5$ | $CH_2CH_2O\overset{O}{\overset{\parallel}{C}}OC_2H_5$ | CN, CN |
| 418 | $3-CH_3$ | $CH_2CH_2O\overset{O}{\overset{\parallel}{C}}NH-$ 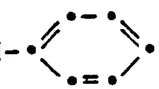 | $CH_2CH_2O\overset{O}{\overset{\parallel}{C}}NH-$ 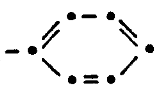 | CN, $CO_2CH_3$ |
| 419 | $3-CH_3$ | $CH_2CH_2O\overset{O}{\overset{\parallel}{C}}C_2H_5$ | $CH_2CH_2O\overset{O}{\overset{\parallel}{C}}C_2H_5$ | CN, $SO_2-$ 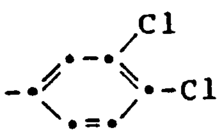 |
| 420 | $3-CH_3$ | $CH_2CH_2O\overset{O}{\overset{\parallel}{C}}C_2H_5$ | $CH_2CH_2O\overset{O}{\overset{\parallel}{C}}C_2H_5$ | CN, 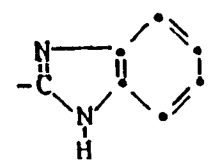 |
| 421 | $3-CH_3$ | $CH_2CH_2O\overset{O}{\overset{\parallel}{C}}-$ 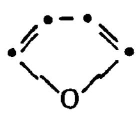 | $CH_2CH_2O\overset{O}{\overset{\parallel}{C}}-$ 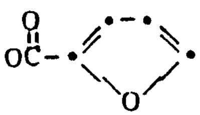 | CN, $CO_2CH_3$ |
| 422 | $3-CH_3$ | $CH_2CH_2O\overset{O}{\overset{\parallel}{C}}CH_2-$ 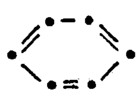 | $CH_2CH_2O\overset{O}{\overset{\parallel}{C}}CH_2-$ 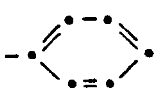 | CN, CN |
| 423 | $3-CH_3$ | $CH_3$ | $CH_3$ | CN, $CO_2CH_2-$ 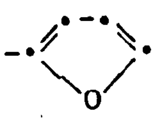 |

TABLE 10

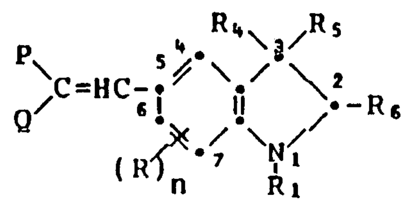

| Example No. | (R)n | R1 | R4, R5 | R6 | P, Q |
|---|---|---|---|---|---|
| 424 | H | $CH_3$ | $CH_3$, $CH_3$ | $CH_3$ | CN, $CO_2CH_3$ |
| 425 | H | $CH_3$ | $CH_3$, $CH_3$ | $CH_3$ | CN, $CO_2C_2H_4OH$ |
| 426 | H | $CH_3$ | $CH_3$, $CH_3$ | $CH_3$ | CN, $CONHC_2H_4OH$ |
| 427 | H | $CH_3$ | $CH_3$, $CH_3$ | $CH_3$ | CN, $CO_2CH_2CH_2CN$ |
| 428 | H | $CH_3$ | $CH_3$, $CH_3$ | $CH_3$ | CN, $CO_2CH_2CH_2OC_2H_5$ |
| 429 | H | $CH_3$ | $CH_3$, $CH_3$ | $CH_3$ | CN, $CO_2CH_2CH_2C_6H_5$ |
| 430 | H | $CH_3$ | $CH_3$, $CH_3$ | $CH_3$ | CN, $CO_2CH_2C_6H_5$ |
| 431 | H | $CH_3$ | $CH_3$, $CH_3$ | $CH_3$ | CN, $CO_2CH_2CH_2OC_6H_5$ |
| 432 | H | $CH_2CH_2Cl$ | $CH_3$, $CH_3$ | $CH_3$ | CN, $CO_2C_2H_5$ |
| 433 | H | $CH_2CH_2OH$ | $CH_3$, $CH_3$ | $CH_3$ | $CO_2C_2H_5$, $CO_2C_2H_5$ |
| 434 | H | $CH_2CH_2OC_6H_5$ | $CH_3$, $CH_3$ | $CH_3$ | $CO_2CH_3$, $SO_2CH_3$ |
| 435 | H | $CH_2CH_2OC(=O)CH_3$ | $CH_3$, $CH_3$ | $CH_3$ | CN, CN |
| 436 | H | $CH_2CH_2OC(=O)CH_3$ | $CH_3$, $CH_3$ | $CH_3$ | CN, $SO_2C_6H_5$ |

TABLE 10 (continued)

| Example No. | (R)n | $R_1$ | $R_4$, $R_5$ | $R_6$ | P, Q |
|---|---|---|---|---|---|
| 437 | H | $CH_2CH_2O\overset{O}{\overset{\|}{C}}CH_3$ | $CH_3$, $CH_3$ | $CH_3$ | CN, $SO_2CH_3$ |
| 438 | H | $CH_2CH_2O\overset{O}{\overset{\|}{C}}CH_3$ | $CH_3$, $CH_3$ | $CH_3$ | CN, $CONHC_6H_5$ |
| 439 | H | $CH_2CH_2O\overset{O}{\overset{\|}{C}}CH_3$ | $CH_3$, H | $CH_3$ | CN, $CONHC_2H_4OH$ |
| 440 | H | $CH_2CH_2O\overset{O}{\overset{\|}{C}}CH_3$ | H, H | H | CN, $SO_2CH_3$ |
| 441 | H | $CH_2CH_2O\overset{O}{\overset{\|}{C}}CH_3$ | H, H | $C_6H_5$ | CN, $CO_2CH_3$ |
| 442 | 6-Cl | $CH_2CH_2O\overset{O}{\overset{\|}{C}}CH_3$ | H, H | $CH_3$ | 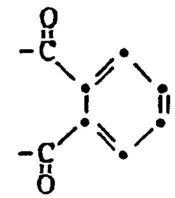 |
| 443 | 7-$CH_3$ | $CH_2CH_2O\overset{O}{\overset{\|}{C}}CH_3$ | H, H | $CH_3$ | CN, CONH- 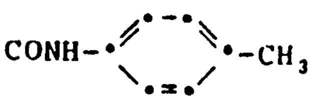 |
| 444 | H | $CH_2CH_2O\overset{O}{\overset{\|}{C}}OC_2H_5$ | H, H | $CH_3$ | 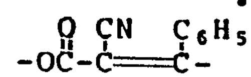 |
| 445 | H | $CH_2CH_2O\overset{O}{\overset{\|}{C}}C_6H_5$ | H, H | $CH_3$ | 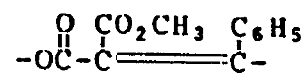 |
| 446 | H | $CH_2CH_2O\overset{O}{\overset{\|}{C}}NH$- 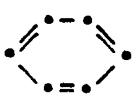 | H, H | $CH_3$ | 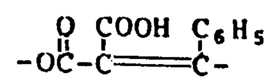 |
| 447 | H | $CH_2CH_2CH_2NH\overset{O}{\overset{\|}{C}}$- 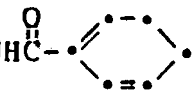 | H, H | $CH_3$ | CN, $CO_2CH_3$ |
| 448 | H | $CH_2CH_2Cl$ | H, H | $CH_3$ | CN, $CO_2CH_3$ |

TABLE 10 (continued)

| Example No. | (R)n | $R_1$ | $R_4$, $R_5$ | $R_6$ | P, Q |
|---|---|---|---|---|---|
| 449 | H | 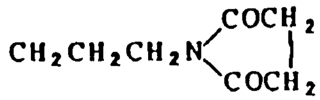 | H, H | $CH_3$ | CN, $CO_2C_6H_5$ |
| 450 | H | 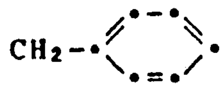 | H, H | $CH_3$ | CN, $CO_2CH_2C_6H_5$ |
| 451 | H | 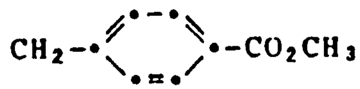 | H, H | $CH_3$ | CN, CN |
| 452 | H | 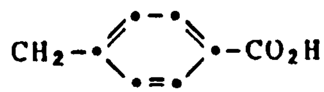 | H, H | $CH_3$ | CN, CN |
| 453 | H | 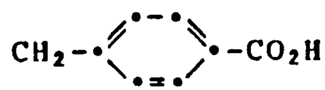 | H, H | $CH_3$ | CN, 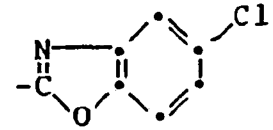 |
| 454 | H | 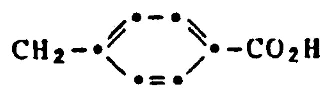 | H, H | $CH_3$ | CN, 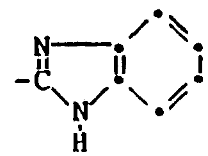 |
| 455 | H | 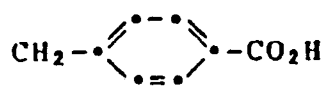 | H, H | $CH_3$ | CN, 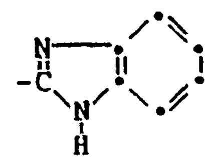 |
| 456 | H | 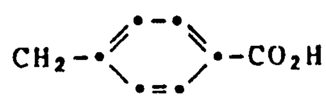 | H, H | $CH_3$ | CN, 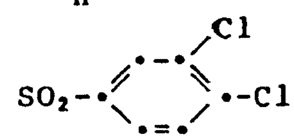 |
| 457 | H | $C_6H_5$ | H, H | $CH_3$ | CN, $CO_2CH_3$ |
| 458 | H | $CH_2CH_2O\overset{O}{\overset{\|}{C}}CH_2C_6H_5$ | H, H | $CH_3$ | CN, $CO_2CH_2CH_2CN$ |

TABLE 10 (continued)

| Example No. | (R)n | R1 | R4, R5 | R6 | P, Q |
|---|---|---|---|---|---|
| 459 | H | $CH_2CH_2O\overset{O}{\overset{\|}{C}}CH_3$ | H, H | $CH_3$ | CN, $COC(CH_3)_2$ |
| 460 | H | $CH_2CH_2O\overset{O}{\overset{\|}{C}}CH_3$ | H, H | $CH_3$ | CN, $COC_6H_5$ |
| 461 | H | $CH_2CH_2O\overset{O}{\overset{\|}{C}}CH_3$ | H, H | $CH_3$ | CN, COOH |
| 462 | H | $CH_2CH_2O\overset{O}{\overset{\|}{C}}CH_3$ | H, H | $CH_3$ | CN, $C_6H_5$ |
| 463 | H | $CH_2CH_2O\overset{O}{\overset{\|}{C}}CH_3$ | H, H | $CH_3$ | CN, 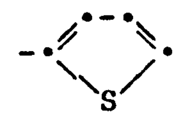 |
| 464 | H | $CH_2CH_2O\overset{O}{\overset{\|}{C}}CH_3$ | H, H | $CH_3$ | CN, 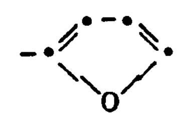 |

TABLE 11

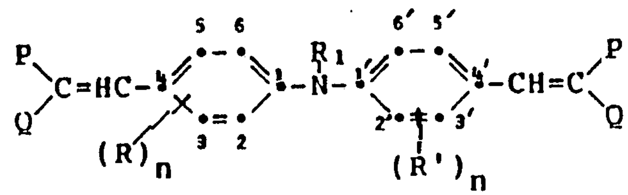

| Example No. | (R)n | (R')n | $R_1$ | P, Q |
|---|---|---|---|---|
| 465 | H | H | $CH_2CH_2OH$ | CN, CN |
| 466 | H | H | $CH_2CH_2OH$ | CN, $CONH_2$ |
| 467 | H | H | $CH_2CH_2OH$ | CN, $CO_2CH_3$ |
| 468 | H | H | $CH_2CH_2OH$ | CN, $CONHC_6H_5$ |
| 469 | H | H | $CH_2CH_2OH$ | 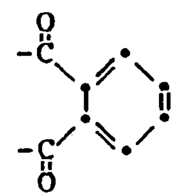 |
| 470 | H | H | $CH_2CH_2O\overset{O}{\overset{\|}{C}}CH_3$ | CN, CN |
| 471 | H | H | $CH_2CH_2O\overset{O}{\overset{\|}{C}}CH_3$ | CN, $CO_2CH_3$ |
| 472 | H | H | $CH_2CH_2O\overset{O}{\overset{\|}{C}}CH_3$ | CN, $SO_2CH_3$ |
| 473 | H | H | $CH_2CH_2O\overset{O}{\overset{\|}{C}}CH_3$ | CN, $SO_2C_6H_5$ |

TABLE 11 (continued)

| Example No. | (R)n | (R')n | R1 | P, Q |
|---|---|---|---|---|
| 474 | H | H | $CH_2CH_2OC(O)CH_3$ | CN, 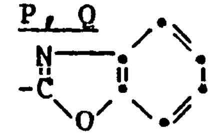 |
| 475 | H | H | $CH_2CH_2OC(O)CH_3$ | 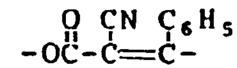 |
| 476 | H | H | $CH_2CH_2OC(O)CH_3$ | 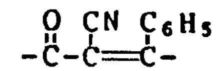 |
| 477 | H | H | $CH_2CH_2OC(O)CH_3$ | CN, $COC_6H_5$ |
| 478 | H | H | $CH_2CH_2OC(O)CH_3$ | CN, $COC(CH_3)_3$ |
| 479 | H | H | $CH_2CH_2OC(O)OC_2H_5$ | CN, $C_6H_5$ |
| 480 | H | H | $CH_2CH_2Cl$ | CN, $CO_2CH_3$ |
| 481 | 3-$CH_3$ | H | $CH_2CH_2OH$ | CN, $CO_2CH_3$ |
| 482 | 3-$CH_3$ | H | $C_2H_5$ | CN, $CO_2CH_3$ |
| 483 | 3-$CH_3$ | H | $CH_2$- 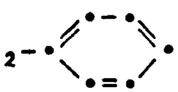 | CN, $CO_2CH_3$ |
| 484 | H | H | $CH_3$ | $CO_2C_2H_5$, $CO_2C_2H_5$ |
| 485 | H | H | $CH_2CH(OH)CH_3$ | 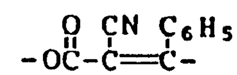 |
| 486 | H | H | $CH_2$- 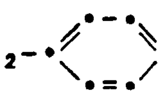 -COOH | CN, CN |
| 487 | H | H | $CH_2$- 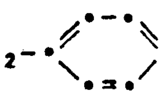 -$CO_2CH_3$ | CN, CN |

TABLE 11 (continued)

| Example No. | (R)n | (R')n | $R_1$ | P, Q |
|---|---|---|---|---|
| 488 | H | H | $CH_2$- 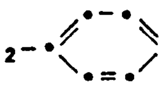 -$CO_2CH_3$ | CN, $CONH_2$ |
| 489 | 3-$CH_3$ | 3'-$CH_3$ | $CH_2$- 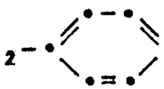 -$CO_2CH_3$ | CN, $CO_2CH_2CH_2CN$ |
| 490 | 3-$Cl_3$ | 2'-$CH_3$ | $CH_2$- 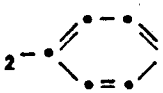 -$CO_2CH_3$ | CN, $CO_2CH_2CH_2OC_2H_5$ |
| 491 | 3-$CH_3$ | 3'-$OCH_3$ | $CH_2$- 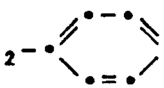 -$CO_2CH_3$ | CN, CN |
| 492 | 2,5-di-$OCH_3$ | H | $CH_2$- 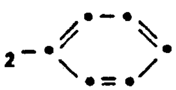 -$CO_2CH_3$ | CN, $SO_2$- 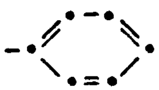 -$CH_3$ |
| 493 | 2,5-di-$CH_3$ | H | $CH_3$ | CN, $CO_2CH_3$ |
| 494 | 3-Br | 3'-$CH_3$ | $CH_3$ | CN, $CO_2CH_3$ |
| 495 | H | H | $C_2H_5$ | CN, $CO_2CH_3$ |
| 496 | H | H | $CH_2CH(CH_3)_2$ | CN, $CO_2CH_3$ |
| 497 | H | H | $CH_2C_6H_5$ | CN, $CO_2CH_3$ |
| 498 | H | H | $CH_2CH_2SO_2C_6H_5$ | CN, $CO_2CH_3$ |
| 499 | H | H | $CH_2CH_2CN$ | CN, $CO_2CH_3$ |
| 500 | H | H | $CH_2CH_2OC_6H_5$ | CN, $CO_2CH_3$ |
| 501 | H | H | $CH_2CH_2S$- 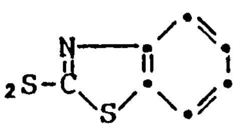 | CN, $CO_2CH_3$ |

TABLE 11 (continued)

| Example No. | (R)n | (R')n | R1 | P, Q |
|---|---|---|---|---|
| 502 | H | H | 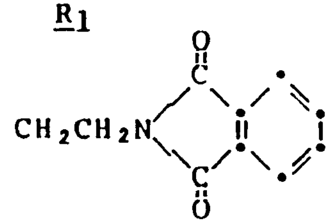 | CN, $CO_2CH_3$ |
| 503 | H | H | 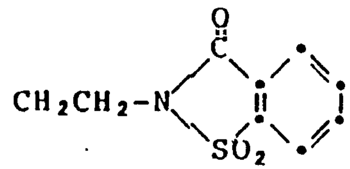 | CN, $CO_2CH_3$ |
| 504 | H | H | 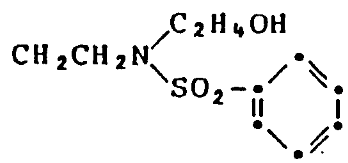 | CN, $CO_2CH_3$ |
| 505 | H | H | 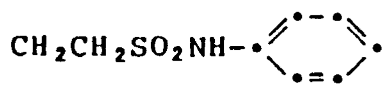 | CN, $CO_2CH_3$ |
| 506 | H | H | 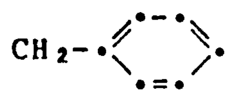 | 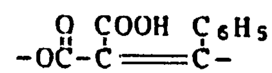 |
| 507 | H | H | 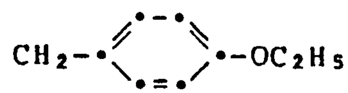 | CN, $CO_2CH_2CH_2OH$ |
| 508 | H | H | 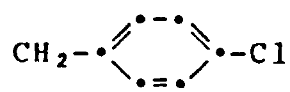 | CN, $CONHC_2H_4OH$ |

TABLE 12

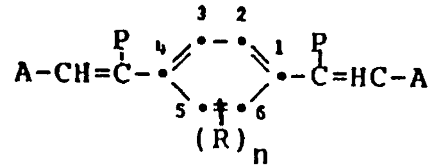

| Example No. | A | $(R)_n$ | P |
|---|---|---|---|
| 509 | 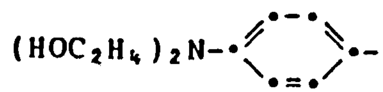 | H | CN |
| 510 | 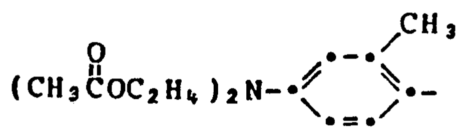 | H | CN |
| 511 | 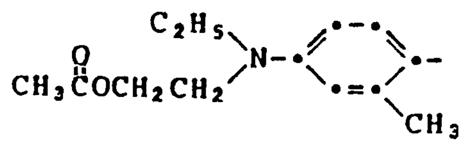 | H | CN |
| 512 | 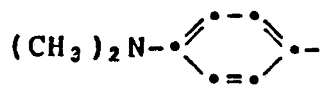 | H | $CO_2CH_3$ |
| 513 | 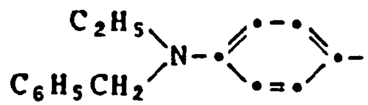 | H | $CO_2CH_3$ |
| 514 | 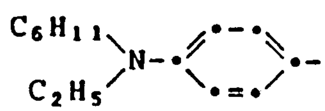 | 2-$CH_3$ | $CO_2CH_3$ |
| 515 | 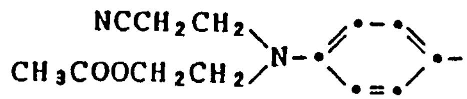 | 2-Cl | $CONH_2$ |

TABLE 12 (continued)

| Example No. | A | (R)n | P |
|---|---|---|---|
| 516 | 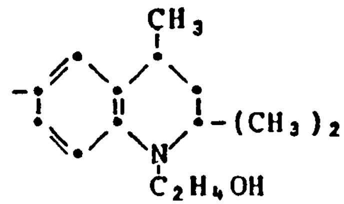 | H | CN |
| 517 | 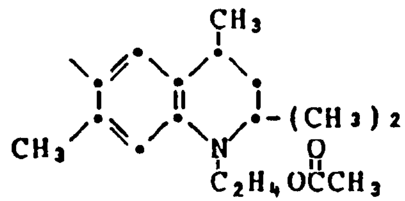 | 2,5-di-$CH_3$ | CN |
| 518 | 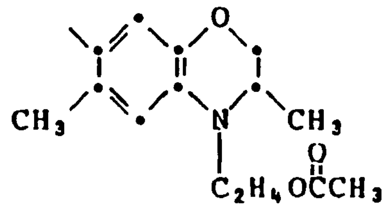 | H | CN |
| 519 | 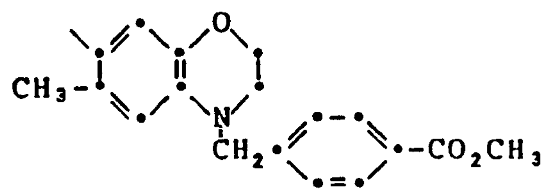 | H | CN |
| 520 | 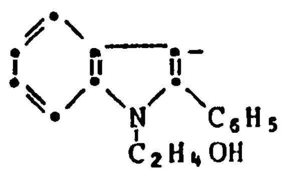 | H | CN |
| 521 | 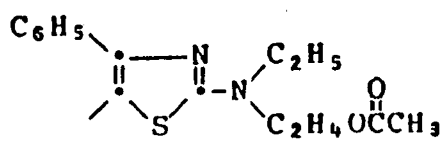 | 2-$OCH_3$ | CN |

TABLE 12 (continued)

| Example No. | A | (R)n | P |
|---|---|---|---|
| 522 | 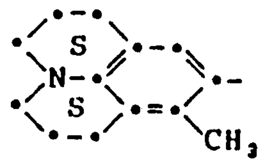 | H | $CO_2CH_3$ |
| 523 | 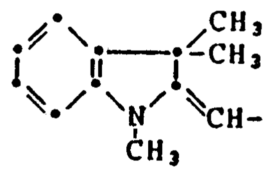 | H | $CO_2CH_3$ |
| 524 | 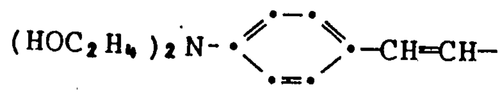 | H | CN |
| 525 | 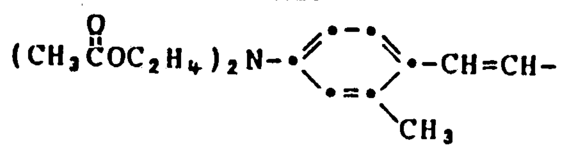 | H | CN |
| 526 | 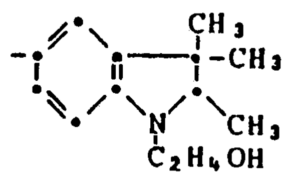 | H | CN |
| 527 | 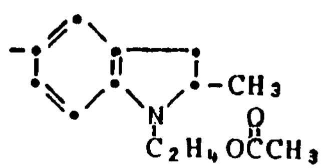 | H | CN |

TABLE 12 (continued)

| Example No. | A | (R)n | P |
|---|---|---|---|
| 528 | 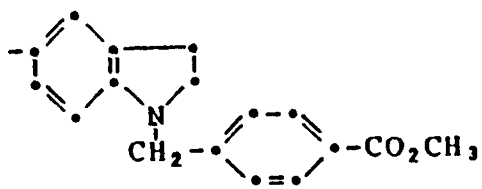 | H | CN |
| 529 | 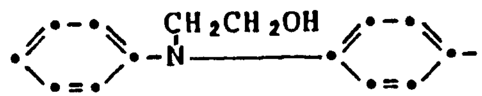 | H | CN |
| 530 | 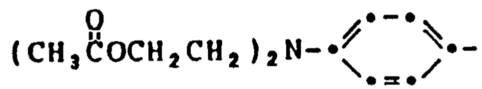 | H | $SO_2CH_3$ |
| 531 | 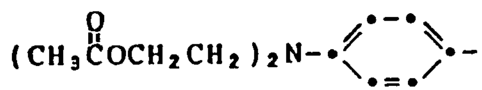 | H | 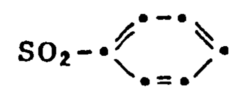 |

TABLE 13

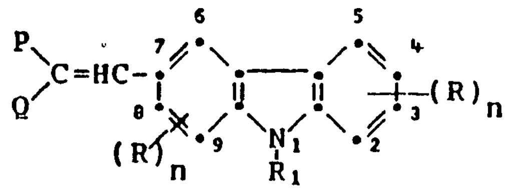

| Example No. | (R)n | R1 | P, Q |
|---|---|---|---|
| 532 | H | $C_2H_4OH$ | CN, CN |
| 533 | H | $C_2H_4OH$ | CN, $SO_2CH_3$ |
| 534 | H | $C_2H_4OH$ | CN, $SO_2C_6H_5$ |
| 535 | H | $C_2H_4OH$ | CN, $CONHC_6H_5$ |
| 536 | H | $C_2H_4OH$ | CN, $CONHC_2H_4OH$ |
| 537 | H | $CH_2CH_2O\overset{O}{\overset{\|}{C}}CH_3$ | CN, CN |
| 538 | H | $CH_2CH_2O\overset{O}{\overset{\|}{C}}CH_3$ | CN, $SO_2CH_3$ |
| 539 | 4,8-di-$CH_3$ | $CH_2CH_2O\overset{O}{\overset{\|}{C}}CH_3$ | CN, $CO_2CH_3$ |
| 540 | 8-$CH_3$ | $CH_2CH_2O\overset{O}{\overset{\|}{C}}-C_6H_5$ | CN, $CO_2CH_3$ |
| 541 | 8-$CH_3$ | $CH_2CH_2O\overset{O}{\overset{\|}{C}}CH_3$ | 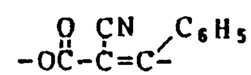 |
| 542 | 8-$CH_3$ | $CH_2CH(OH)CH_3$ | 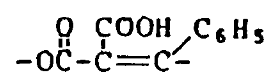 |

TABLE 13 (continued)

| Example No. | (R)n | $R_1$ | P, Q |
|---|---|---|---|
| 543 | 2-Cl-8-$CH_3$ | $CH_2CH_2OCH_2CH_2OCCH_3$ (with =O on C) | 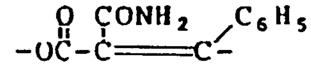 |
| 544 | 4-Cl-8-$CH_3$ | 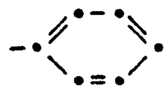-COOH | CN, $CO_2CH_3$ |
| 545 | 8-$CH_3$ | $CH_2$-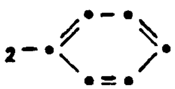-$CO_2CH_3$ | CN, CN |
| 546 | 8-$CH_3$ | $C_6H_5$ | CN, $CO_2CH_3$ |
| 547 | H | $CH_2$-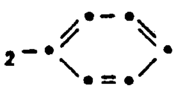-COOH | CN, $CO_2CH_3$ |
| 548 | 4-$OCH_3$-8-$CH_3$ | $CH_2$-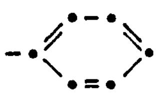-COCl | CN, CN |
| 549 | 3,4-di-Cl-8-$CH_3$ | $CH_2CH_2O\overset{O}{\overset{\|\|}{C}}CH_3$ | 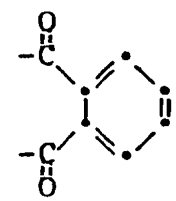 |
| 550 | H | 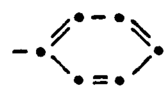-$CO_2CH_3$ | CN, $CO_2CH_3$ |
| 551 | H | $CH_2CH_2N(CH_3)SO_2CH_3$ | CN, $CO_2CH_3$ |
| 552 | 6,8-di-$CH_3$ | $CH_2CH_2O\overset{O}{\overset{\|\|}{C}}NHC_6H_5$ | CN, $CO_2CH_3$ |
| 553 | 6,9-di-$CH_3$ | $CH_2CH_2Cl$ | CN, $CO_2CH_3$ |
| 554 | 8-$CH_3$ | $CH_2CH_2OC_6H_5$ | CN, $CO_2CH_3$ |
| 555 | 8-$CH_3$ | $CH_2CH_2SC_6H_5$ | CN, $CO_2CH_3$ |

TABLE 13 (continued)

| Example No. | (R)n | $R_1$ | P, Q |
|---|---|---|---|
| 556 | $4-OCH_3-8-CH_3$ | $CH_2CH_2S-$ 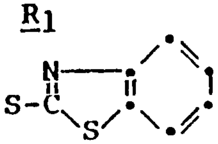 | CN, COOH |
| 557 | $8-CH_3$ | $CH_2CH_2SO_2-$ 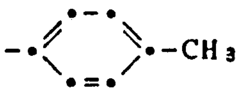 | CN, $CO_2CH_3$ |
| 558 | $8-CH_3$ | $CH_2CH_2$ 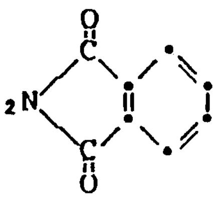 | CN, $CO_2CH_3$ |
| 559 | $8-CH_3$ | $-CH_2CH_2$ 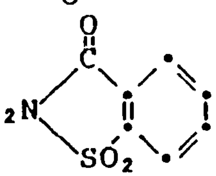 | CN, $CO_2CH_3$ |
| 560 | $8-CH_3$ | $-CH_2CH=CH_2$ | CN, $CO_2CH_3$ |
| 561 | $8-CH_3$ | $-CH_2CH_2OC_2H_4OH$ | CN, CN |
| 562 | $8-CH_3$ | $-CH_2CH_2SO_2CH=CH_2$ | CN, $CO_2CH_3$ |
| 563 | $8-CH_3$ | $-CH_2CH(OH)CH_2OH$ | CN, $COC(CH_3)_2$ |
| 564 | $9-CH_3$ | $-CH_2C_6H_{11}$ | CN, $CO_2CH_3$ |
| 565 | $9-CH_3$ | $-CH_2CH_2SO_2N(CH_3)_2$ | CN, $CO_2CH_3$ |
| 566 | $9-CH_3$ | $-CH_2CH_2CON(CH_3)C_6H_5$ | CN, $CO_2CH_3$ |
| 567 | $4-CH_3$ | $-CH_2CH_2OC(=O)CH_2Cl$ | CN, $CO_2CH_3$ |
| 568 | $4-CH_3$ | $-CH_2CH_2OC(=O)CH_3$ | CN, 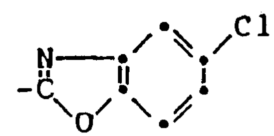 |

TABLE 13 (continued)

| Example No. | (R)n | $R_1$ | P, Q |
|---|---|---|---|
| 569 | $4-CH_3$ | $-CH_2CH_2O\overset{O}{\overset{\|}{C}}CH_3$ | CN, 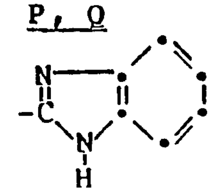 |
| 570 | $4-CH_3$ | $-CH_2CH_2O\overset{O}{\overset{\|}{C}}CH_3$ | CN, 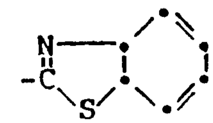 |
| 571 | $2-CH_3$, $8-OCH_3$ | 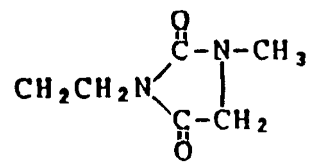 | CN, $CO_2CH_3$ |
| 572 | 4,8-di-$OCH_3$ | 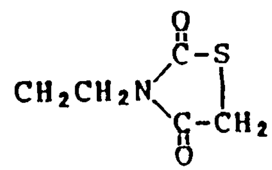 | CN, $CO_2CH_3$ |
| 573 | 6,9-di-$CH_3$ | 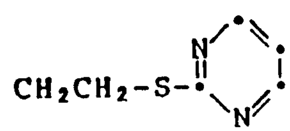 | CN, $CO_2CH_3$ |

TABLE 14

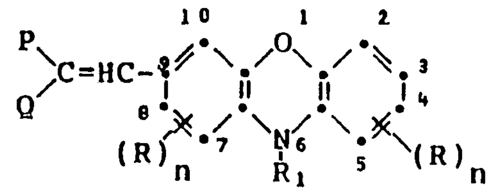

| Example No. | (R)n | $R_1$ | P, Q |
|---|---|---|---|
| 574 | H | $C_2H_4OH$ | CN, CN |
| 575 | H | $C_2H_4OH$ | CN, $SO_2CH_3$ |
| 576 | H | 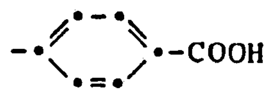 | CN, $SO_2C_6H_5$ |
| 577 | H | 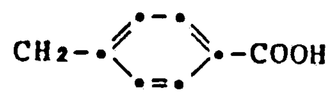 | CN, $CONHC_6H_5$ |
| 578 | H | $C_2H_4OH$ | CN, $CONHC_2H_4OH$ |
| 579 | H | $CH_2CH_2O\overset{O}{\overset{\|}{C}}CH_3$ | CN, CN |
| 580 | H | $CH_2CH_2O\overset{O}{\overset{\|}{C}}CH_3$ | CN, $SO_2CH_3$ |
| 581 | 8-$CH_3$ | $CH_2CH_2O\overset{O}{\overset{\|}{C}}CH_3$ | CN, $CO_2CH_3$ |
| 582 | 8-$CH_3$ | $CH_2CH_2O\overset{O}{\overset{\|}{C}}-C_6H_5$ | CN, $CO_2CH_3$ |

TABLE 14 (continued)

| Example No. | (R)n | $R_1$ | P, Q |
|---|---|---|---|
| 583 | 8-$CH_3$ | $CH_2CH_2O\overset{O}{\overset{\|}{C}}CH_3$ | 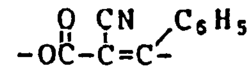 |
| 584 | 8-$CH_3$ | $CH_2CH(OH)CH_3$ | 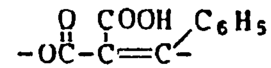 |
| 585 | 2-Cl-8-$CH_3$ | $CH_2CH_2OCH_2CH_2O\overset{O}{\overset{\|}{C}}CH_3$ | 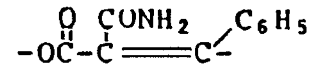 |
| 586 | 4-Cl-8-$CH_3$ | 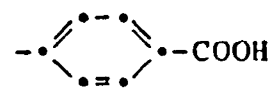 | CN, $CO_2CH_3$ |
| 587 | 5,8-di-$CH_3$ | 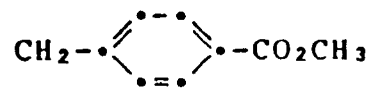 | CN, CN |
| 588 | H | $C_6H_5$ | CN, $CO_2CH_3$ |
| 589 | H | 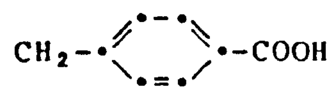 | CN, $CO_2CH_3$ |
| 590 | H | 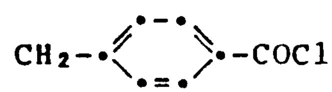 | CN, CN |
| 591 | 3-$CH_3$ | $CH_2CH_2O\overset{O}{\overset{\|}{C}}CH_3$ | 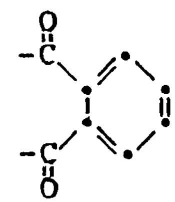 |
| 592 | 3-$CH_3$ | 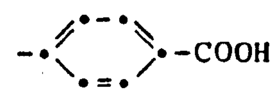 | CN, $CO_2CH_3$ |
| 593 | 3-$CH_3$ | $CH_2CH_2N(CH_3)SO_2CH_3$ | CN, $CO_2CH_3$ |
| 594 | 3-$OCH_3$-8-$CH_3$ | $CH_2CH_2O\overset{O}{\overset{\|}{C}}NHC_6H_5$ | CN, $CO_2CH_3$ |

TABLE 14 (continued)

| Example No. | (R)n | R1 | P, Q |
|---|---|---|---|
| 595 | 3-$OCH_3$-7-$CH_3$ | $CH_2CH_2Cl$ | CN, $CO_2CH_3$ |
| 596 | 3-$OCH_3$-7-$CH_3$ | $CH_2CH_2OC_6H_5$ | CN, $CO_2CH_3$ |
| 597 | 3-$OCH_3$-7-$CH_3$ | $CH_2CH_2SC_6H_5$ | CN, $CO_2CH_3$ |
| 598 | 3,5-di-$CH_3$ | $CH_2CH_2S$- 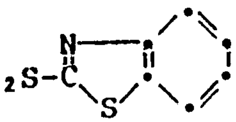 | CN, COOH |
| 599 | 3,5-di-$CH_3$ | $CH_2CH_2SO_2$- 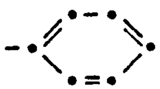 -$CH_3$ | CN, $CO_2CH_3$ |
| 600 | 3-$OCH_3$ | $CH_2CH_2$ 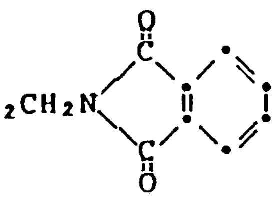 | CN, $CO_2CH_3$ |
| 601 | 7,10-di-$CH_3$ | -$CH_2CH_2$ 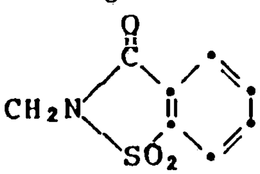 | CN, $CO_2CH_3$ |
| 602 | 3,8-di-$CH_3$ | -$CH_2CH{=}CH_2$ | CN, $CO_2CH_3$ |
| 603 | 3,8-di-$OCH_3$ | -$CH_2CH_2OC_2H_4OH$ | CN, CN |
| 604 | 8-$OCH_3$ | -$CH_2CH_2SO_2CH{=}CH_2$ | CN, $CO_2CH_3$ |
| 605 | 8-Cl | -$CH_2CH(OH)CH_2OH$ | CN, $COC(CH_3)_2$ |
| 606 | 8-$CH_3$ | -$CH_2C_6H_{11}$ | CN, $CO_2CH_3$ |
| 607 | 8-$CH_3$ | -$CH_2CH_2SO_2N(CH_3)_2$ | CN, $CO_2CH_3$ |
| 608 | 8-$CH_3$ | -$CH_2CH_2CON(CH_3)C_6H_5$ | CN, $CO_2CH_3$ |

TABLE 14 (continued)

| Example No. | (R)n | R1 | P, Q |
|---|---|---|---|
| 609 | H | $-CH_2CH_2O\overset{O}{\overset{\|}{C}}CH_2Cl$ | CN, $CO_2CH_3$ |
| 610 | H | $-CH_2CH_2O\overset{O}{\overset{\|}{C}}CH_3$ | CN, 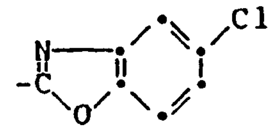 |
| 611 | H | $-CH_2CH_2O\overset{O}{\overset{\|}{C}}CH_3$ | CN, 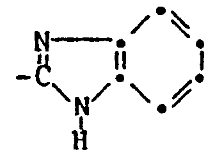 |
| 612 | 5,8-di-$CH_3$ | $-CH_2CH_2O\overset{O}{\overset{\|}{C}}CH_3$ | CN, 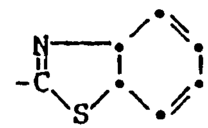 |
| 613 | 5,8-di-$CH_3$ | 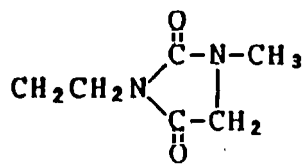 | CN, $CO_2CH_3$ |
| 614 | 8-$CH_3$ | 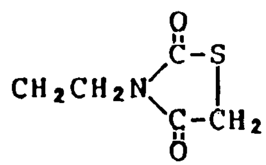 | CN, $CO_2CH_3$ |
| 615 | 7,9-di-$OCH_3$ | $CH_2CH_2-S-$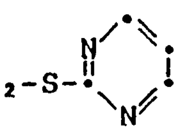 | CN, $CO_2CH_3$ |

TABLE 15

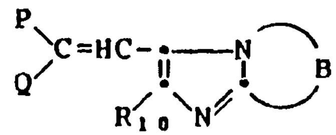

| Example No. | (R)10 | B | P, Q |
|---|---|---|---|
| 616 | $C_6H_5$ | 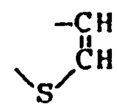 | CN, $CO_2CH_3$ |
| 617 | H | 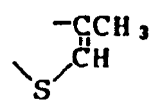 | 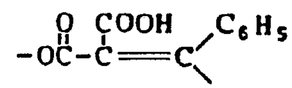 |
| 618 | $CH_3$ | 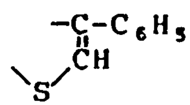 | $CO_2CH_3$, $CO_2CH_3$ |
| 619 | 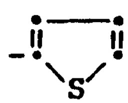 | 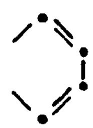 | CN, $CO_2CH_3$ |
| 620 | $C_6H_5$ | 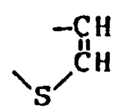 | CN, $CO_2CH_2CH_2OH$ |

TABLE 15 (continued)

| Example No. | $(R)_{10}$ | B | P, Q |
|---|---|---|---|
| 621 | $C_6H_4$-p-$CO_2CH_3$ | 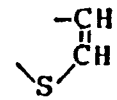 | 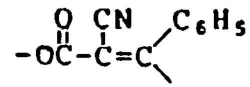 |
| 622 | $C_6H_4$-p-COOH | 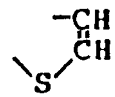 | 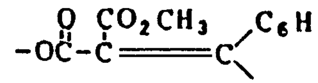 |
| 623 | $C_6H_4$-p-Br | 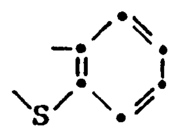 | CN, $CONHC_2H_4OH$ |
| 624 | $C_6H_4$-p-$OCH_3$ | 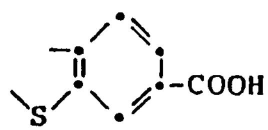 | 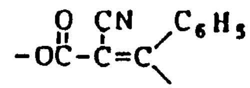 |
| 625 | $C_6H_4$-p-$CH_3$ | 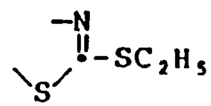 | CN, $CO_2CH_3$ |
| 626 | $C_6H_4$-o-Cl | 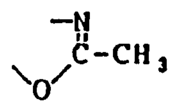 | CN, $SO_2$- 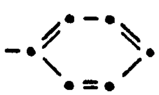 -$CO_2CH_3$ |
| 627 | $C_6H_4$-m-$CO_2CH_3$ | 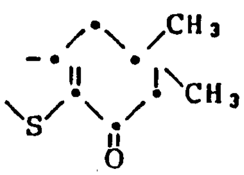 | 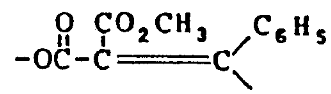 |
| 628 | $C_6H_4$-p-$CO_2CH_3$ | 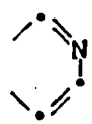 | 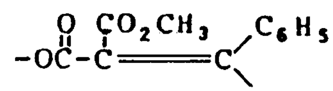 |

TABLE 15 (continued)

| Example No. | (R)10 | B | P, Q |
|---|---|---|---|
| 629 | $C_6H_5$ | 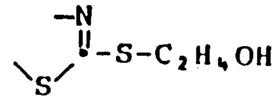 | CN, $CO_2CH_3$ |
| 630 | $C_6H_5$ | 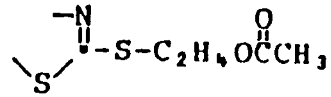 | CN, $CONHC_2H_4OH$ |
| 631 | $C_6H_5$ | 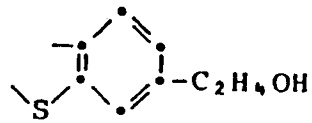 | 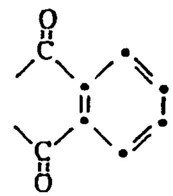 |
| 632 | $C_6H_5$ | 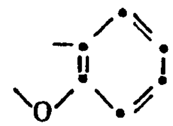 | CN, $CO_2CH_3$ |
| 633 | $C_6H_5$ | 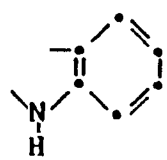 | CN, CONH- 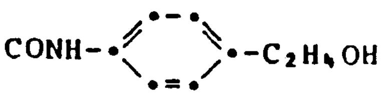 |
| 634 | $C_6H_5$ | 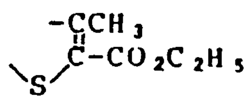 | 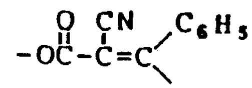 |
| 635 | $C_6H_5$ | 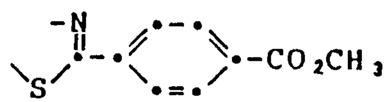 | 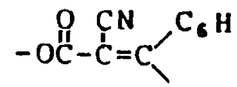 |
| 636 | $C_6H_5$ | 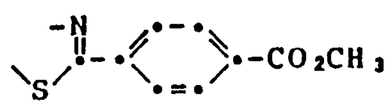 | CN, 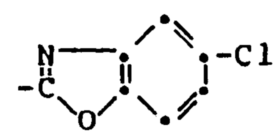 |

The preparation of the unsaturated polyesters of this invention is according to well known techniques in the art as described, for example, in U.S. Patents: 3,642,672; 3,549,586; 4,299,927; and 4,355,136. It is preferred that these polyesters have an acid number of from about 10 to about 28, an inherent viscosity of from about 0.05 to about 0.25, and a number average molecular weight of from about 1100 to about 3800. Typical colored polyester material prepared in accordance with this invention are as follows:

Example 637

Preparation of Unsaturated Polyester Material from Neopentyl Glycol, Isophthalic Acid, and Maleic Anhydride Copolymerized with Methine Colorant and Cured With Styrene

The reaction apparatus comprising a one-litre flask is fitted with a stirrer, thermometer, nitrogen inlet tube, and heated Vigreux column. The top of the Vigreux column is also fitted with a Dean-Stark trap and cold water condenser. The flask is charged with 251.6 g of neopentyl glycol (2.415 mol), 191.0 g of isophthalic acid (1.15 m), 0.0951 g (200 ppm) of the reactive methine compound

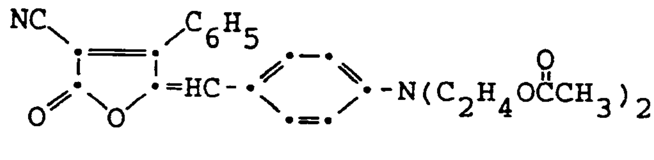

and 0.55 g of dibutyltin oxide. The flask is then heated to reflux and held at this temperature until the theoretical amount of distillate is collected from this esterification stage. The reaction system is cooled to 145°C and 112.8 g of maleic anhydride (1.15 m) and 0.055 g of toluhydroquinone are added. The esterification and polycondensation reactions are continued for one hour at 175°C, then for one hour at 185°C, and then at 195°C until an acid number of 18.1 for the unsaturated polyester is obtained. The inherent viscosity of this polyester is 0.133, the number average molecular weight is 2640 and the color is bright red-orange. Sufficient of this polyester material is blended or dissolved in styrene monomer to give 40 wt.% monomer and the system then blended with 1 wt% benzoyl peroxide. One-eighth inch thick sheet castings are prepared by decanting the blend between glass plates separated by $\frac{1}{8}$-inch thick gasket. The blend is cured by heating for two hours at 70°C, then for one hour at 100°C, then for two hours at 125°C, and then for one hour at 150°C. After cooling, the cured polyester sheet is removed and cut into bars. The flexural strength of the bars is $18.46 \times 10^3$ psi (127 N/mm$^2$).

Example 638

Preparation of Unsaturated Polyester Material from Propylene Glycol, Isophthalic Acid, and Maleic Anhydride Copolymerized With Methine Colorant and Cured With Styrene

The following components are charged into the equipment described in Example 801:

192.5 g propylene glycol (2.53 mol);
191.0 g isophthalic acid (1.15 mol);
0.50 g dibutyltin oxide;) and

0.0869 g. 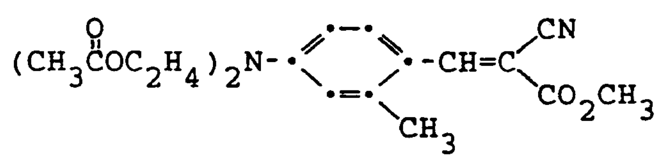

These components are reacted as described in Example 801 until the theoretical amount of distillate is collected. The reaction is cooled to 145°C and 112.8 g maleic anhydride (1.15 mol) and 0.05 g of toluhydroquinone are added. The reaction is continued for one hour at 175°C, then for one hour at 185°C, and then at 195°C until an acid number of 14.1 for the unsaturated polyester is obtained. The inherent viscosity of this polyester is 0.11, the number average molecular weight is 1912, and the color is yellow. A curable blend of this unsaturated polyester and sheet casting and bars thereof were prepared as in Example 801. The bars had a flexural strength of $18.60 \times 10^3$ psi (128 N/mm$^2$).

The above inherent viscosities, acid numbers, number average molecular weights, and flexural strengths were determined as follows:

Acid number by ASTM D—1639—70;

Number Average Moplecular Weight by ASTM D—08.03 (Gel Permeation Chromatography);

Flexural Strength by ASTM D—790—81;

Inherent Viscosity according to ASTM D2857—70 procedure in a Wagner Viscometer of Lab Glass Inc. of Vineland, N.J. having a 1/2 ml capilary bulb, using a polymer concentration of 0.5% by weight in 60/40 by weight, phenol/tetrachloroethane solvent. The procedure comprises heating the polymer/solvent system at

120°C for 15 minutes to enhance dissolution of the polymer, cooling the solution to 25°C and measuring the time of flow at 25°C. The I.V. is calculated from the equation

$$(\eta)^{25°C}_{0.50\%} = \frac{\ln \frac{t_s}{t_o}}{C}$$

where:

$(\eta)$ = Inherent viscosity at 25°C at a polymer concentration of 0.5 g/100 ml of solvent;
ln = Natural logarithm;
$t_s$ = Sample flow time;
$t_o$ = Solvent-blank flow time; and
C = Concentration of polymer in grams per 100 ml of solvent = 0.50.

**Claims**

1. A colored composition comprising unsaturated polyester material, wherein the polyester has an I.V. of 0.05 to 0.25, an acid number of 10 to 28, and a number average molecular weight of from 1100 to 3800, having copolymerized therein a total of from 1.0 to 5000 ppm of at least one methine moiety, said moiety absorbing in the range of from 320 nm to 650 nm and being nonextractable from said polyester material, wherein each methine moiety is derived from a reactive compound having the formula

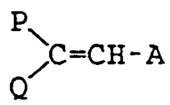 ,

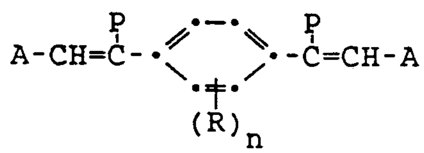 ,

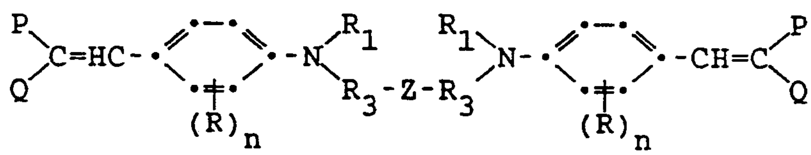 ,

or 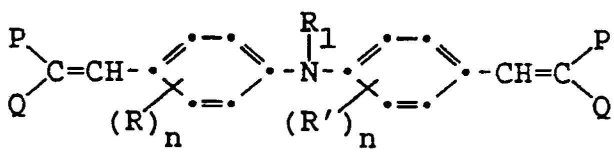

wherein each A is selected from the following radicals:

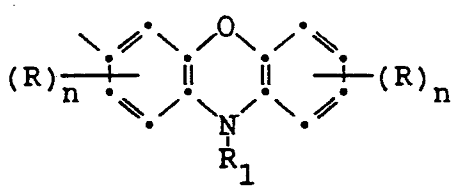 ,

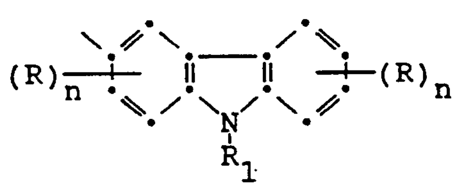 ,

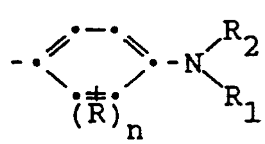 ,

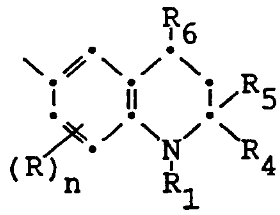 ,

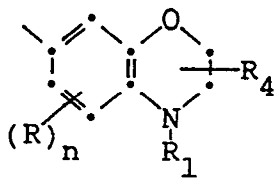 , 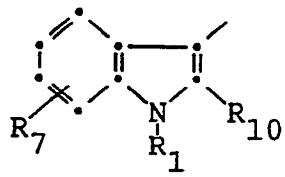 ,

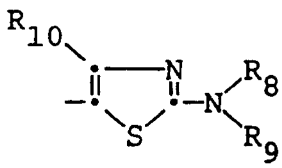 , 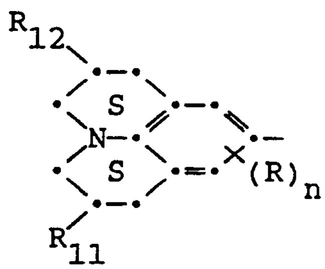 ,

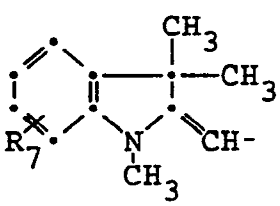 , 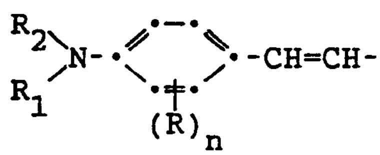

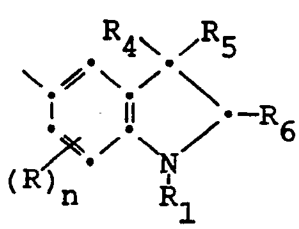 , and 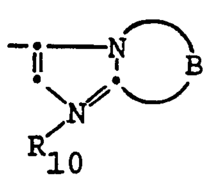

wherein:

R and R′ are selected from hydrogen, fluorine, chlorine, bromine, alkyl, alkoxy, phenyl, phenoxy, alkyltio, and arylthio; n is 0, 1, 2;

$R_1$ and $R_2$ are selected from hydrogen; cycloalkyl; cycloalkyl substituted with one or two of alkyl, —OH, alkoxy, halogen, or hydroxy substituted alkyl; phenyl; phenyl substituted with alkyl, alkoxy, halogen, alkanoylamino, carboxy, cyano, or alkoxycarbonyl; straight or branched lower alkenyl; straight or branched alkyl of 1—8 carbons and such alkyl substituted with the following: hydroxy; halogen; cyano; succinimido; hydroxysuccinimido; acyloxysucinimido; glutarimido; phenylcarbamoyloxy; phthalimido; 4-carboxyphthalimido; phthalimidino; 2-pyrrolidono; cyclohexyl; phenyl; phenyl substituted with alkyl, alkoxy, halogen, hydroxy alkanoylamino, carboxy, cyano, or alkoxycarbonyl; alkylsulfamoyl; vinylsulfonyl; acrylamido; sulfamyl; benzoylsulfonicimido; alkylsulfonamido; phenylsulfonamido; alkoxycarbonylamino; alkylcarbamoyloxy; alkoxycarbonyl; alkoxycarbonyloxy; alkenylcarbonylamino; groups of the formula

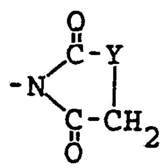

wherein Y is —NH—,

$$\overset{|}{-N\text{-alkyl}},$$

—O—, —S—, or —$CH_2O$—; —S—$R_{14}$; $SO_2CH_2CH_2SR_{14}$; wherein $R_{14}$ is alkyl, phenyl phenyl substituted with halogen, alkyl, alkoxy, alkanoylamino, cyano, or alkoxycarbonyl; pyridyl; pyrimidinyl; benzoxazolyl; benzimidazolyl; benzothiazolyl; radicals of the formulae

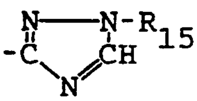 ;

—$OSR_{16}$; —$NHXR_{16}$; —X—$R_{16}$; —$CONR_{15}R_{15}$; and —$SO_2NR_{15}R_{15}$; wherein $R_{15}$ is selected from H, aryl, alkyl, and alkyl substituted with halogen, —OH, phenoxy, aryl, —CN, cycloalkyl, alkylsulfonyl, alkylthio, alkanoyloxy, or alkoxy; X is —CO—, —COO—, or —$SO_2$—; $R_{16}$ is selected from alkyl and alkyl substituted with halogen, hydroxy, phenoxy, aryl, cyano, cycloalkyl, alkylsulfonyl, alkylthio, alkanoyloxy, and alkoxy; and when X is —CO—, $R_{16}$ also can be hydrogen, amino, alkenyl, alkylamino, dialkylamino, arylamino, aryl, or furyl; alkoxy; alkoxy substituted with hydroxy, cyano, alkanoyloxy, or alkoxy; phenoxy; phenoxy substituted with one or more of alkyl, carboxy, alkoxy, carbalkoxy, or halogen; $R_1$ and $R_2$ can be a single combined group such as pentamethylene, tetramethylene, ethyleneoxy ethylene, ethylene sulfonylethylene, or

$$\text{ethylene}-\overset{\overset{XR_{17}}{|}}{N}\text{-ethylene}$$

which, with the nitrogen to which it is attached, forms a ring; $R_{17}$ is alkyl, aryl, or cycloalkyl;

$R_3$ is alkylene, arylene, aralkylene, alkyleneoxy, or alkyleneoxyalkylene;

Z is a direct single bond, OCO, O, S, $SO_2$, $R_{17}SO_2N$=,

$$-O\overset{\overset{O}{\|}}{C}\text{-alkylene-}\overset{\overset{O}{\|}}{C}O-,\quad -O\overset{\overset{O}{\|}}{C}\text{-arylene-}\overset{\overset{O}{\|}}{C}O-,\ -S-S-,$$

$$-O\overset{\overset{O}{\|}}{C}NH\text{-alkylene-}NH\overset{\overset{O}{\|}}{C}O,\ -O\overset{\overset{O}{\|}}{C}NH\text{-arylene-}NH\overset{\overset{O}{\|}}{C}O-,\ -O\overset{\overset{O}{\|}}{C}O-,$$

arylene, or alkylene;

$R_4$, $R_5$, and $R_6$ are each selected from hydrogen and alkyl;

$R_7$ is carboxy, carbalkoxy, or $(R)_n$;

$R_{10}$ is hydrogen, alkyl, and aryl;

$R_8$ and $R_9$ are each selected from hydrogen and substituted or unsubstituted alkyl, aryl, or cycloalkyl;

$R_{11}$ and $R_{12}$ are each selected from hydrogen, alkyl, hydroxyl, or acyloxy;

B represents the atoms necessary to complete a five or six membered ring and is selected from

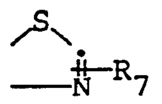 , 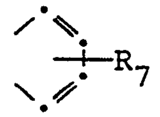 , 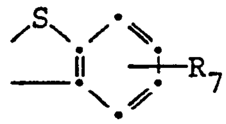 ,

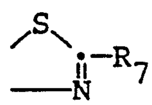 , 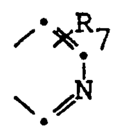 , 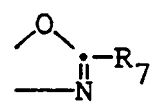 ,

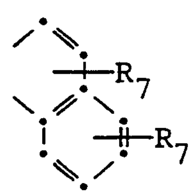 , 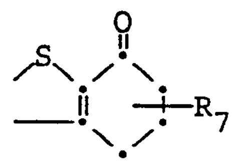 , 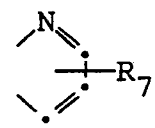

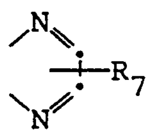 and 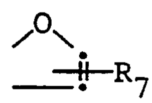 ;

each P and Q are selected from cyano, carbalkoxy, carbaryloxy, carbaralkyloxy, carbamyl, carboxy, N-alkylcarbamyl, N-alkyl-N-arylcarbamyl, N,N-dialkylcarbamyl, N-arylcarbamyl, N-cyclohexylcarbamyl, aryl, 2-benzoxazolyl, 2-benzothiazolyl, 2-benzimidazolyl, 1,3,4-thiadiazol-2-yl, 1,3,4-oxadiazol-2-yl, $SO_2$ alkyl, $SO_2$ aryl, and acyl, or P and Q may be combined as

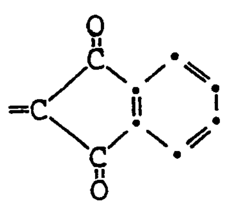 or 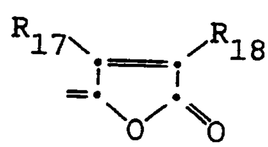

wherein $R_{17}$ is defined above and $R_{18}$ is CN, COOH, $CO_2$ alkyl, carbamyl, or N-alkylcarbamyl;

wherein at least one of A, P, and Q for each dye molecule must be or bear a condensable group selected from carboxy, carbalkoxy, carbaryloxy, N-alkylcarbamyloxy, acyloxy, chlorocarbonyl, carbamyloxy, N-(alkyl)$_2$carbamyloxy, amino, alkylamino, hydroxyl, N-phenylcarbamyloxy, cyclohexanoyloxy, and carbocyclohexyloxy; and

wherein in the above definitions, each alkyl, aryl, or cycloalkyl moiety or portion of a group or radical may be substituted where appropriate with hydroxyl, acyloxy, alkyl, cyano, alkoxycarbonyl, halogen, alkoxy, or aryl, aryloxy, or cycloalkyl.

2. The composition of claim 1 wherein the polyester acid component is comprised of 40—60 mole% isophthalic acid and conversely 60—40 mole% of either or a mixture of maleic fumaric acid, and the alcohol component is comprised of propylene glycol, neopentyl glycol, or mixtures thereof.

3. The composition of claim 3 wherein the polyester acid component is comprised of isophthalic acid and maleic acid, and the alcohol is neopentyl glycol or neopentyl glycol mixed with less than about 75 mole% propylene glycol.

4. The composition of claim 1 wherein the reactive compound has the formula

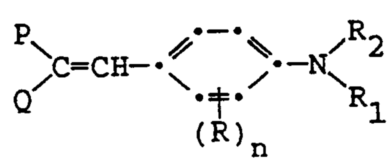

5. The composition of claim 4 wherein:

R is H, alkyl, halogen or alkoxy; and

$R_1$ and $R_2$ are each selected from H, alkyl, cycloalkyl, cycloalkyl substituted with one or more of alkyl, OH, CN, alkoxy, carbalkoxy or alkanoyloxy, and alkyl substituted with one or more of OH, CN, alkanoyloxy, carbalkoxy, aryl, substituted aryl, alkoxy, alkoxyalkoxy, halogen, succinimido or carbamyl.

6. The composition of claim 1 wherein the reactive compound has the formula

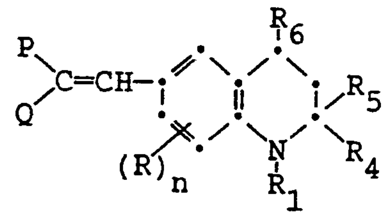

7. The composition of claim 6 where:

R is H, alkyl, halogen or alkoxy;

$R_1$ is selected from H, alkyl, cycloalkyl, cycloalkyl substituted with one or more of alkyl, OH, CN, alkoxy, carbalkoxy or alkanoyloxy, and alkyl substituted with one or more of OH, CN, alkanoyloxy, carbalkoxy, aryl, substituted aryl, alkoxy, alkoxy-alkoxy, halogen, succinimido or carbamyl; and

$R_4$, $R_5$ and $R_6$ are each H or alkyl.

8. The composition of claim 1 wherein the reactive compound has the formula

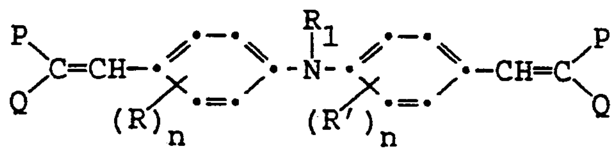 .

9. The composition of claim 8 wherein:

R and R′ are each selected from H, alkyl, halogen and alkoxy; and

$R_1$ is H, alkyl, cycloalkyl, cycloalkyl substituted with one or more of alkyl, OH, CN, alkoxy, carbalkoxy or alkanoyloxy, and alkyl substituted with one or more of OH, CN, alkanoyloxy, carbalkoxy, aryl, substituted aryl, alkoxy, alkoxyalkoxy, halogen, succinimido or carbamyl.

10. The composition of claim 1 wherein the reactive compound has the formula

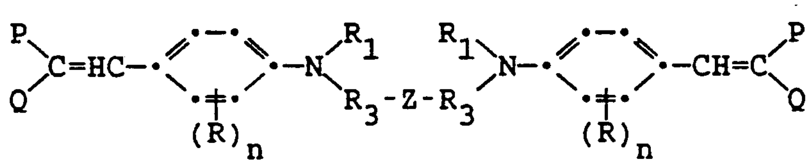 .

11. The composition of claim 10 wherein:

each R is H, alkyl, halogen or alkoxy; each $R_1$ is H, alkyl, cycloalkyl, cycloalkyl substituted with one or more of alkyl, OH, CN, alkoxy, carbalkoxy or alkanoyloxy, and alkyl substituted with one or more of OH, CN, alkanoyloxy, carbalkoxy, aryl, substituted aryl, alkoxy, alkoxyalkoxy, halogen, succinimido or carbamyl; and

Z is a direct bond, O, S, $SO_2$, 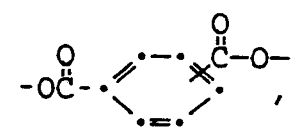 ,

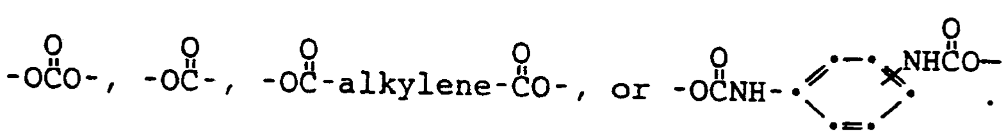 .

12. The composition of claim 1 wherein the condensable group is one or more of carboxy, carbalkoxy or hydroxy.

13. The composition of claim 1 wherein the reactive compound is

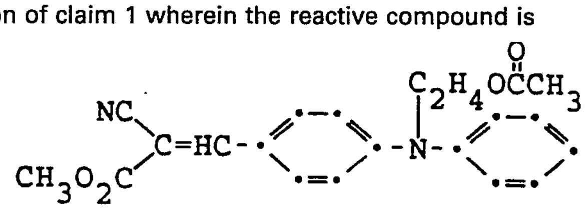

14. The composition of claim 1 wherein the reactive compound is

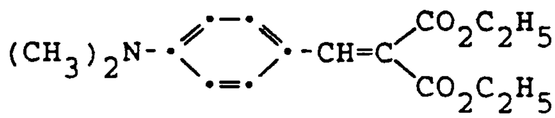 .

15. The composition of claim 1 wherein the reactive compound is

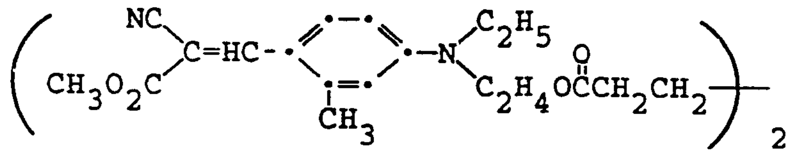 .

16. The composition of claim 1 wherein the reactive compound is

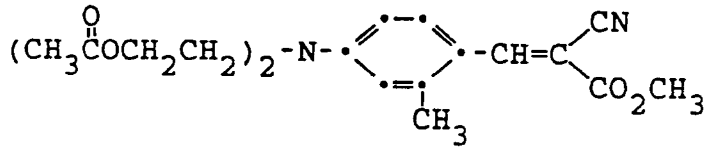 .

17. The composition of claim 1 wherein the reactive compound is

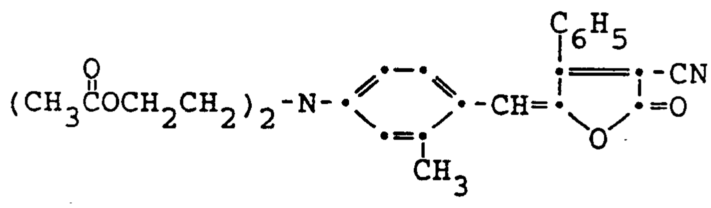 .

18. The composition of claim 1 wherein the reactive compound is

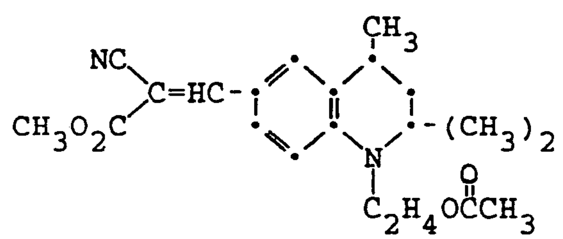 .

19. The composition of any of claims 1—3 blended with a curing agent.
20. The cured composition of claim 19.
21. Formed articles of the cured composition of claim 20.

**Patentansprüche**

1. Farbige Zusammensetzung mit einem ungesättigten Polyestermaterial, in der der Polyester eine I.V. von 0,05 bis 0,25, eine Säurezahl von 10 bis 28 sowie eine mittlere Molekulargewichtszahl von 1100 bis 3800 hat, und der eincopolymerisiert insgesamt 1,0 bis 5000 ppm mindestens einer Methingruppierung enthält, die im Bereich von 320 nm bis 650 nm absorbiert und aus dem Polyestermaterial nicht extrahierbar ist, wobei jede Methingruppierung sich von einer reaktiven Verbindung mit der Formel

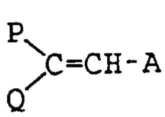 , 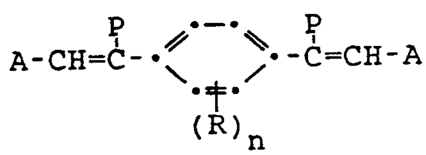 ,

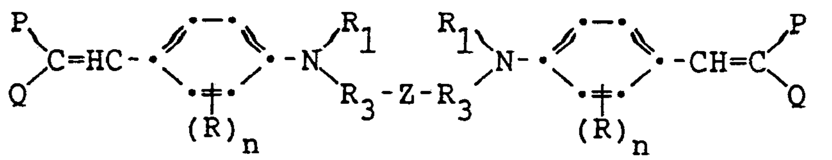 ,

oder 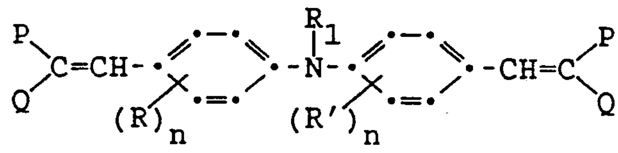

ableitet, wobei jedes A aus einem der folgenden Reste ausgewählt ist:

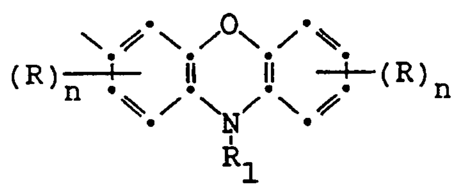 ,

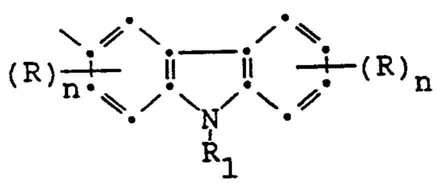 , 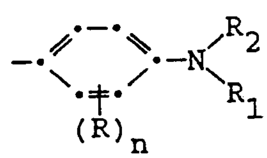 ,

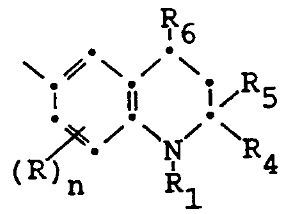 ,

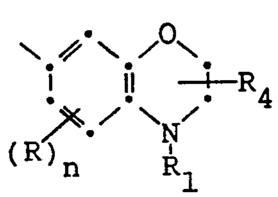 , 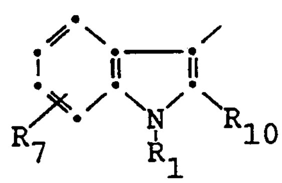 ,

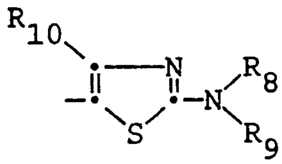 , 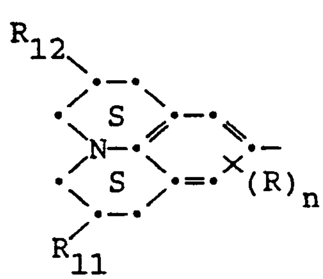 ,

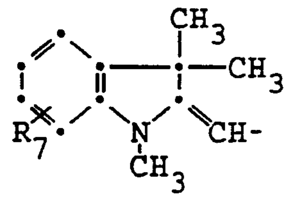 ,

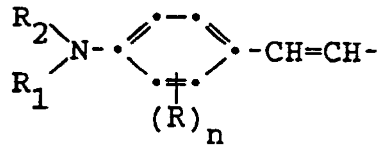 ,

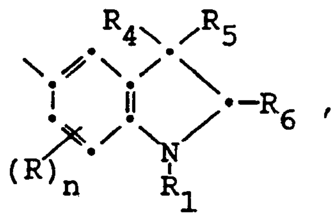 , und 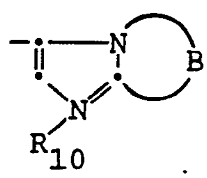

worin:

R und R' ausgewählt sind aus Wasserstoff, Fluor, Chlor, Brom, Alkyl, Alkoxy, Phenyl, Phenoxy, Alkylthio und Arylthio; und n gleich 0, 1 oder 2 ist;

$R_1$ und $R_2$ ausgewählt sind aus Wasserstoff, Cycloalkyl; Cycloalkyl, das substituiert ist durch ein oder zwei Alkyl, OH—, Alkoxy, Halogen, oder Hydro-substituiertem Alkyl; Phenyl; Phenyl, substituiert durch Alkyl, Alkoxy, Halogen, Alkanoylamino, Carboxy, Cyano oder Alkoxycarbonyl; geradkettigem oder verzweigtkettigem kurzkettigen Alkenyl; geradkettigem oder verzweigtkettigem Alkyl mit 1—8 Kohlenstoffatomen und solchem Alkyl, das substituiert ist durch: Hydroxy; Halogen; Cyano; Succinimido; Hydroxysuccinimido; Acyloxysuccinimido; Glutarimido; Phenylcarbamoyloxy; Phthalimido; 4-Carboxyphthalimido; Phthalimidino; 2-Pyrrolidono; Cyclohexyl; Phenyl; Phenyl substituiert mit Alkyl, Alkoxy, Halogen, Hydroxyalkanoylamino, Carboxy, Cyano oder Alkoxycarbonyl; Alkylsulfamoyl; Vinylsulfonyl; Acrylamido; Sulfamyl, Benzoylsulfonicimido; Alkylsulfonamido, Phenylsulfonamido; Alkoxycarbonylamino, Alkylcarbamoyloxy; Alkoxycarbonyl; Alkoxycarbonyloxy; Alkenylcarbonylamino; Gruppen der Formel

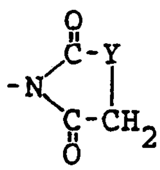

in der Y gleich —NH—, —N-alkyl, —O—, —S—, oder —$CH_2O$— ist; —$SR_{14}$; $SO_2CH_2CH_2SR_{14}$; worin $R_{14}$ gleich Alkyl, Phenyl, Phenyl substituiert mit Halogen, Alkyl, Alkoxy, Alkanoylamino, Cyano oder Alkoxycarbonyl ist; Pyridyl; Pyrimidinyl; Benzoxazolyl, Benzimidazolyl; Benzothiazolyl; Resten der Formeln

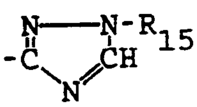 ;

—$OXR_{16}$; —$NHXR_{16}$; —X—$R_{16}$; —$CONR_{15}R_{15}$; und —$SO_2NR_{15}R_{15}$; worin $R_{15}$ ausgewählt ist aus H, Aryl, Alkyl, und Alkyl substituiert mit Halogen, —OH, Phenoxy, Aryl-, —CN, Cycloalkyl, Alkylsulfonyl, Alkylthio, Alkanoyloxy oder Alkoxy; X ist gleich —CO—, —COO—, oder —$SO_2$—, $R_{16}$ ist, ausgewählt aus Alkyl und Alkyl, substituiert mit Halogen, Hydroxy, Phenoxy, Aryl, Cyano, Cycloalkyl, Alkylsulfonyl, Alkylthio, Alkanoyloxy, und Alkoxy, wobei gilt, daß wenn X gleich —CO— ist, $R_{16}$ ferner stehen kann für Wasserstoff, Amino, Alkenyl, Alkylamino, Dialkylamino, Arylamino, Aryl oder Furyl; Alkoxy; Alkoxy, substituiert mit Hydroxy, Cyano, Alkanoyloxy oder Alkoxy; Phenoxy; Phenoxy, substituiert mit einem oder mehreren Alkyl, Carboxy, Alkoxy, Carbalkoxy oder Halogen; und worin $R_1$ und $R_2$ eine einzelne kombinierte Gruppe bilden können, z.B. Pentamethylen, Tetramethylen, Ethylenoxyethylen, Ethylensulfonylethylen oder

$XR_{17}$
|
Ethylen-N-ethylen,

die mit dem Stickstoff, an den sie gebunden ist, einen Ring bildet, wobei $R_{17}$ für Alkyl, Aryl oder Cycloalkyl steht;

$R_3$ steht für Alkylen, Arylen, Aralkylen, Alkylenoxy oder Alkylenoxyalkylen;
Z ist eine direkte einfache Bindung oder OCO, O, S, $SO_2$, $R_{17}SO_2N=$,

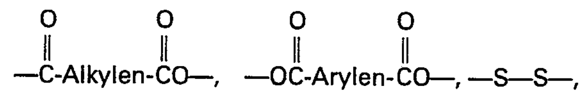

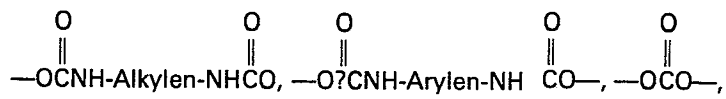

Arylen oder Alkylen;

$R_4$, $R_5$ und $R_6$ sind jeweils ausgewählt aus Wasserstoff und Alkyl;

$R_7$ steht für Carboxy, Carbalkoxy oder $(R)_n$;

$R_{10}$ ist Wasserstoff, Alkyl oder Aryl;

$R_8$ ud $R_9$ sind ausgewählt aus Wasserstoff und substituiertem oder unsubstituiertem Alkyl, Aryl oder Cycloalkyl;

$R_{11}$ und $R_{12}$ sind jeweils ausgewählt aus Wasserstoff, Alkyl, Hydroxyl oder Acyloxy;

B steht für die Atome, die erforderlich sind, um einen fünf- oder sechsgliedrigen Ring zu vervollständigen und ist ausgewählt aus:

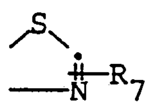 , 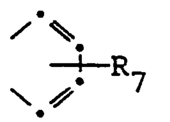 , 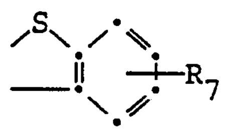 ,

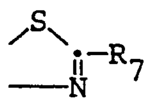 , 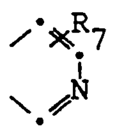 , 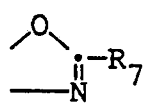 ,

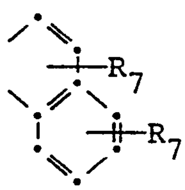 , 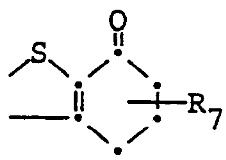 , 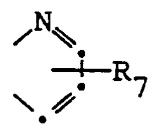

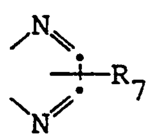 und 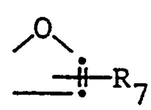 ;

P und Q sind jeweils ausgewählt aus Cyano, Carbalkoxy, Carbaryloxy, Carbaralkyloxy, Carbamyl, Carboxy, N-Alkylcarbamyl, N-Alkyl-N-arylcarbamyl, N-N-Dialkylcarbamyl, N-Arylcarbamyl, N-Cyclohexylcarbamyl, Aryl, 2-Benzoxazolyl, 2-Benzothiazoyl, 2-Benzimidazolyl, 1,3,4-Thiadiazol-2-yl, 1,3,4-Oxadiazol-2-yl, $SO_2$-Alkyl, $SO_2$-Aryl und Acyl oder P und Q können kombiniert sein zu

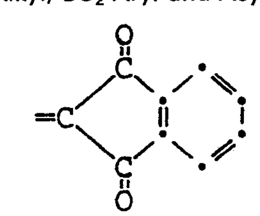 oder 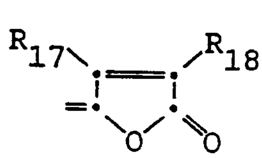

worin $R_{17}$ die bereits angegebene Bedeutung hat und $R_{18}$ steht für-CN, —COOH, —$CO_2$-alkyl, Carbamyl oder N-alkylcarbamyl;

wobei mindestens einer der Reste A, P und Q für jedes Farbstoffmolekül eine kondensierbare Gruppe,

ausgewählt aus den folgenden Gruppen sein muß oder eine solche Gruppe tragen muß: Carboxy, Carbalkoxy, Carbaryloxy, N-Alkylcarbamyloxy, Acyloxy, Chlorocarbonyl, Carbamyloxy, N-$(Alkyl)_2$carbamyloxy, Amino, Alkylamino, Hydroxyl, N-Phenylcarbamyloxy, Cyclohexanoyloxy und Carbocyclohexyloxy;
und worin in den angegebenen Definitionen jeder Alkyl-, Aryl- oder Cycloalkylrest gegebenenfalls substituiert sein kann mit Hydroxyl, Acyloxy, Alkyl, Cyano, Alkoxycarbonyl, Halogen, Alkoxy oder Aryl, Aryloxy oder Cycloalkyl.

2. Zusammensetzung nach Anspruch 1, in der die Polyester-Säurekomponente zu 40—60 Mol-% aus Isophthalsäure und zu 60—40 Mol-% aus entweder Maleinsäure oder Fumarsäure oder einer Mischung hiervon besteht und die Alkoholkomponente aus Propylenglykol, Neopentylglykol oder einer Mischung hiervon.

3. Zusammensetzung nach Anspruch 2, in der die Polyester-Säurekomponente aus Isophthalsäure und Maleinsäure und die Alkoholkomponente aus Neopentylglykol, oder Neopentylglykol, vermischt mit weniger als etwa 75 Mol-% Proylenglykol besteht.

4. Zusammensetzung nach Anspruch 1, in der die reaktive Verbindung die folgende Formel hat;

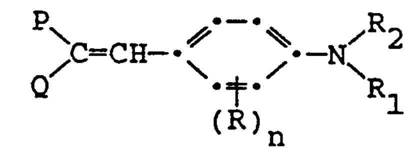

5. Zusammensetzung nach Anspruch 4, in der bedeuten:
R gleich Wasserstoff, Alkyl, Halogen oder Alkoxy und
$R_1$ und $R_2$ jeweils ausgewählt sind aus Wasserstoff, Alkyl, Cycloalkyl, Cycloalkyl substituiert mit einem oder mehreren Alkyl, —OH, —CN, Alkoxy, Carbalkoxy, oder Alkanoyloxy und Alkyl substituiert mit einem oder mehreren —OH, —CN, Alkanoyloxy, Carbalkoxy, Aryl, substituiertem Aryl, Alkoxy, Alkoxyalkoxy, Halogen, Succinimido oder Carbamyl.

6. Zusammensetzung nach Anspruch 1, in der die reaktive Verbindung der folgenden Formel entspricht:

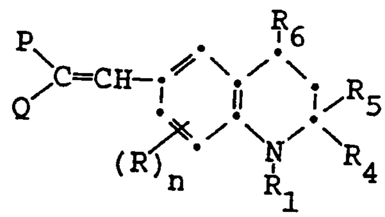

7. Zusammensetzung nach Anspruch 6, worin bedeuten:
R Wasserstoff, Alkyl, Halogen oder Alkoxy;
$R_2$ Wasserstoff, Alkyl, Cycloalkyl, Cycloalyl substituiert mit einem oder mehreren Alkyl, —OH, —CN, Alkoxy, Carbalkoxy oder Alkanoyloxy und Alkyl substituiert mit einem oder mehreren —OH, —CH, Alkanoyloxy, Carbalkoxy, Aryl, substituiertem Aryl, Alkoxy, Alkoxy-alkoxy, Halogen, Succinimido oder Carbamyl und
$R_4$, $R_5$ und $R_6$ jeweils Wasserstoff oder Alkyl.

8. Zusammensetzung nach Anspruch 1, in der die reaktive Verbindung der folgenden Formel entspricht:

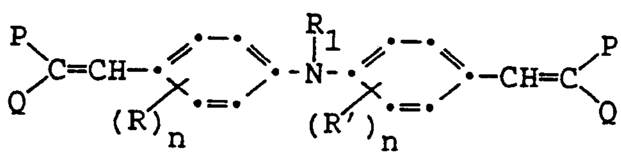 .

9. Zusammensetzung nach Anspruch 8, in der bedeuten:
R und R' jeweils Wasserstoff, Alkyl, Halogen oder Alkoxy und
$R_1$ Wasserstoff, Alkyl, Cycloalkyl, Cycloalkyl substituiert mit einem oder mehreren Alkyl-, —OH, —CN, Alkoxy, Carbalkoxy oder Alkanoyloxy und Alkyl substituiert mit einem oder mehreren —OH, —CN, Alkanoyloxy, Carbalkoxy, Aryl, substituiertem Aryl, Alkoxy, Alkoxyalkoxy, Halogen, Succinimido oder Carbamyl.

10. Zusammensetzung nach Anspruch 1, in der die reaktive Verbindung der folgenden Formel entspricht:

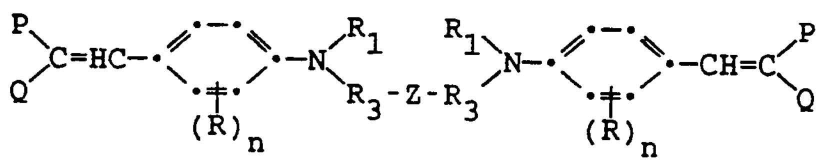 .

11. Zusammensetzung nach Anspruch 10, worin bedeuten:
R jeweils Wasserstoff, Alkyl, Halogen oder Alkoxy;
$R_1$ jeweils Wasserstoff, Alkyl, Cycloalkyl, Cycloalkyl substituiert mit einem oder mehreren Alkyl, —OH, —CN, Alkoxy, Carbalkoxy oder Alkanoyloxy und Alkyl substituiert mit einem oder mehreren —OH, —CN, Alkanoyloxy, Carbalkoxy, Aryl, substituiertem Aryl, Alkoxy, Alkoxyalkoxy, Halogen, Succinimido oder Carbamyl; und

Z eine direkte Bindung, O, S, $SO_2$, 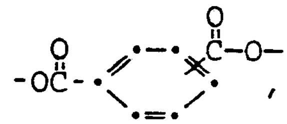 ,

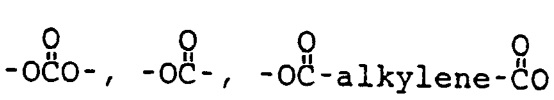 oder 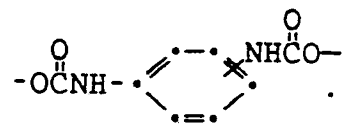 .

12. Zusammensetzung nach Anspruch 1, in der die kondensierbare Gruppe aus einer oder mehreren Carboxy-, Carbalkoxy- oder Hydroxygruppen besteht.

13. Zusammensetzung nach Anspruch 1, in der die reaktive Verbindung besteht aus:

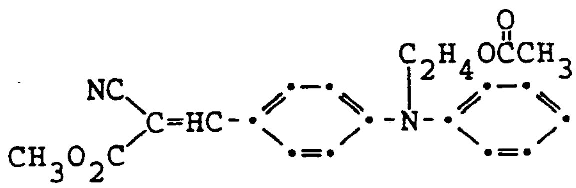

14. Zusammensetzung nach Anspruch 1, in der die reaktive Verbindung besteht aus:

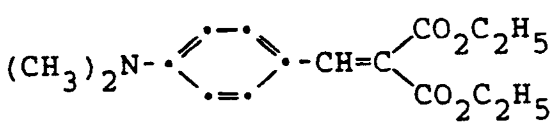 .

15. Zusammensetzung nach Anspruch 1, in der die reaktive Verbindung besteht aus:

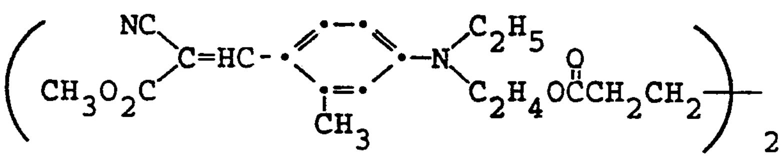 .

16. Zusammensetzung nach Anspruch 1, in der die reaktive Verbindung besteht aus:

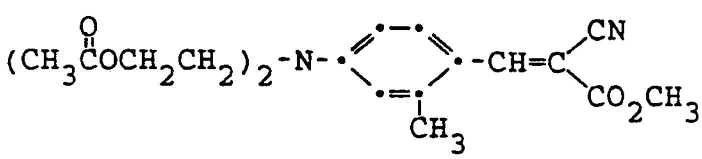 .

17. Zusammensetzung nach Anspruch 1, in der die reaktive Verbindung besteht aus:

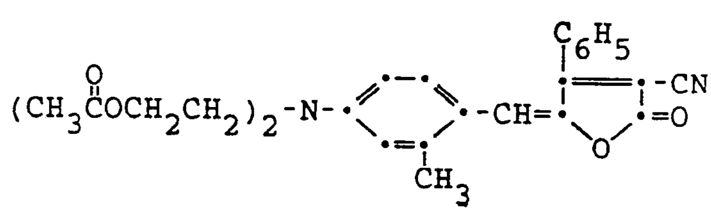 .

18. Zusammensetzung nach Anspruch 1, in der die reaktive Verbindung besteht aus:

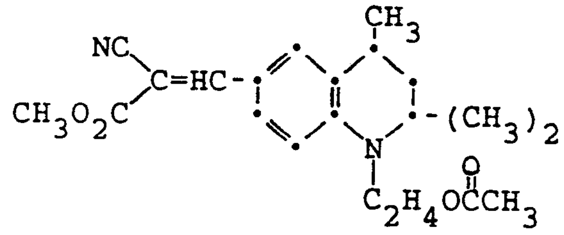

19. Zusammensetzung nach einem der Ansprüche 1—3, vermischt mit einem Härungsmittel.

20. Die gehärtete Zusammensetzung nach Anspruch 19.

21. Formkörper, hergestellt aus der gehärteten Zusammensetzung nach Anspruch 20.

## Revendications

1. Composition colorée comprenant un polyester insaturé, ayant une viscosité inhérente comprise entre 0,05 et 0,25, un indice d'acidité compris entre 10 et 28 et un poids moléculaire moyen en nombre compris entre 1100 et 3800, dans lequel est copolymérisé un total de 1,0 à 5000 ppm d'au mons un groupement méthine, ledit groupement méthine absorbant dans la région du spectre comprise entre 320 nm et 650 nm et étant non extractible dudit polymère, et dans lequel chaque groupement méthine est dérivé d'un composé réactif ayant la formule:

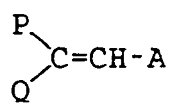 , 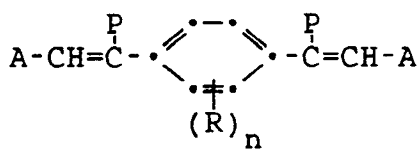 ,

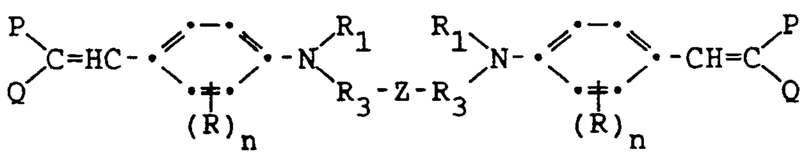 ,

ou 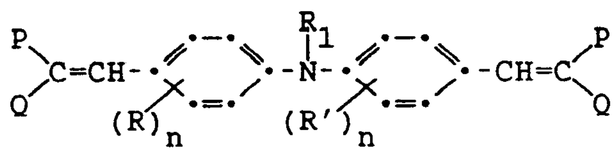

où chaque A est choisi parmi les radicaux suivants:

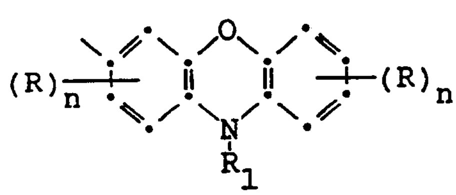 ,

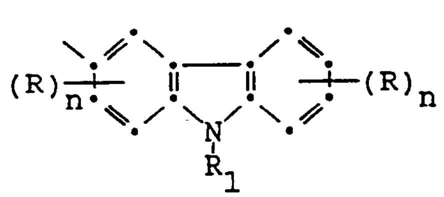 , 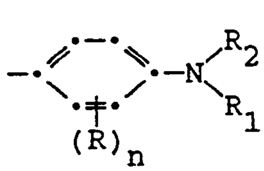 ,

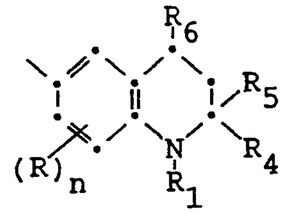 ,

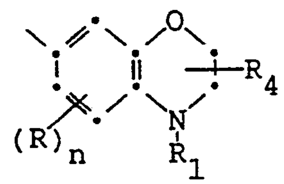 , 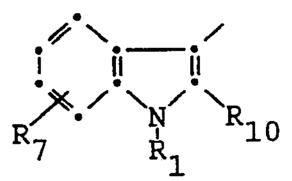 ,

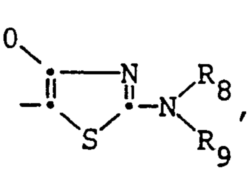 , 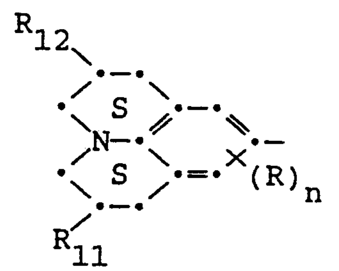 ,

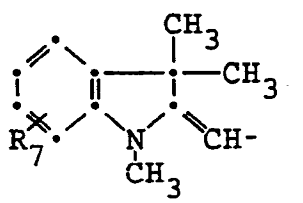 , 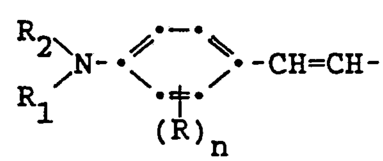

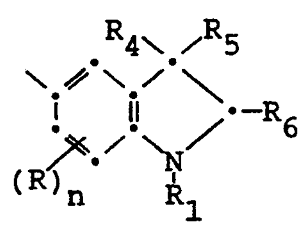 , et 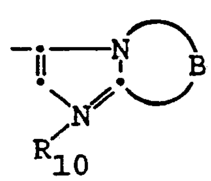

où:

R et R′ sont choisis parmi les atomes d'hydrogène, de fluor, de chlore, de brome, les radicaux alkyle, alkoxy, phényle, phénoxy, alkylthio, et arylthio; n est 0, 1, 2;

$R_1$ et $R_2$ sont choisis parmi un atome d'hydrogène; un radical cycloalkyle; cycloalkyle substitué par un ou deux alkyle, —OH, alkoxy, halogène ou alkyle substitué par un groupe hydroxy; un radical phényle; phényle substitué par un alkyle, alkoxy, halogène, alkanoylamino, carboxy, cyano, ou alkoxycarbonyle; un radical alkènyle inférieur droit ou ramifié; un radical alkyle droit ou ramifié de 1 à atomes de carbone et un radical alkyle substitué par les groupes suivants; hydroxy; halogène; cyano; succinimido; hydroxysuccinimido; acyloxysuccinimido; glutarimido; phénylcarbamoyloxy; phtalimido; 4-carboxyphtalimido; phtalimidono; 2-pyrrolidono; cyclohexyle; phényle; phényle substitué par un groupe alkyle, alkoxy, halogène hydroxy alkanoylamino, carboxy, cyano, ou alkoxycarbonyle; alkylsulfamoyle;

vinylsulfonyle; acrylamido; sulfamyle; benzoylsulfonicimido; alkylsulfonamido; phénylsulfonamido; alkoxycarbonylamino; alkylcarbamoyloxy; alkoxycarbonyle; alkoxycarbonyloxy; alkénylcarbonylamino; les groupes de formule:

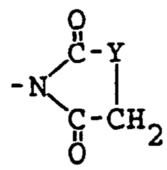

où Y est —NH—, —N-alkyle, —O—, —S—, ou —$CH_2O$—; —S—$R_{14}$; $SO_2CH_2CH_2SR_{14}$; où $R_{14}$ est un radical alkyle, phényle, phényle substitué par un halogène, alkyle, alkoxy, alkanoylamino, cyano, ou alkoxycarbonyle; pyridyle; pyrimidinyle; benzoxazolyle; benzimidazolyle; benzothiazolyle; ou un radical de formule:

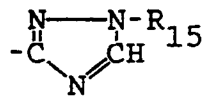 ;

—$OXR_{16}$; —$NHXR_{16}$; —X—$R_{16}$; —$CONR_{15}R_{15}$; et —$SO_2NR_{15}R_{15}$; où $R_{15}$ est choisi parmi un atome d'hydrogène, un groupe aryle, alkyle, et alkyle substitué par un halogène, —OH, phénoxy, aryle, —CN, cycloalkyle, alkylsulfonyle, alkylthio, alkanoyloxy, ou alkoxy; X est —CO—, —COO—, ou —$SO_2$—; $R_{16}$ est choisi parmi les radicaux alkyle et alkyle substitué par un halogène, hydroxy, phénoxy, aryle, cyano, cycloalkyle, alkylsulfonyle, alkylthio, alkanoyloxy, et alkoxy; et lorsque X est —CO—, $R_{16}$ peut aussi être un atome d'hydrogène, un radical amino, alkényle, alkylamino, dialkylamino, arylamino, aryle ou furyle; alkoxy; alkoxy substitué par un hydroxy, cyano, alkanoyloxy, ou alkoxy; phénoxy; phénoxy substitué par un ou plusieurs alkyle, carboxy, alkoxy, carbalkoxy, ou halogène; $R_1$ et $R_2$ peuvent former un groupe unique tel que pentaméthylène, tétraméthylène, éthylèneoxyéthylène, éthylène sulfonyléthylène, ou

$$\underset{\text{éthylène-N-éthylène}}{\overset{XR_{17}}{|}}$$

qui, avec l'atome d'azote auquel il est attaché forme un cycle; $R_{17}$ est alkyle, aryle ou cycloalkyle;

$R_3$ est alkylène, arylène, aralkylène, alkylèneoxy, ou alkylèneoxyalkylène;

Z est une liaison simple, OCO, O, S, $SO_2$, $R_{17}SO_2N$=,

$$—O\overset{O}{\overset{\|}{C}}\text{-alkylène-}\overset{O}{\overset{\|}{C}}O—,\ —O\overset{O}{\overset{\|}{C}}\text{-arylène-}\overset{O}{\overset{\|}{C}}O—,\ —S—S—,$$

$$—O\overset{O}{\overset{\|}{C}}NH\text{-alkylène-}NH\overset{O}{\overset{\|}{C}}O,\ —O\overset{O}{\overset{\|}{C}}NH\text{-arylène-}NH\overset{O}{\overset{\|}{C}}O—,\ —O\overset{O}{\overset{\|}{C}}O—,$$

arylène, ou alkylène;

$R_4$, $R_5$, et $R_6$ sont chacun choisis parmi un atome d'hydrogène et un radical alkyle;

$R_7$ est un radical carboxy, carbalkoxy, ou $(R)_n$;

$R_{10}$ est un atome d'hydrogène, un radical alkyle, et aryle;

$R_8$ et $R_9$ chacun choisis parmi un atome d'hydrogène, un radical alkyle, aryle ou cycloalkyle substitué ou non;

$R_{11}$ et $R_{12}$ sont chacun choisis parmi un atome d'hydrogène, un radical alkyle, hydroxyle ou acycloxy

B représente le nombre d'atomes nécessaire pour compléter un cycle à 5 ou 6 chaînons et est choisi parmi;

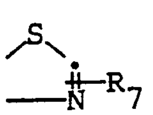 , 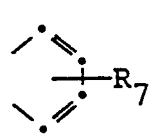 , 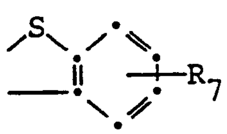 ,

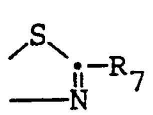 , 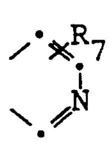 , 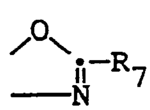 ,

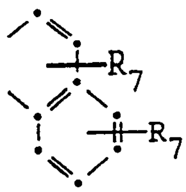 , 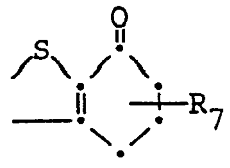 , 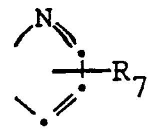

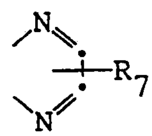 et 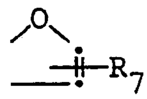 ;

chacun des P et Q est choisi parmi les radicaux cyano, carbalkoxy, carbaryloxy, carbaralkyloxy, carbamyle, carboxy, N-alkylcarbamyle, N-alkyl-N-arylcarbamyle, N,N-dialkylcarbamyle, N-arylcarbamyle, N-cyclohexylcarbamyle, aryle, 2-benzoxazolyle, 2-benzothiazolyle 2-benzimidazolyle, 1,3,4-thiadiazol-2-yle, 1,3,4-oxadiazol-2-yle, $SO_2$-alkyle, $SO_2$-aryle, et acyle, ou P et Q peuvent être combinés sous la formule

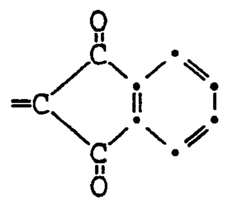 ou 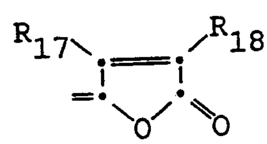

où $R_{17}$ est défini comme ci-dessus et $R_{18}$ est CN, COOH, $CO_2$-alkyle, carbamyle, ou N-alkylcarbamyle;

où au moins un des A, P, et Q pour chaque molécule de colorant peut être ou peut porter un groupe condensable choisi parmi les groupes carboxy, carbalkoxy, carbaryloxy, N-alkylcarbamyloxy, acyloxy, chlorocarbonyle, carbamyloxy, N-(alkyl)$_2$-carbamyloxy, amino, alkylamino, hydroxyle, N-phénylcarbamyloxy, cyclohexanoyloxy, et carbocyclohexyloxy; et

où dans les définitions ci-dessus, chaque groupement ou portion de groupement ou radical alkyle, aryle, ou cycloalkyle peut être substitué là où c'est possible par un radical hydroxyle, acyloxy, alkyle, cyano, alkoxycarbonyle, un atome d'halogène, un radical alkoxy, ou aryle, aryloxy, ou cycloalkyle.

2. Composition selon la revendication 1, dans lequel la composante acide du polyester comprend de 40 à 60% en poids d'acide isophtalique, et inversement 60 à 40% soit d'acide maléique soit d'acide fumarique, soit d'un mélange d'acide maléique et d'acide fumarique, et la composante alcool du polyester comprend du polypropylène glycol, du néopentyl glycol ou des mélanges de ces derniers.

3. Composition selon la revendication 2, dans lequel la composante acide du polyester comprend de l'acide isophtalique et de l'acide maléique et l'alcool est le néopentyl glycol, où du néopentyl glycol mélange à au moins d'environ 75% en moles de propylène glycol.

4. Composition selon la revendication 1, dans laquelle le composé réactif a la formule:

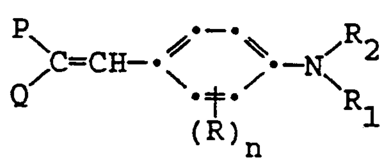

5. Composition selon la revendication 4, dans laquelle;

R est H, alkyle, halogène ou alkoxy; et

$R_1$ et $R_2$ sont chacun séparément choisis parmi H alkyle, cycloalkyle, cycloalkyle substitué par un ou plusieurs groupes alkyle, OH, CN, alkoxy, carbalkoxy ou alkanoyloxy, et alkyle substitué par un ou plusieurs OH, CN, alkanoyloxy, carbalkoxy, aryle, aryle substitué, alkoxy, alkoxyalkoxy, halogène, succinimido ou carbamyle.

6. Composition selon la revendication 1, dans laquelle le composé réactif a la formule:

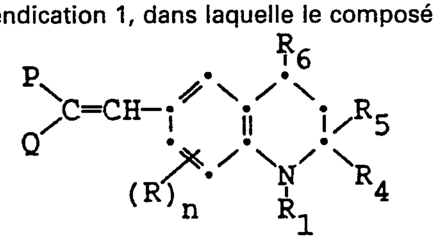

7. Composition selon la revendication 6, dans laquelle;

R est H, alkyle, halogène ou alkoxy;

$R_1$ est choisi parmi H, alkyle, cycloalkyle, cycloalkyle substitué par un ou plusieurs alkyle, OH, CN, alkoxy, carbalkoxy ou alkanoyloxy, et alkyle substitué par un ou plusieurs OH, CN, alkanoyloxy, carbalkoxy, aryle, aryle substitué, alkoxy, alkoxyalkoxy, halogène, succinimido ou carbamyle; et

$R_4$, $R_5$ et $R_6$ sont chacun H ou alkyle.

8. Composition selon la revendication 1, dans laquelle le groupe réactif a la formule:

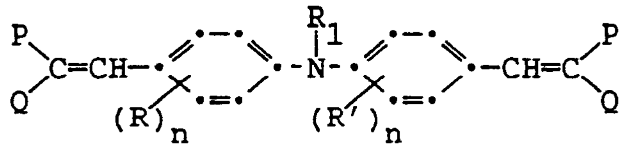
.

9. Composition selon la revendication 10, dans laquelle R et R′ sont chacun choisis parmi H, alkyle, halogène et alkoxy; et

$R_1$ est H, alkyle, cycloalkyle, cycloalkyle substitué par un ou plusieurs alkyle, OH, CN, alkoxy, carbalkoxy ou alkanoyloxy, et alkyle substitué par un ou plusieurs OH, CN, alkanoyloxy, carbalkoxy, aryle, aryle substitué, alkoxy, alkoxyalkoxy, halogène, succinimido ou carbamyle.

10. Composition selon la revendication 1, dans laquelle le composé réactif a la formule:

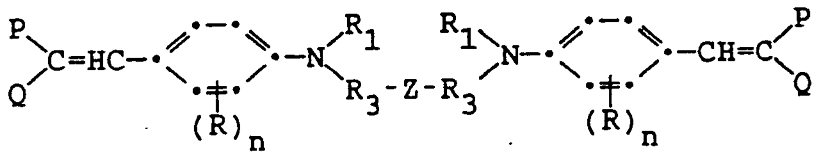
.

11. Composition selon la revendication 10, dans laquelle chaque R est H, alkyle, halogène ou alkoxy; chaque $R_1$ est H, alkyle, cycloalkyle, cycloalkyle substitué par un ou plusieurs alkyle, OH, CN, alkoxy, carbalkoxy ou alkanoyloxy, et alkyle substitué par un ou plusieurs OH, CN, alkanoyloxy, carbalkoxy, aryle, aryle substitué, alkoxy, alkoxyalkoxy, halogène, succinimido ou carbamyle, et

Z est une liaison simple, O, S, $SO_2$, 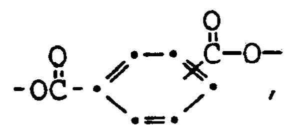 ,

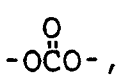 , 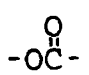 , 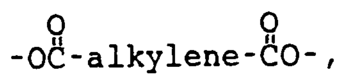 , ou 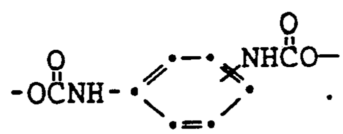 .

12. Composition selon la revendication 1, dans laquelle le groupe condensable est composé d'un ou plusieurs carboxy, carbalkoxy ou hydroxy.

13. Composition selon la revendication 1, dans laquelle le composé réactif est

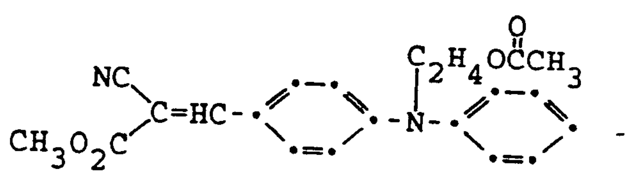
.

14. Composition selon la revendication 1, dans laquelle le composé réactif est

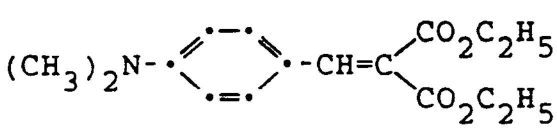
.

15. Composition selon la revendication 1, dans laquelle le composé réactif est

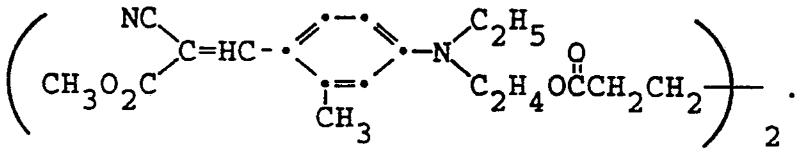

16. Composition selon la revendication 1, dans laquelle le composé réactif est

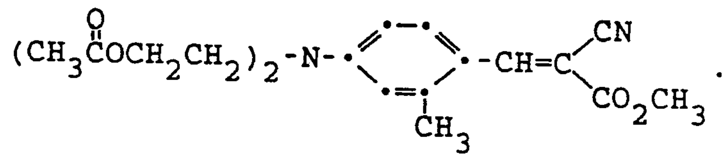

17. Composition selon la revendication 1, dans laquelle le composé réactif est

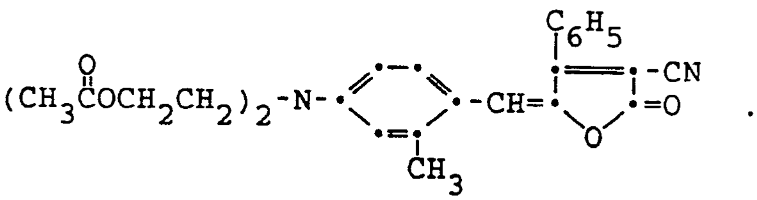

18. Composition selon la revendication 1, dans laquelle le composé réactif est

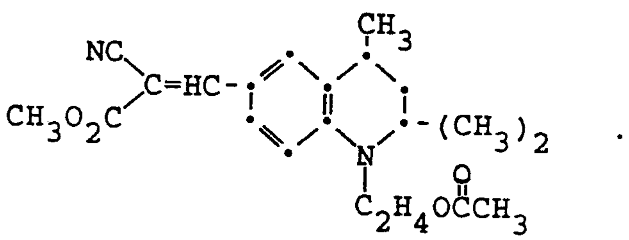

19. Composition selon l'uen quelconque des revendications 1 à 3 en mélange avec un agent de réticulation.

20. Composition réticulée selon la revendication 19.

21. Articles formés à partir de la composition réticulée de la revendication 20.